(19) Europäisches Patentamt
European Patent Office
Office européen des brevets

(11) EP 4 434 549 A1

(12) **EUROPEAN PATENT APPLICATION**
published in accordance with Art. 153(4) EPC

(43) Date of publication:
**25.09.2024 Bulletin 2024/39**

(21) Application number: **22894913.7**

(22) Date of filing: **17.11.2022**

(51) International Patent Classification (IPC):
***A61K 47/68*** *(2017.01)* ***A61K 39/395*** *(2006.01)*
***A61P 35/00*** *(2006.01)*

(52) Cooperative Patent Classification (CPC):
**A61K 31/4745; A61K 39/395; A61K 47/68; A61P 35/00; A61P 35/02; C07K 16/28; C07K 16/32**

(86) International application number:
**PCT/CN2022/132627**

(87) International publication number:
**WO 2023/088382 (25.05.2023 Gazette 2023/21)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **17.11.2021 CN 202111365644**

(71) Applicant: **CSPC Megalith Biopharmaceutical Co., Ltd.**
**Shijiazhuang, Hebei 050025 (CN)**

(72) Inventors:
- **YAO, Bing**
  **Shijiazhuang, Hebei 050025 (CN)**
- **HUI, Xiwu**
  **Shijiazhuang, Hebei 050025 (CN)**
- **PAN, Fujun**
  **Shijiazhuang, Hebei 050025 (CN)**
- **SHEN, Mingyue**
  **Shijiazhuang, Hebei 050025 (CN)**
- **DAN, Mo**
  **Shijiazhuang, Hebei 050025 (CN)**
- **LIU, Boning**
  **Shijiazhuang, Hebei 050025 (CN)**
- **YANG, Jinyu**
  **Shijiazhuang, Hebei 050025 (CN)**
- **GAO, Xiao**
  **Shijiazhuang, Hebei 050025 (CN)**
- **YUAN, Can**
  **Shijiazhuang, Hebei 050025 (CN)**
- **GAO, Hu**
  **Shijiazhuang, Hebei 050025 (CN)**

(74) Representative: **Barrow, Nicholas Martin et al**
**Venner Shipley LLP**
**406 Cambridge Science Park**
**Milton Road**
**Cambridge CB4 0WW (GB)**

Remarks:
The complete document including Reference Table(s) and the Sequence Listing(s) can be downloaded from the EPO website

(54) **ANTIBODY-DRUG CONJUGATE AND USE THEREOF**

(57) An antibody-drug conjugate targeting HER2, HER3 and EGFR, and a pharmaceutically acceptable salt, a hydrate, a solvate or an isotopically labelled analogue thereof, the use thereof, and a preparation method therefor.

EP 4 434 549 A1

## Description

## Technical Field

The present invention relates to an antibody-drug conjugate of camptothecin derivatives with anti-tumour effect and use thereof.

## Background of Art

The antibody-drug conjugate (ADC) consists of three different components (antibody, linker and drug). The ADC technology is to couple an antibody and a drug molecule together through a linker, utilize the specificity of the antibody to transport the drug molecule in a targeted manner to a target tissue to play a role, e.g. reducing the systemic toxic and side effects of the drug, expanding the drug treatment window, and improving the therapeutic potential of the antibody.

As a low-molecular weight compound having antitumour activity, camptothecin derivatives are known to be able to exhibit antitumour activity as topoisomerase I inhibitors. Efforts have been made to find highly potent, low toxicity camptothecin derivatives, and to date a series of semi-synthetic and fully synthetic camptothecin derivatives have emerged and entered clinical use or clinical trials.

Daiichi Sankyo's ADC drug DS-8201 uses the cytotoxic drug (payload) DXd, which has a unique mechanism of action and is 10 times more active than the common chemotherapy drug irinotecan. DXd has a strong ability to penetrate cell membranes, which allows it to kill nearby cancer cells after damaging cancer cells that swallow ADC, resulting in a "bystander effect"; and its half-life in the blood is significantly shortened, which helps reduce the occurrence of toxic side effects. The linker has high stability, and non-tumour tissues will not be affected by toxic drugs; it can be specifically cleaved by lysosomal proteases highly expressed in tumours; it can be coupled with multiple cytotoxic drugs in an antibody molecule to increase the drug antibody ratio (DAR). It provides a new research direction for the development of ADC drugs.

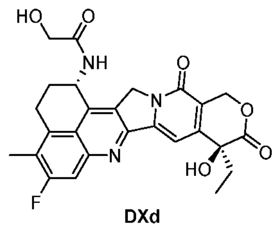

**DXd**

WO2020063673A1 and WO2020063676A1 disclose some exatecan analogs and their ligand-drug conjugates, wherein the exatecan analogs have proliferation inhibitory activity on SK-BR-3 cells and U87 cells.

CN111689980A discloses some exatecan analogs and antibody-drug conjugates thereof, but this application only describes the cellular activities of exatecan analogs and SN38. Most compounds showed activities comparable to SN38 based on the reported activities. For the antibody-drug conjugates, no antibody used for the antibody-drug conjugates was disclosed. It can be seen that this patent application has only made preliminary studies on the activities of exatecan analogs and is silent about the effects in other aspects.

The toxin used in the ADC drug is highly toxic, and the therapeutic window is relatively narrow after the ADC drug is formed. The ADC drug DS-8021, exatecan, co-developed by Daiichi Sankyo, has successfully came into the market. Aiming at the HER2 target, DS-8021 was in the form of 8 toxins linked to a single antibody. However, the antibody-to-drug ratios in the drugs designed in subsequent clinical trials against the Trop2 target were reduced due to safety concerns. However, the reduction in the number of the conjugated drug also reduces the therapeutic efficacy of the ADC drug.

Prodrugs and ADCs require various enzymes and targets, which can cause great individual variability, leading to patient variability in their response to the prodrug, and also being prone to form the toxicity. In order to solve the above problems, the development of highly effective and low toxic antibody-camptothecin derivative ADCs to increase the therapeutic effect is the direction of our research.

The epidermal growth factor receptor (HER) family comprises 4 structurally similar receptor molecules, ErbB1 (EGFR), ErbB2 (HER2), ErbB3 (HER3) and ErbB4 (HER4), which all belong to receptor tyrosine kinases.

The epidermal growth factor receptor (EGFR, also known as HER1, ErbB1) is a transmembrane glycoprotein consisting of 1186 amino acid residues and having the molecular weight of 170kDa and has tyrosine kinase activity. EGFR is expressed in many epithelial tissues, including skin and hair follicle. EGFR is normally monomeric and forms

homo/heterodimers when bound to related ligands such as epidermal growth factor (EGF), transforming growth factor (TGF- $\alpha$), etc.. The dimers phosphorylate, further activating various downstream signaling pathways, promoting cell proliferation, angiogenesis, metastasis, invasion and inhibiting apoptosis. Over-expression of EGFR is found in many solid tumours, such as colorectal caner, head and neck caner, lung caner, ovarian caner, cervical caner, bladder caner, and esophageal caner. The EGFR becomes a good tumour treatment target.

Currently, anti-EGFR drugs mainly include tyrosine kinase inhibitors (TKI), anti-EGFR monoclonal antibodies (mabs), antibody-drug conjugates, and the like. TKI competes with adenosine 5'-triphosphate (ATP) for binding to the intracellular catalytic domain of EGFR, thereby inhibiting the self-phosphorylation and downstream signaling of EGFR. Since EGFR is prone to mutate, therefore, EGFR-TKIs competitive binding has also evolved from the first generation of non-selective reversible binding (represented by Gefitinib, Erlotinib, and Icotinib) to the second generation of non-selective irreversible binding (Afatinib and Dacomitinib), and finally to the third generation of selective irreversible binding (represented by Osimertinib and Almonertinib). The binding ability of EGFR TKI is becoming stronger and stronger, and the selectivity is getting higher and higher. However, other rare EGFR mutations (such as exon 20 insertion mutations) are still a blind spot in treatment, so other treatment methods are needed.

Anti-EGFR monoclonal antibodies or bispecific antibodies block ligand-induced EGFR tyrosine kinase activation by competitively binding to the EGFR extracellular domain with ligands. Domestically approved EGFR monoclonal antibodies include Cetuximab and Nimotuzumab. Clinical data shows that EGFR monoclonal antibodies are only effective for KRAS wild type and do not exhibit tumour inhibition activity against KRAS mutants.

The antibody-drug conjugate is endocytosed by binding to EGFR on the surface of tumour cells, releasing a small molecule toxin to kill the tumour cells. Currently, MRG003 from Meiyake Pharmaceutical Co., Ltd., a subsidiary of Lepu Biopharma, is the first EGFR ADC drug in China to enter the clinical stage. It is conjugated by coupling the humanized anti-EGFR monoclonal antibody JMT101 with the toxin microtubule protein inhibitor MMAE via a degradable mc-vc-PAB (abbreviated as Vc) linker. The clinical results of Phase Ib showed that the objective response rate (ORR) of patients with non-small cell lung cancer was 40% (4/10), and the ORR of patients with nasopharyngeal carcinoma was 44% (4/9), demonstrating good efficacy. However, the patients enrolled in Phase Ib of MRG003 were EGFR-positive and had advanced or metastatic colorectal cancer, head and neck squamous cell carcinoma, or nasopharyngeal carcinoma that had progressed despite multiple treatments. The clinical trial data did not elaborate on whether MRG003 is effective for EGFR-TKI resistant patients (905P FIH phase I dose escalation and dose expansion study of anti-EGFR ADC MRG003 in patients with advanced solid tumours. doi.org/10.1016/j.annonc.2021.08.1315). In order to solve the EGFR-TKI resistance problem, the development of safe and effective ADC drugs is our research direction.

If the target antigen targeted by ADC drugs is widely expressed in normal tissues, it is likely to cause target-mediated on-target toxicity, thereby limiting the clinical development of ADC drugs. In order to reduce the on-target toxicity of ADC drugs as much as possible, conditionally activated ADCs have emerged as a solution to reduce toxicity at the target site. There are many differences between the tumour microenvironment and the normal internal environment of the human body in physicochemical properties. The most significant differences are hypoxia, low PH and high pressure. Due to these characteristics, the immunoinflammatory response generated by a large number of growth factors, cell chemotactic factors and various proteolytic enzymes exists in the tumour microenvironment, and this kind of characteristics are very conducive to tumour proliferation, invasion, adhesion, angiogenesis, and resistance to radiotherapy and chemotherapy. Among others, the tumour undergoes glucolysis under anaerobic conditions and glycolysis produces a large amount of lactic acid, which causes a decrease in pH. At present, the development of the pH-dependent ADC drug has been applied to the clinic. BioAtla company has two ADC projects at the clinical stage, namely AXL-targeted BA3011 and ROR2-targeted BA3021, and early data published by the company show that no obvious target-related toxicity occurs, and the pH-dependent antibody technology can reduce the targeted toxicity of the ADC drug.

The purpose of pH-dependent antibody modification is to make the constructed ADC molecule unable to bind or bind to antigen at a low level in healthy tissue or circulation system after antibody modification, while maintaining the ability to bind to antigen in the tumour microenvironment, thereby reducing the targeted toxicity of the ADC drug and expanding the therapeutic window. The development of efficient and low-toxicity ADC drugs by antibody modification is our research direction.

## Summary of the Invention

The present invention provides an antibody-drug conjugate represented by formula I, and pharmaceutically acceptable salts, hydrates, solvates, stereoisomers or isotopic labels thereof:

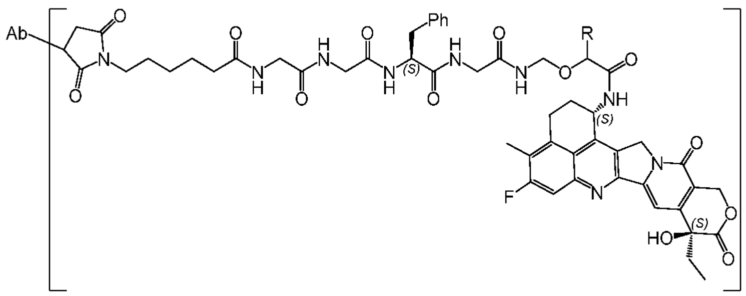

formula I

wherein Ab is an antibody targeting HER2, HER3 or EGFR or an antigen-binding fragment thereof, R is $C_{1-6}$alkyl, and n is an integer of 1-8 or a decimal of 1-8.

Further, n is an integer of 4-8 or a decimal of 4-8.

Further,when n is an integral 1-8, it can be 1, 2, 3, 4, 5, 6, 7, or 8; when n is an integral of 4-8, it can be 4, 5, 6, 7, or 8.

Further, when n is a decimal, it refers to the average number of the linker-drug molecules conjugated per antibody unit.

In a preferable example of the present invention, R is $C_{1-3}$alkyl.

In a preferable example of the present invention, R is methyl, ethyl, propyl, or isopropyl.

In a preferable example of the present invention, R is methyl.

Further, the present invention provides an antibody-drug conjugate represented by formula Ia or formula Ib, and pharmaceutically acceptable salts, hydrates, solvates, stereoisomers or isotopic labels thereof:

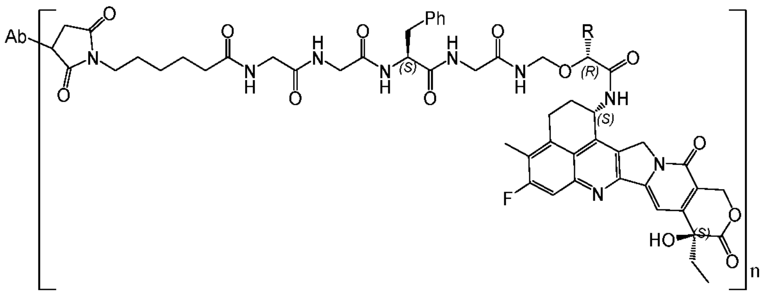

formula Ia

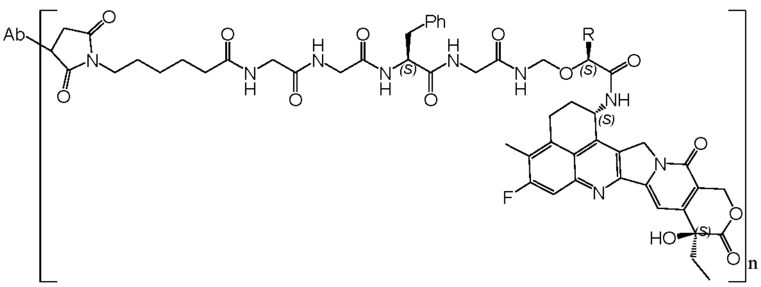

formula Ib

wherein in formula Ia and formula Ib,Ab, R and n are defined as in formula I.

Further, the present invention provides an antibody-drug conjugate represented byformula I-1,formula Ia-1 and formula Ib-1, and pharmaceutically acceptable salts, hydrates, solvates, stereoisomers or isotopic labels thereof:

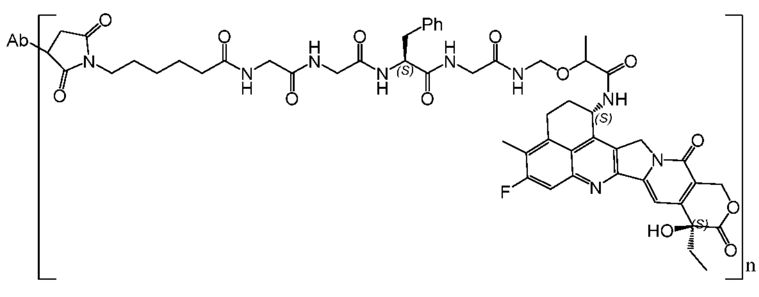

formula I-1

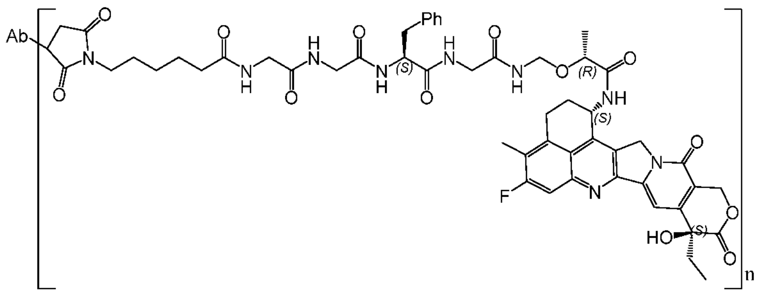

formula Ia-1

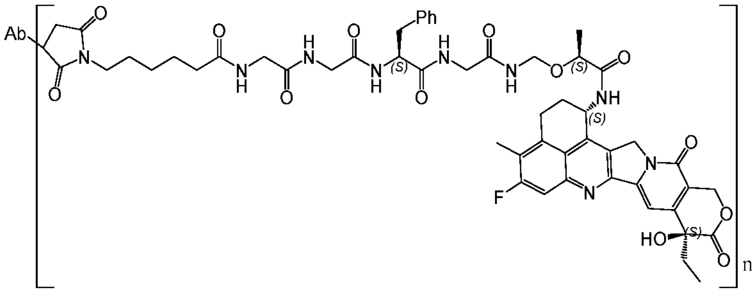

formula Ib-1

wherein informula 1-1, formula Ia-1 and formula Ib-1,Ab and n are defined as in formula I.

On the other hand, the present invention provides an antibody-drug conjugate represented by formula II, and pharmaceutically acceptable salts, hydrates, solvates, stereoisomers or isotopic labels thereof:

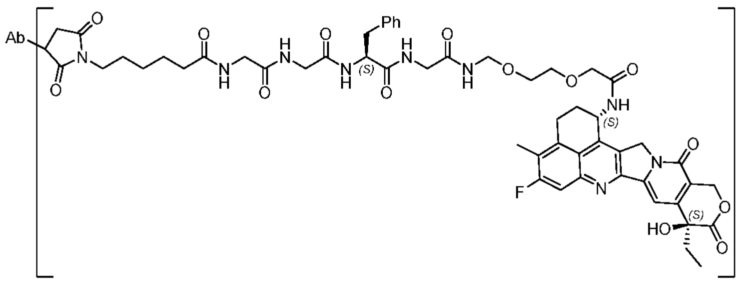

formula II

wherein Ab is an antibody targeting HER2, HER3 or EGFR, n is an integer of 1-8 or a decimal of 1-8.

Further, n is an integer of 4-8 or a decimal of 4-8.

Further, when n is an integral 1-8, it can be 1, 2, 3, 4, 5, 6, 7, or 8; when n is an integral of 4-8, it can be 4, 5, 6, 7, or 8.

Further, when n is a decimal, it refers to the average number of the linker-drug molecules conjugated per antibody unit.

In a preferable example of the present invention, in the above-mentioned antibody-drug conjugate, and pharmaceutically acceptable salts, hydrates, solvates, stereoisomers or isotopic labels thereof, in case that the Ab is an antibody targeting HER2 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:1, the amino acid sequence of HCDR2 is represented by SEQ ID NO:2, the amino acid sequence of HCDR3 is represented by SEQ ID NO:3, and/or the amino acid sequence of LCDR1 is represented by SEQ ID NO:4, the amino acid sequence of LCDR2 is represented by SEQ ID NO:5, the amino acid sequence ofLCDR3 is represented by SEQ ID NO:6.

Further preferably, in case that the Ab is an antibody targeting HER2 or an antigen-binding fragment thereof, it comprises a variable region of a heavy chain (HV) and a variable region of a light chain (LV), wherein the amino acid sequence of HV is represented by SEQ ID NO:7, and the amino acid sequence of LV is represented by SEQ ID NO:8.

More preferably, in case that the Ab is an antibody targeting HER2 or an antigen-binding fragment thereof, it

comprises a heavy chain (HC) and a light chain (LC), wherein the amino acid sequence of HC is represented by SEQ ID NO:9, and the amino acid sequence of of LC is represented by SEQ ID NO: 10.

In a preferable example of the present invention, in case that the Ab is an antibody targeting HER3 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:11, the amino acid sequence of HCDR2 is represented by SEQ ID NO:12, the amino acid sequence of HCDR3 is represented by SEQ ID NO: 13, and/or the amino acid sequence of LCDR1 is represented by SEQ ID NO:14, the amino acid sequence of LCDR2 is represented by SEQ ID NO:15, the amino acid sequence of LCDR3 is represented by SEQ ID NO: 16.

Further preferably, in case that the Ab is an antibody targeting HER3 or an antigen-binding fragment thereof, it comprises a variable region of a heavy chain (HV) and a variable region of a light chain (LV), wherein the amino acid sequence of HV is represented by SEQ ID NO: 17, and the amino acid sequence of LV is represented by SEQ ID NO:18.

More preferably, in case that the Ab is an antibody targeting HER3 or an antigen-binding fragment thereof, it comprises a heavy chain (HC) and a light chain (LC), wherein the amino acid sequence of HC is represented by SEQ ID NO: 19, the amino acid sequence of LC is represented by SEQ ID NO:20.

In a preferable example of the present invention, in case that the Ab is an antibody targeting EGFR or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:21, the amino acid sequence of HCDR2 is represented by SEQ ID NO:22, the amino acid sequence of HCDR3 is represented by SEQ ID NO:23, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

In a preferable example of the present invention, in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:23 or SEQ ID NO:32, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

In a preferable example of the present invention, in case that the Ab is an antibody targeting EGFR or an antigen-binding fragment thereof, it comprises a variable region of a heavy chain (HV) and a variable region of a light chain (LV), wherein the amino acid sequence of HCDR1 is represented by SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:32, and the amino acid sequence of LCDR1 is represented by SEQ ID NO:24, the amino acid sequence of LCDR2 is represented by SEQ ID NO:25, the amino acid sequence of LCDR3 is represented by SEQ ID NO:26.

Further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:27, the amino acid sequence of LV is represented by SEQ ID NO:28.

Further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:33 or SEQ ID NO:36 or SEQ ID NO:39, the amino acid sequence of LV is represented by SEQ ID NO:28.

More preferably, in case that the Ab is an antibody targeting EGFR or an antigen-binding fragment thereof, it comprises a heavy chain (HC) and a light chain (LC), wherein the amino acid sequence of HC is represented by SEQ ID NO:29, the amino acid sequence of LC is represented by SEQ ID NO:30.

More preferably, in case that the Ab is an antibody targeting EGFR or an antigen-binding fragment thereof, it comprises a heavy chain (HC) and a light chain (LC), wherein the amino acid sequence of HC is represented by SEQ ID NO:34 or SEQ ID NO:37 or SEQ ID NO:40, the amino acid sequence of LC is represented by SEQ ID NO:30.

In case that Ab is an antibody targeting HER2, HER3 or EGFR, the antibody is preferably a monoclonal antibody,

and the monoclonal antibody is preferably selected from human-source antibody, humanized antibody, and chimeric antibody.

In case that Ab is an antigen-binding fragment targeting HER2, HER3 or EGFR, the antigen-binding fragment is preferably selected from Fab', (Fab')2, Fab, Fv, scFv, dAb.

In the present invention, the numbering rule of the antibody amino acid sequence adopts the Kabat numbering rule. The present invention also provides any of the above non-conjugated antibodies (Ab) or antigen-binding fragments thereof.

Further, the present invention provides the following antibody-drug conjugates, and pharmaceutically acceptable salts, hydrates, solvates, stereoisomers or isotopic labels thereof:

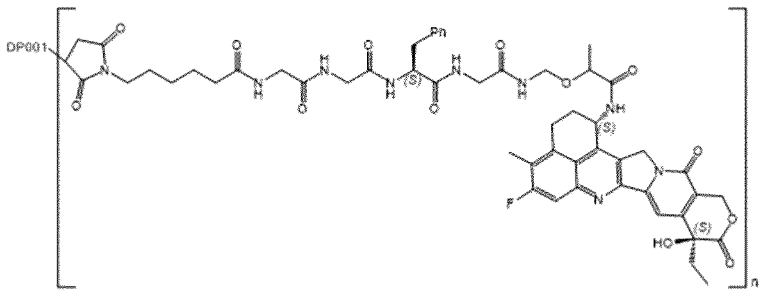

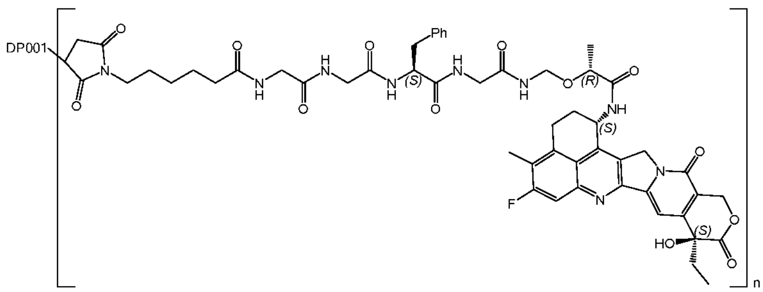

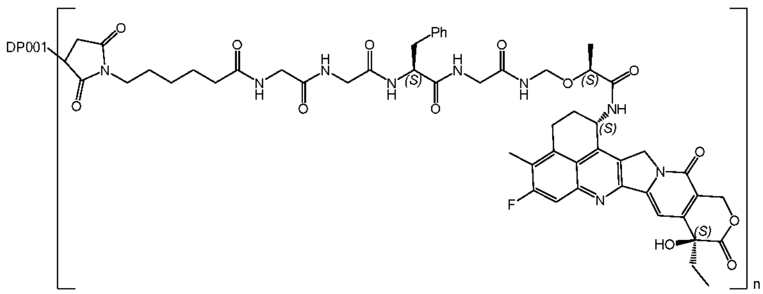

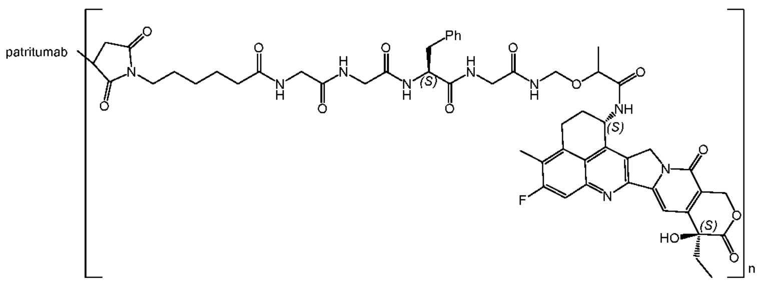

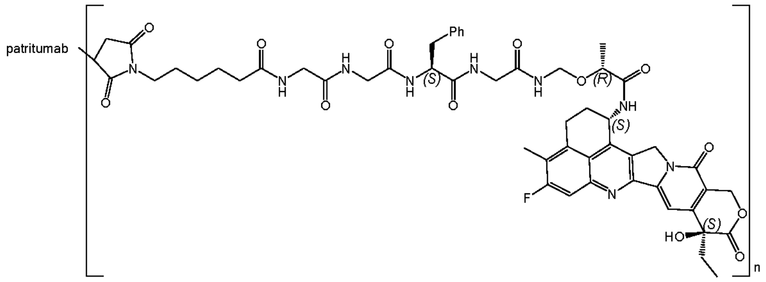

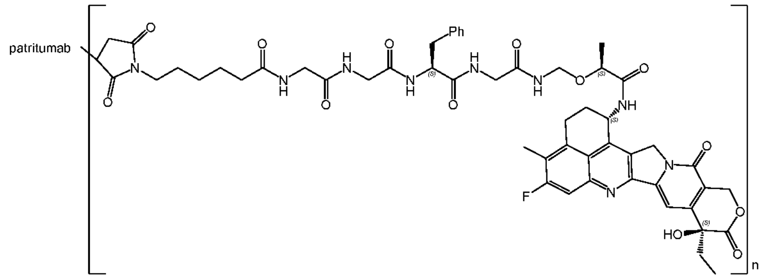

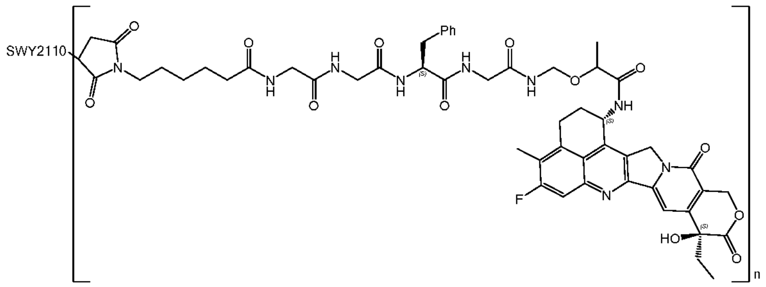

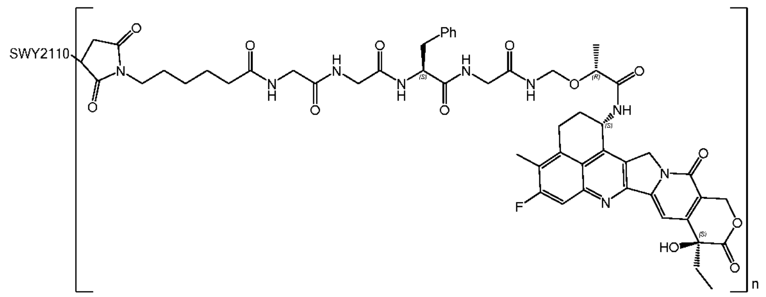

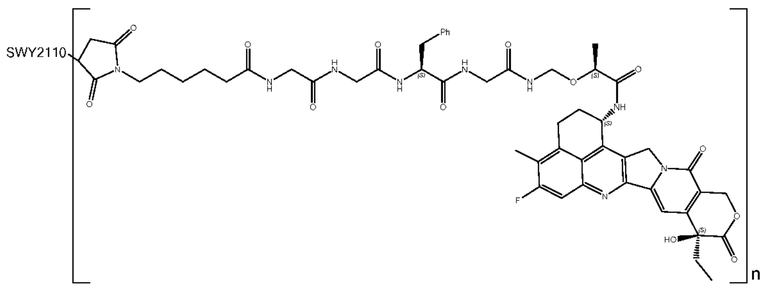

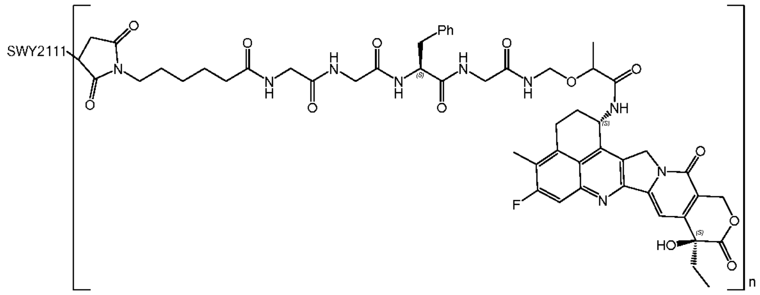

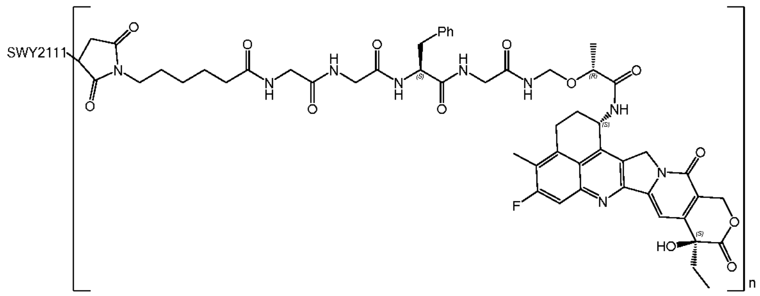

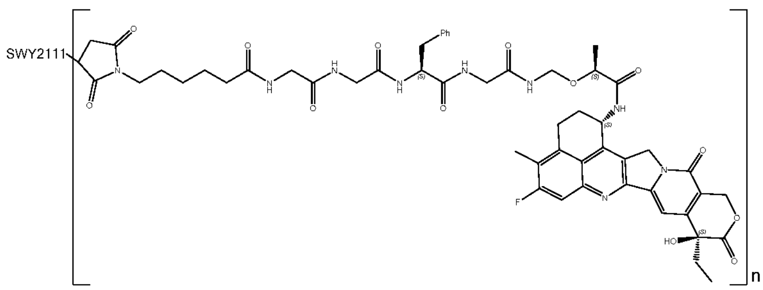

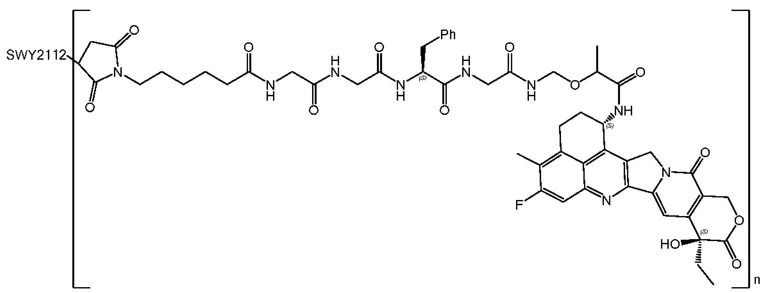

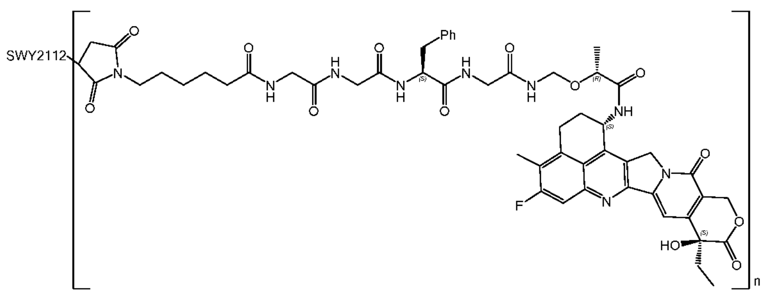

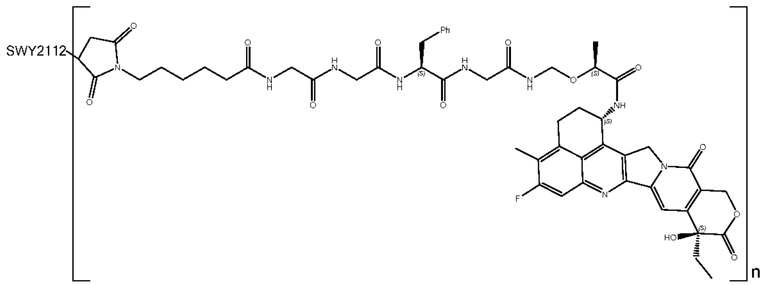

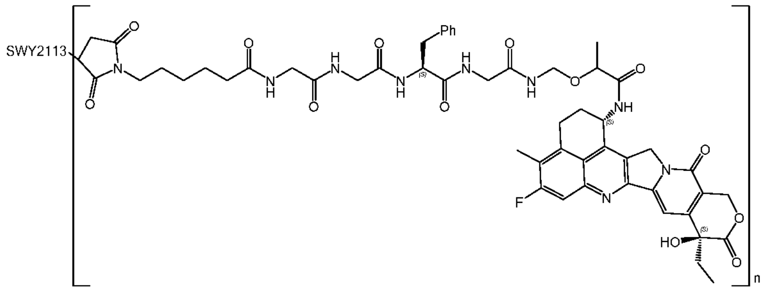

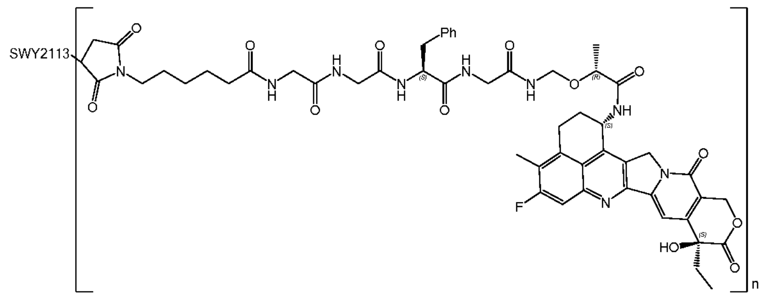

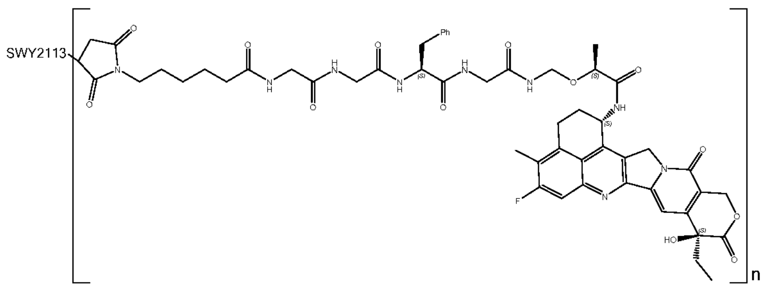

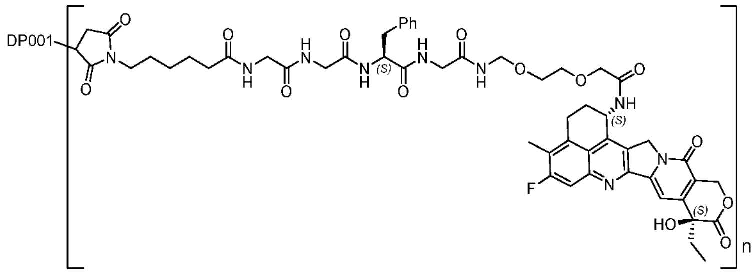

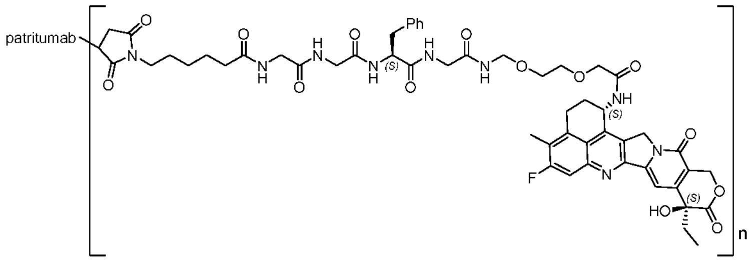

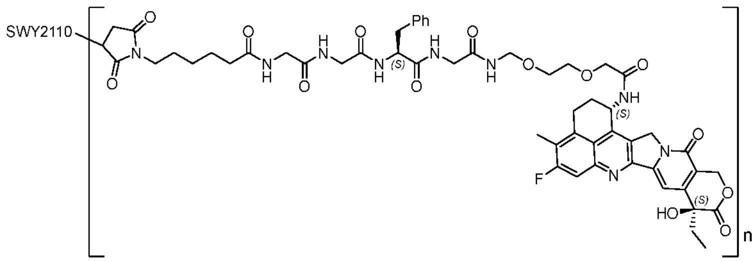

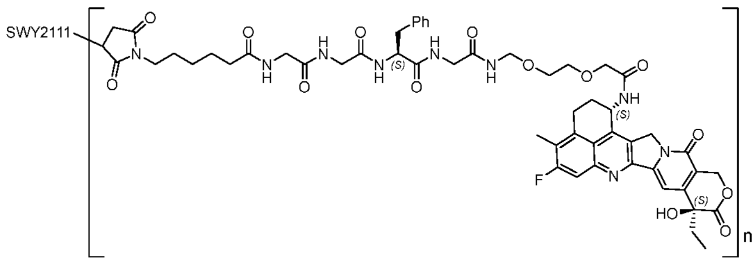

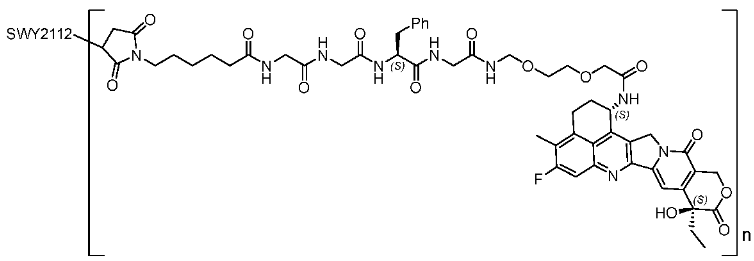

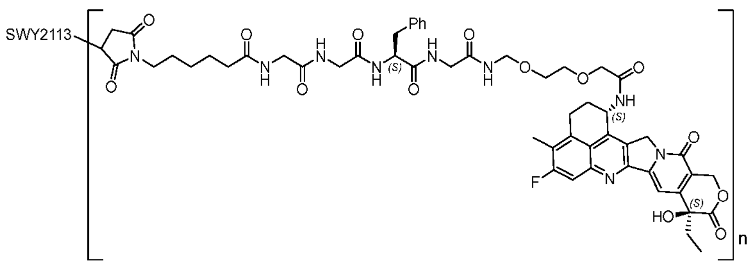

In the above-mentioned antibody-drug conjugates:

DP001 is an antibody having two heavy chains with the amino acid sequence represented by SEQ ID NO:9 and two light chains with the amino acid sequence represented by SEQ ID NO:10;

Patritumab is an antibody having two heavy chains with the amino acid sequence represented by SEQ ID NO:19 and two light chains with the amino acid sequence represented by SEQ ID NO:20;

SWY2110 is an antibody having two heavy chains with the amino acid sequence represented by SEQ ID NO:29 and two light chains with the amino acid sequence represented by SEQ ID NO:30;

SWY2111 is an antibody having two heavy chains with the amino acid sequence represented by SEQ ID NO:34 and two light chains with the amino acid sequence represented by SEQ ID NO:30;
SWY2112 is an antibody having two heavy chains with the amino acid sequence represented by SEQ ID NO:37 and two light chains with the amino acid sequence represented by SEQ ID NO:30;
SWY2113 is an antibody having two heavy chains with the amino acid sequence represented by SEQ ID NO:40 and two light chains with the amino acid sequence represented by SEQ ID NO:30;
n is an integer of 1-8 or a decimal of 1-8.

Further, n is an integer of 4-8 or a decimal of 4-8.
Further, when n is an integral 1-8, it can be 1, 2, 3, 4, 5, 6, 7, or 8; when n is an integral of 4-8, it can be 4, 5, 6, 7, or 8.
On the other hand, the present invention provides a linker-drug compound represented by formula III, and pharmaceutically acceptable salts, hydrates, solvates, stereoisomers or isotopic labels thereof:

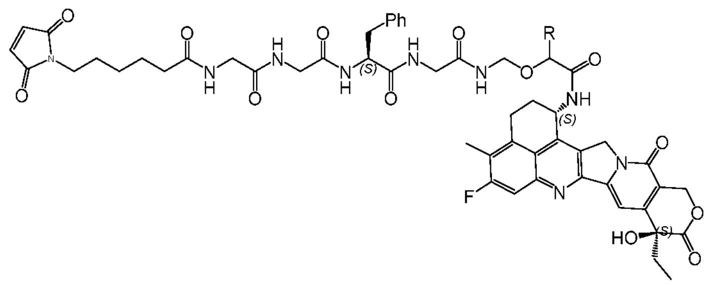

formula III

wherein R is defined as in formula I.
Further, the present invention provides linker-drug compounds represented by formula IIIa and formula IIIb, and pharmaceutically acceptable salts, hydrates, solvates, stereoisomers or isotopic labels thereof:

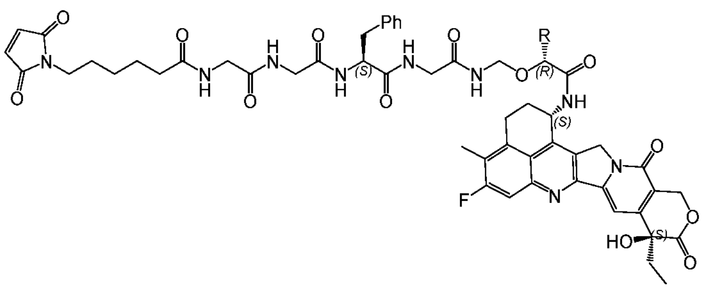

formula IIIa

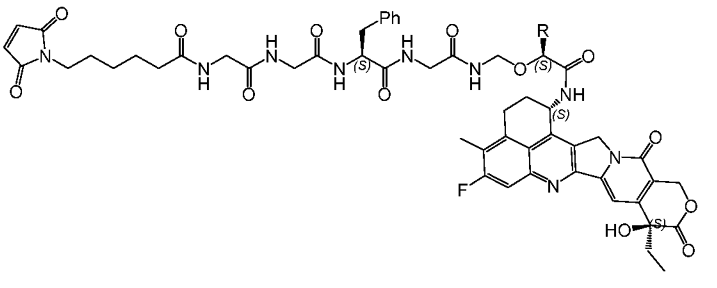

formula IIIb

wherein in formula IIIa and formula IIIb, R is defined as in formula III.
Further, the present invention provides the following linker-drug compounds, and pharmaceutically acceptable salts, hydrates, solvates, stereoisomers or isotopic labels thereof:

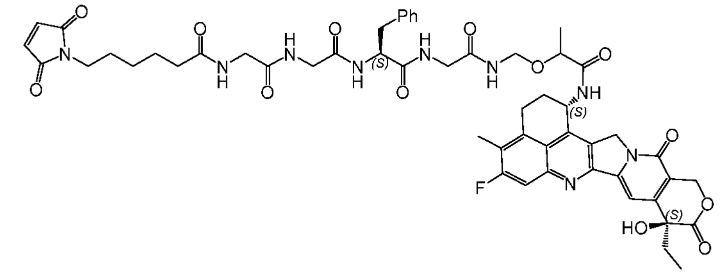

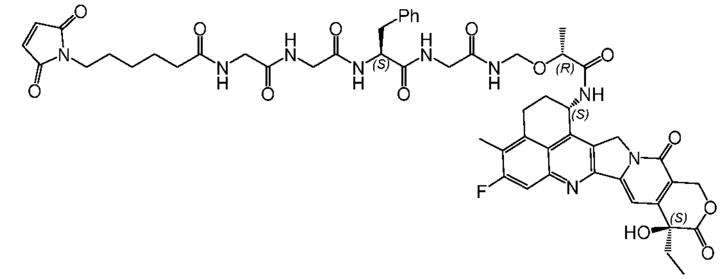

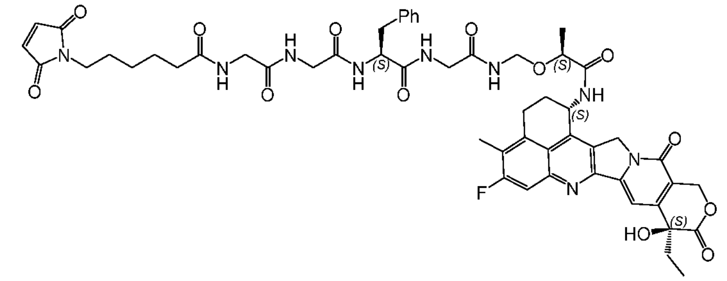

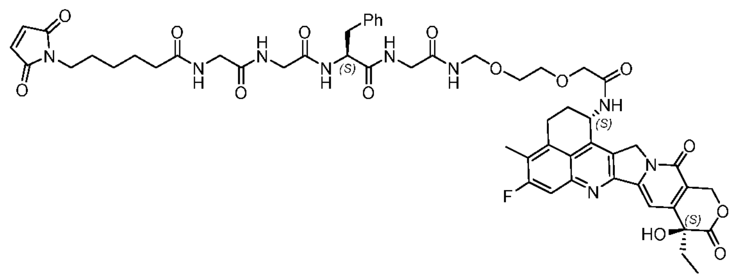

Further, the present invention provides a pharmaceutical composition, which contains the antibody-drug conjugate of the present invention, and pharmaceutically acceptable salts, hydrates, solvates, stereoisomers or isotopic labels thereof. The pharmaceutical composition further comprises pharmaceutically acceptable adjuvants and carriers. The present invention provides use of the antibody-drug conjugate mentioned in the present invention and pharmaceutically acceptable salts, hydrates, solvates, stereoisomers or isotopic labels thereof in manufacture of a medicament for treating proliferative diseases. The proliferative disease is preferably a disease related to abnormal expression of HER2, HER3 or EGFR, including cancer. The cancer is selected from breast cancer, ovarian cancer, cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma).

The present invention provides a method for treating or preventing proliferative diseases, which method includes administering a therapeutically effective dose of the antibody-drug conjugate of the present invention, and pharmaceutically acceptable salts, hydrates, solvates, stereoisomers or isotopic labels thereof or a pharmaceutical composition containing the same to a patient in need thereof; the proliferative disease is preferably a disease related to abnormal expression of HER2, HER3 or EGFR, including cancer. The cancer is selected from breast cancer, ovarian cancer,

cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma).

The present invention provides use of the antibody-drug conjugate mentioned in the present invention and pharmaceutically acceptable salts, hydrates, solvates, stereoisomers or isotopic labels thereof in manufacture of a medicament for treating drug-resistant proliferative diseases. The drug-resistant proliferative diseases is a drug-resistant disease related to abnormal expression of HER2, HER3 or EGFR, including cancer. The drug-resistant cancer is preferably a drug-resistant cancer caused by mutation in HER2, HER3 or EGFR genes. The cancer is preferably breast cancer, ovarian cancer, cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma), further preferably colon cancer, rectal cancer, lung cancer or pancreas cancer. Among others, the drug resistance preferably refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib. The present invention provides a method for treating or preventing drug-resistant proliferative diseases, which method includes administering a therapeutically effective dose of the antibody-drug conjugate of the present invention, and pharmaceutically acceptable salts, hydrates, solvates, stereoisomers or isotopic labels thereof or a pharmaceutical composition containing the same to a patient in need thereof; the drug-resistant proliferative diseases is a drug-resistant disease related to abnormal expression of HER2, HER3 or EGFR, including cancer. The drug-resistant cancer is preferably a drug-resistant cancer caused by mutation in HER2, HER3 or EGFR genes. The cancer is preferably breast cancer, ovarian cancer, cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma), further preferably colon cancer, rectal cancer, lung cancer or pancreas cancer. Among others, the drug resistance preferably refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib.

The present invention provides use of the linker-drug compound and pharmaceutically acceptable salts, hydrates, solvates, stereoisomers or isotopic labels thereof in preparation of the antibody-drug conjugate.

The antibody-drug conjugate of the present invention can be used alone or in combination with other anti-tumour agents.

The present invention further provides a pharmaceutical composition, which contains the antibody-drug conjugate of the present invention and pharmaceutically acceptable salts, hydrates, solvates, stereoisomers or isotopic labels thereof, and other anti-tumour agents.

The present invention provides a process for preparing the antibody-drug conjugate mentioned in the present invention and pharmaceutically acceptable salts, hydrates, solvates, stereoisomers or isotopic labels thereof, which comprises the following steps:

S1. Reduction of antibody;
Specifically, the antibody medium is replaced with PBS6.0/EDTA to prepare a solution having an antibody concentration of 10 mg/mL. An appropriate amount of 10mM aqueous TCEP solution and 1M aqueous dipotassium hydrogen phosphate solution are added according to the reduction amount. After confirming that the pH of the solution is proper, the incubation at 37°C is performed for several hours to reduce the disulfide bonds in the antibody. S2: Conjugation of antibody and linker-drug compound;
Specifically an appropriate amount of a solution (10mM) of the compound dissolved in dimethyl sulfoxide is added to the above solution at room temperature, and the mixture is mixed evenly and reacted at room temperature for 0.5-4 hours to attach the linker-drug compound to the antibody. Next, 100mM aqueous NAC solution is added, and

the mixture is further stirred at room temperature to terminate the reaction.

S3. Purification of antibody-drug conjugates:

Specifically, the reaction solution is purified by ultrafiltration using ultrafiltration centrifuge tubes. 25mM 2-(N-morpholine)ethanesulfonic acid (MES) is added as purification buffer to the reaction solution, pH=6.5. The resulting solution is concentrated to 1-2mL, the purification buffer is added again to make the replacement factor greater than 1000 times. Thus, the non-conjugated linker-drug compound and other low molecular weight reagents are removed to obtain the purified antibody-drug conjugate.

Compared with known antibody-drug conjugates in the prior art such as DS-8201 or current laboratory samples with better effects on different targets as controls, the antibody-drug conjugates of the present invention show better anti-cell proliferation effect and significant tumour inhibition effect. For drug-resistant tumours such as those caused by mutation in HER2, HER3 or EGFR genes, especially those resistant to anti-tumour agents of receptor tyrosine kinase inhibitors, the antibody-drug conjugates of the present invention have superior inhibitory activity to DS-8201, have better stability, including plasma stability and buffer stability, have lower toxicity, have better bystander effect, and have a wider therapeutic window. The present invention carries out the pH-dependent modification of the antibody, and the ADC obtained by conjugating the modified antibody with the linker-drug compound has comparable tumour inhibitory activity to the original ADC in terms of efficacy, and it shows reduced on-target toxicity to normal tissues in terms of safety. The ADC obtained after antibody optimization further expands the therapeutic window.

Definition

Unless otherwise specified, the term "antibody-drug conjugate" refers to the linkage of an antibody (such as a monoclonal antibody) or an antibody fragment to a biologically active cytotoxin drug through a stable chemical linker compound.

Unless otherwise specified, the term "linker-drug compound" refers to a partial structure in the "antibody-drug conjugate", which is composed of the linker compound and the drug compound.

In the present invention, the linker-drug compound and the antibody are attached by conjugation manners conventionally in the art, including lysine conjugation, reductive disulfide bond conjugation between light and heavy chains, and directional conjugation (Beck, Alain, and Janice M. Reichert. "Antibody-drug conjugates: present and future." MAbs. Vol. 6. No. 1. Taylor & Francis, 2014.; McCombs, Jessica R., and Shawn C. Owen. "Antibody drug conjugates: design and selection of linker, payload and conjugation chemistry." The AAPS journal 17 (2015): 339-351.). In the present invention, the reductive disulfide bond conjugation between the light and heavy chains is preferred, which involves the attachment through the reaction of thiol groups (sulfur atoms of cysteine residues) formed after reducing one or more of disulfide bond sites between the light and heavy chains (two sites between the heavy chains, and two sites between the heavy and light chain).

Unless otherwise specified, the term "alkyl" refers to a monovalent saturated aliphatic hydrocarbon group, including a linear or branched group containing 1-20 carbon atoms, preferably containing 1-10 carbon atoms (namely $C_{1-10}$alkyl), further preferably containing 1-8 carbon atoms ($C_{1-8}$alkyl), more preferably containing 1-6 carbon atoms (namely $C_{1-6}$alkyl). For example "$C_{1-6}$alkyl" means that the group is an alkyl group, and the number of carbon atoms in the carbon chain is between 1 and 6 (specifically 1, 2, 3, 4, 5 or 6). Examples include, but are not limited to methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl and the like.

Unless specified otherwise, the term "pharmaceutically acceptable salt" or the similar expression refers to a salt that is suitable for use in contact with the tissues of mammals, especially humans, without undue toxicity, irritation, allergic response or the like, and commensurates with a reasonable benefit/risk ratio within the range of reasonable medical judgment. For example, the pharmaceutically acceptable salts of amines, carboxylic acids and other types of compounds are well known in the art. The salts can be prepared in situ during the final isolation and purification of the compounds according to the present invention, or separately by reacting the free base or acid with a suitable reagent.

Unless otherwise specified, the term "isotopic label" means that the compounds of the present invention can exist in isotope tagged or enriched form containing one or more atoms having an atomic mass or mass number different from the atomic mass or mass number most abundantly found in nature. Isotopes can be radioactive or nonradioactive isotopes. Isotopes commonly used as isotopic labels are: hydrogen isotopes: $^{2}H$ and $^{3}H$; carbon isotopes: $^{13}C$ and $^{14}C$; chlorine isotopes: $^{35}Cl$ and $^{37}Cl$; fluorine isotope: $^{18}F$; iodine isotopes: $^{123}I$ and $^{125}I$; nitrogen isotopes: $^{13}N$ and $^{15}N$; oxygen isotopes: $^{15}O$, $^{17}O$ and $^{18}O$; and sulfur isotope: $^{35}S$. These isotope labelled compounds can be used to study the distribution of pharmaceutical molecules in tissues. In particular, $^{2}H$ and $^{13}C$ are more widely used due to their ease of labeling and ease of detection. The substitution with certain heavy isotopes, such as heavy hydrogen ($^{2}H$), can enhance the stability of metabolism, and prolong the half-life, so as to achieve the purpose of reducing dosage and providing therapeutic advantages. Isotope labelled compounds are generally synthesized starting from labelled starting materials

in the same way as non-isotope labelled compounds using known synthetic techniques.

Unless specified otherwise, the term "solvate" or the similar expression refers to a physical association of a compound according to the present invention with one or more solvent molecules, regardless of organic or inorganic. This physical association includes hydrogen bond. In some cases, for example, when one or more solvent molecules are incorporated into the crystal lattice of a crystalline solid, the solvates can be isolated. Solvent molecules in the solvates may exist in regular and/or disordered arrangements. The solvates may contain stoichiometric or non-stoichiometric amounts of solvent molecules. The "solvate" encompasses both solution phase and isolatable solvate. Exemplary solvates include, but are not limited to, hydrates, ethanolates, methanolates, and isopropanolates. Solvation methods are well known in the art.

Unless otherwise specified, the term "stereoisomers" refers to compounds which have identical chemical constitution, but differ with regard to the arrangement of the atoms or groups in space. Stereoisomers include enantiomer, diastereomers, conformer (rotamer), geometric (cis/trans) isomer, atropisomer, etc. Any resulting mixtures of stereoisomers can be separated on the basis of the physicochemical differences of the constituents, into the pure or substantially pure geometric isomers, enantiomers, diastereomers, for example, by chromatography and/or fractional crystallization.

**Description of the drawings**

Figure 1 shows the decection result for the DAR value of DP001-JSSW-001.
Figure 2 shows the decection result for the DAR value of DP001-ZW-002.
Figure 3 shows the decection result for the DAR value of DP001-JSSW-001.
Figure 4 shows the decection result for the DAR value of partitumab-JSSW-001.
Figure 5 shows the decection result for the DAR value of partitumab-ZW-002.
Figure 6 shows the decection result for the DAR value of SWY2110-JSSW-001.
Figure 7 shows the decection result for the DAR value of SWY2111-JSSW-001.
Figure 8 shows the decection result for the DAR value of SWY2112-JSSW-001.
Figure 9 shows the decection result for the DAR value of SWY2113-JSSW-001.
Figure 10 shows the decection result for the DAR value of partitumab-Dxd.
Figure 11 shows the decection result for the DAR value of SWY2110-Dxd.
Figure 12 shows the decection result for the DAR value of SWY2110-Vc MMAE.
Figure 13 shows the effect of DP001-ADC on the tumour volume of HER2-expressing human breast cancer JIMT-1 xenograft tumour in NU/NU mice.
Figure 14 shows the effect of patritumab-ADC on the tumour volume of human lung adenocarcinoma PC9-GR (gefitinib-resistant cell) xenograft tumour in NU/NU mice.
Figure 15 shows the effect of SWY2110-ADC on the tumour volume of human colorectal cancer DiFi xenograft tumour in NU/NU mice.
Figure 16 shows the effect of SWY2110-ADC on the tumour volume of human lung adenocarcinoma PC9-GR (gefitinib-resistant cell) xenograft tumour in NU/NU mice.
Figure 17 shows the detection result of the inhibitory activity of SWY2110-ADC on DiFi cells.
Figure 18 shows the detection result of the inhibitory activity of SWY2110-ADC on PC9-GR cells
Figure 19 shows the detection result of the bystander effect of DP001-ADC.
Figure 20 shows the effect of SWY2110-ADC on the tumour volume of human lung adenocarcinoma PC9-AR (PC9-Del19/T790M/C797S, osimertinib-resistant cell) xenograft tumour in NU/NU mice.
Figure 21 shows the effect of SWY2110-ADC, SWY2111-ADC, SWY2112-ADC, SWY2113-ADC on the tumour volume of human lung adenocarcinoma NCI-H1975 xenograft tumour in NU/NU mice.
Figure 22 shows the effect of SWY2110-ADC, SWY2111-ADC, SWY2112-ADC, SWY2113-ADC on the tumour volume of human breast cancer MDA-MB-468 xenograft tumour in NOD-SCID mice.
Figure 23 shows the effect of SWY2110-ADC, SWY2111-ADC, SWY2112-ADC, SWY2113-ADC on the tumour volume of human colorectal cancer DiFi xenograft tumour in NU/NU mice.
Figure 24 shows the effect of SWY2110-ADC, SWY2111-ADC, SWY2112-ADC, SWY2113-ADC on the tumour volume of human lung adenocarcinoma PC9-GR (gefitinib-resistant cell) xenograft tumour in NU/NU mice.
Figure 25 shows the effect of patritumab-ADC on the tumour volume of human lung adenocarcinoma PC9-DTC (Del19/T790M/C797S, osimertinib-resistant cell) xenograft tumour in NOD-SCID mice.
Figure 26 shows the effect of patritumab-ADC on the tumour volume of human colon cancer SW620 xenograft tumour in NU/NU mice.
Figure 27 shows the detection result of the inhibitory activity of patritumab-ADC on DiFi cells.

## Detailed description

The present invention is further described below in connection with specific examples. It should be understood that these examples are intended to illustrate the present invention only and are not intended to limit the scope of the present invention. Experimental methods for which no specific conditions are indicated in the following examples are generally in accordance with conventional conditions or in accordance with the conditions recommended by the manufacturers. Unless otherwise defined, all professional and scientific terms used herein have the same meaning as those familiar to those skilled in the art. In addition, any methods and materials similar or equivalent to those described herein may be used in the methods of the present invention. The preferred embodiments and materials shown in this disclosure are illustrative only.

The structures of the compounds according to the present invention are determined by nuclear magnetic resonance (NMR) or/and liquid chromatograph mass spectrometry (LC-MS) or/and liquid chromatography (HPLC). The instrument used for NMR is Bruker Avance III 400 MHz nuclear magnetic resonance apparatus; the instrument used for LC-MS is SHIMADZU LC-20AD-PDA-LCMS-2020; and the instrument used for HPLC is SHIMADZU LC-20AD-PDA high performance liquid chromatograph.

The starting materials in the examples of the present invention are known and commercially available, or can be synthesised using those methods known in the art or according to those methods known in the art.

The antibodies of the present invention can be prepared by hybridoma technology described by Kohler et al., Nature (1975) for the first time, or by recombinant DNA method (U.S. Patent 4,816,567, etc.).

Preparation Example

An antibody having two heavy chains with the amino acid sequence represented by SEQ ID NO:9 and two light chains with the amino acid sequence represented by SEQ ID NO: 10 was defined as DP001;

An antibody having two heavy chains with the amino acid sequence represented by SEQ ID NO:19 and two light chains with the amino acid sequence represented by SEQ ID NO:20 was defined as patritumab;
An antibody having two heavy chains with the amino acid sequence represented by SEQ ID NO:29 and two light chains with the amino acid sequence represented by SEQ ID NO:30 was defined as SWY2110;
An antibody having two heavy chains with the amino acid sequence represented by SEQ ID NO:34 and two light chains with the amino acid sequence represented by SEQ ID NO:30 was defined as SWY2111;
An antibody having two heavy chains with the amino acid sequence represented by SEQ ID NO:37 and two light chains with the amino acid sequence represented by SEQ ID NO:30 was defined as SWY2112;
An antibody having two heavy chains with the amino acid sequence represented by SEQ ID NO:40 and two light chains with the amino acid sequence represented by SEQ ID NO:30 was defined as SWY2113.

Antibody Sequence Description

1. HER2 antibody DP001 (trastuzumab)

Light chain (SEQ ID NO:10):

DIQMTQSPSSLSASVGDRVTITCRASQDVNTAVAWYQQKPGKAPKLLIYSASFLYSGVPSRFSGSRSGTDF
TLTISSLQPEDFATYYCQQHYTTPPTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C

Heavy chain (SEQ ID NO:9):

EVQLVESGGGLVQPGGSLRLSCAASGFNIKDTYIHWVRQAPGKGLEWVARIYPTNGYTRYADSVKGRFTI SADTSKNTAYLQMNSLRAEDTAVYYCSRWGGDGFYAMDYWGQGTLVTVSSASTKGPSVFPLAPSSKSTS GGTAALGCLVKDYFPEPVTVSWNSGALTSGVHTFPAVLQSSGLYSLSSVVTVPSSSLGTQTYICNVNHKPS NTKVDKKVEPKSCDKTHTCPPCPAPELLGGPSVFLFPPKPKDTLMISRTPEVTCVVVDVSHEDPEVKFNW YVDGVEVHNAKTKPREEQYNSTYRVVSVLTVLHQDWLNGKEYKCKVSNKALPAPIEKTISKAKGQPREP QVYTLPPSREEMTKNQVSLTCLVKGFYPSDIAVEWESNGQPENNYKTTPPVLDSDGSFFLYSKLTVDKSR WQQGNVFSCSVMHEALHNHYTQKSLSLSPGK

DP001 HCDR1: GFNIKDTYIH (SEQ ID NO:1)
DP001 HCDR2: RIYPTNGYTRYAD (SEQ ID NO:2)
DP001 HCDR3: WGGDGFYAMDY (SEQ ID NOG)
DP001 LCDR1: RASQDVNTAVA (SEQ ID NO:4)
DP001 LCDR2: SASFLYS (SEQ ID NO:5)
DP001 LCDR3: QQHYTTPPT (SEQ ID NO:6)
DP001 HV: SEQ ID NO:7

EVQLVESGGGLVQPGGSLRLSCAASGFNIKDTYIHWVRQAPGKGLEWVARIYPTNGYTRYADSVKGRFTI SADTSKNTAYLQMNSLRAEDTAVYYCSRWGGDGFYAMDYWGQGTLVTVSS

DP001 LV: SEQ ID NO:8

DIQMTQSPSSLSASVGDRVTITCRASQDVNTAVAWYQQKPGKAPKLLIYSASFLYSGVPSRFSGSRSGTDF TLTISSLQPEDFATYYCQQHYTTPPTFGQGTKVEIK

2. HER3 antibody patritumab

Light chain (SEQ ID NO:20):

DIEMTQSPDSLAVSLGERATINCRSSQSVLYSSSNRNYLAWYQQNPGQPPKLLIYWASTRESGVPDRFSGS GSGTDFTLTISSLQAEDVAVYYCQQYYSTPRTFGQGTKVEIKRTVAAPSVFIFPPSDEQLKSGTASVVCLLN NFYPREAKVQWKVDNALQSGNSQESVTEQDSKDSTYSLSSTLTLSKADYEKHKVYACEVTHQGLSSPVT KSFNRGEC

Heavy chain (SEQ ID NO:19):

QVQLQQWGAGLLKPSETLSLTCAVYGGSFSGYYWSWIRQPPGKGLEWIGEINHSGSTNYNPSLKSRVTIS VETSKNQFSLKLSSVTAADTAVYYCARDKWTWYFDLWGRGTLVTVSSASTKGPSVFPLAPSSKSTSGGTA ALGCLVKDYFPEPVTVSWNSGALTSGVHTFPAVLQSSGLYSLSSVVTVPSSSLGTQTYICNVNHKPSNTKV DKRVEPKSCDKTHTCPPCPAPELLGGPSVFLFPPKPKDTLMISRTPEVTCVVVDVSHEDPEVKFNWYVDG

VEVHNAKTKPREEQYNSTYRVVSVLTVLHQDWLNGKEYKCKVSNKALPAPIEKTISKAKGQPREPQVYT LPPSREEMTKNQVSLTCLVKGFYPSDIAVEWESNGQPENNYKTTPPVLDSDGSFFLYSKLTVDKSRWQQG NVFSCSVMHEALHNHYTQKSLSLSPGK

Patritumab HCDR1: GGSFSGYYWS SEQ ID NO:11

Patritumab HCDR2: EINHSGSTNYNPSLKS SEQ ID NO:12
Patritumab HCDR3: DKWTWYFDL SEQ ID NO:13
Patritumab LCDR1: RSSQSVLYSSSNRNYLA SEQ ID NO:14
Patritumab LCDR2: WASTRES SEQ ID NO:15
Patritumab LCDR3: QQYYSTPRT SEQ ID NO:16
Patritumab HV: SEQ ID NO:17

QVQLQQWGAGLLKPSETLSLTCAVYGGSFSGYYWSWIRQPPGKGLEWIGEINHSGSTNYNPSLKSRVTIS
VETSKNQFSLKLSSVTAADTAVYYCARDKWTWYFDLWGRGTLVTVSS

Patritumab LV: SEQ ID NO:18

DIEMTQSPDSLAVSLGERATINCRSSQSVLYSSSNRNYLAWYQQNPGQPPKLLIYWASTRESGVPDRFSGS
GSGTDFTLTISSLQAEDVAVYYCQQYYSTPRTFGQGTKVEIK

3. EGFR antibody SWY2110

Light chain (SEQ ID NO:30):

EIVLTQSPDFQSVTPKEKVTITCRASQSIGTNIHWYQQKPDQSPKLLIKYASESISGIPSRFSGSGSGTDFTLTI
NSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDSTYSLSS
TLTLSKADYEKHKVYACEVTHQGLSSPVTKSFNRGEC

Heavy chain (SEQ ID NO:29):

QVQLQESGPGLVKPSETLSLTCTVSGFSLSNYDVHWVRQAPGKGLEWLGVIWSGGNTDYNTPFTSRLTIS
VDTSKNQFSLKLSSVTAADTAVYYCARALDYYDYEFAYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSNT
KVDKRVEPKSCDKTHTCPPCPAPELLGGPSVFLFPPKPKDTLMISRTPEVTCVVVDVSHEDPEVKFNWYV
DGVEVHNAKTKPREEQYNSTYRVVSVLTVLHQDWLNGKEYKCKVSNKALPAPIEKTISKAKGQPREPQV
YTLPPSREEMTKNQVSLTCLVKGFYPSDIAVEWESNGQPENNYKTTPPVLDSDGSFFLYSKLTVDKSRWQ
QGNVFSCSVMHEALHNHYTQKSLSLSPGK

SWY2110 HCDR1: NYDVH SEQ ID NO:21
SWY2110 HCDR2: VIWSGGNTDYNTPFTS SEQ ID NO:22
SWY2110 HCDR3: ALDYYDYEFAY SEQ ID NO:23
SWY2110 LCDR1: RASQSIGTNIH SEQ ID NO:24
SWY2110 LCDR2: YASESIS SEQ ID NO:25
SWY2110 LCDR3: QQNNEWPTS SEQ ID NO:26
SWY2110 HV: SEQ ID NO:27

QVQLQESGPGLVKPSETLSLTCTVSGFSLSNYDVHWVRQAPGKGLEWLGVIWSGGNTDYNTPFTSRLTIS
VDTSKNQFSLKLSSVTAADTAVYYCARALDYYDYEFAYWGQGTLVTVSS

SWY2110 LV: SEQ ID NO:28

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK

4. EGFR antibody SWY2111

Light chain (SEQ ID NO:30):

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK RTVAAPSVFIFPPSDEQLKSGTASVVCLLNNFYPREAKVQWKVDNALQSGNSQESVTEQDSKDSTYSLSS TLTLSKADYEKHKVYACEVTHQGLSSPVTKSFNRGEC

Heavy chain (SEQ ID NO:34):

QVQLQESGPGLVKPSETLSLTCTVSGFSLSDYDVHWVRQAPGKGLEWLGVIWSGGNTDYNTPFTSRLTIS VDTSKNQFSLKLSSVTAADTAVYYCARALDDYDYEFAYWGQGTLVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKRVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLYSKLTVDKSRWQ QGNVFSCSVMHEALHNHYTQKSLSLSPGK

SWY2111 HCDR1: DYDVH SEQ ID NO:31
SWY2111 HCDR2: VIWSGGNTDYNTPFTS SEQ ID NO:22
SWY2111 HCDR3: ALDDYDYEFAY SEQ ID NO:32
SWY2111 LCDR1: RASQSIGTNIH SEQ ID NO:24
SWY2111 LCDR2: YASESIS SEQ ID NO:25
SWY2111 LCDR3: QQNNEWPTS SEQ ID NO:26
SWY2111 HV: SEQ ID NO:33

QVQLQESGPGLVKPSETLSLTCTVSGFSLSDYDVHWVRQAPGKGLEWLGVIWSGGNTDYNTPFTSRLTIS VDTSKNQFSLKLSSVTAADTAVYYCARALDDYDYEFAYWGQGTLVTVSS

SWY2111 LV: SEQ ID NO:28

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK

5. EGFR antibody SWY2112

Light chain (SEQ ID NO:30):

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK RTVAAPSVFIFPPSDEQLKSGTASVVCLLNNFYPREAKVQWKVDNALQSGNSQESVTEQDSKDSTYSLSS TLTLSKADYEKHKVYACEVTHQGLSSPVTKSFNRGEC

Heavy chain (SEQ ID NO:37):

QVQLQESGPGLVKPSETLSLTCTVSGFSLSEYDVHWVRQAPGKGLEWLGVIWSGGNTDYNTPFTSRLTIS VDTSKNQFSLKLSSVTAADTAVYYCARALDDYDYEFAYWGQGTLVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKRVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLYSKLTVDKSRWQ QGNVFSCSVMHEALHNHYTQKSLSLSPGK

SWY2112 HCDR1: EYDVH SEQ ID NO:35
SWY2112 HCDR2: VIWSGGNTDYNTPFTS SEQ ID NO:22
SWY2112 HCDR3: ALDDYDYEFAY SEQ ID NO:32
SWY2112 LCDR1: RASQSIGTNIH SEQ ID NO:24
SWY2112 LCDR2: YASESIS SEQ ID NO:25
SWY2112 LCDR3: QQNNEWPTS SEQ ID NO:26
SWY2112 HV: SEQ ID NO:36

QVQLQESGPGLVKPSETLSLTCTVSGFSLSEYDVHWVRQAPGKGLEWLGVIWSGGNTDYNTPFTSRLTIS VDTSKNQFSLKLSSVTAADTAVYYCARALDDYDYEFAYWGQGTLVTVSS

SWY2112 LV: SEQ ID NO:28

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK

6. EGFR antibody SWY2113

Light chain (SEQ ID NO:30):

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK
RTVAAPSVFIFPPSDEQLKSGTASVVCLLNNFYPREAKVQWKVDNALQSGNSQESVTEQDSKDSTYSLSS TLTLSKADYEKHKVYACEVTHQGLSSPVTKSFNRGEC

Heavy chain (SEQ ID NO:40):

QVQLQESGPGLVKPSETLSLTCTVSGFSLSHYDVHWVRQAPGKGLEWLGVIWSGGNTDYNTPFTSRLTIS VDTSKNQFSLKLSSVTAADTAVYYCARALDDYDYEFAYWGQGTLVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSNT KVDKRVEPKSCDKTHTCPPCPAPELLGGPSVFLFPPKPKDTLMISRTPEVTCVVVDVSHEDPEVKFNWYV DGVEVHNAKTKPREEQYNSTYRVVSVLTVLHQDWLNGKEYKCKVSNKALPAPIEKTISKAKGQPREPQV YTLPPSREEMTKNQVSLTCLVKGFYPSDIAVEWESNGQPENNYKTTPPVLDSDGSFFLYSKLTVDKSRWQ QGNVFSCSVMHEALHNHYTQKSLSLSPGK

SWY2113 HCDR1: HYDVH SEQ ID NO:38
SWY2113 HCDR2: VIWSGGNTDYNTPFTS SEQ ID NO:22

SWY2113 HCDR3: ALDDYDYEFAY SEQ ID NO:32
SWY2113 LCDR1: RASQSIGTNIH SEQ ID NO:24
SWY2113 LCDR2: YASESIS SEQ ID NO:25
SWY2113 LCDR3: QQNNEWPTS SEQ ID NO:26
SWY2113 HV: SEQ ID NO:39

QVQLQESGPGLVKPSETLSLTCTVSGFSLSHYDVHWVRQAPGKGLEWLGVIWSGGNTDYNTPFTSRLTIS VDTSKNQFSLKLSSVTAADTAVYYCARALDDYDYEFAYWGQGTLVTVSS

SWY2113 LV: SEQ ID NO:28

EIVLTQSPDFQSVTPKEKVTITCRASQSIGTNIHWYQQKPDQSPKLLIKYASESISGIPSRFSGSGSGTDFTLTI NSLEAEDAATYYCQQNNEWPTSFGQGTKLEIK

Example 1

N-((2R,10S)-10-benzyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-2-methyl-1,6,9,12,15-pentaoxo-3-oxa-5,8,11,14-tetraazahexadecan-16-yl)-6-(2,5-dioxo-2,5-dihydro-1H-pyrrol-1-yl)hexanamide **(JSSW-001)**

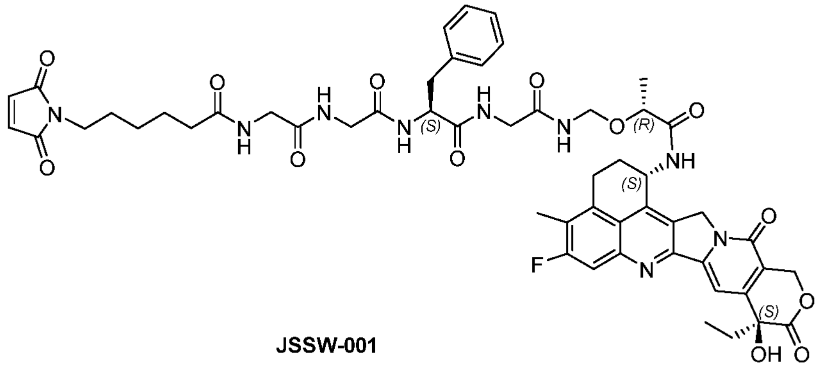

**JSSW-001**

Scheme I:

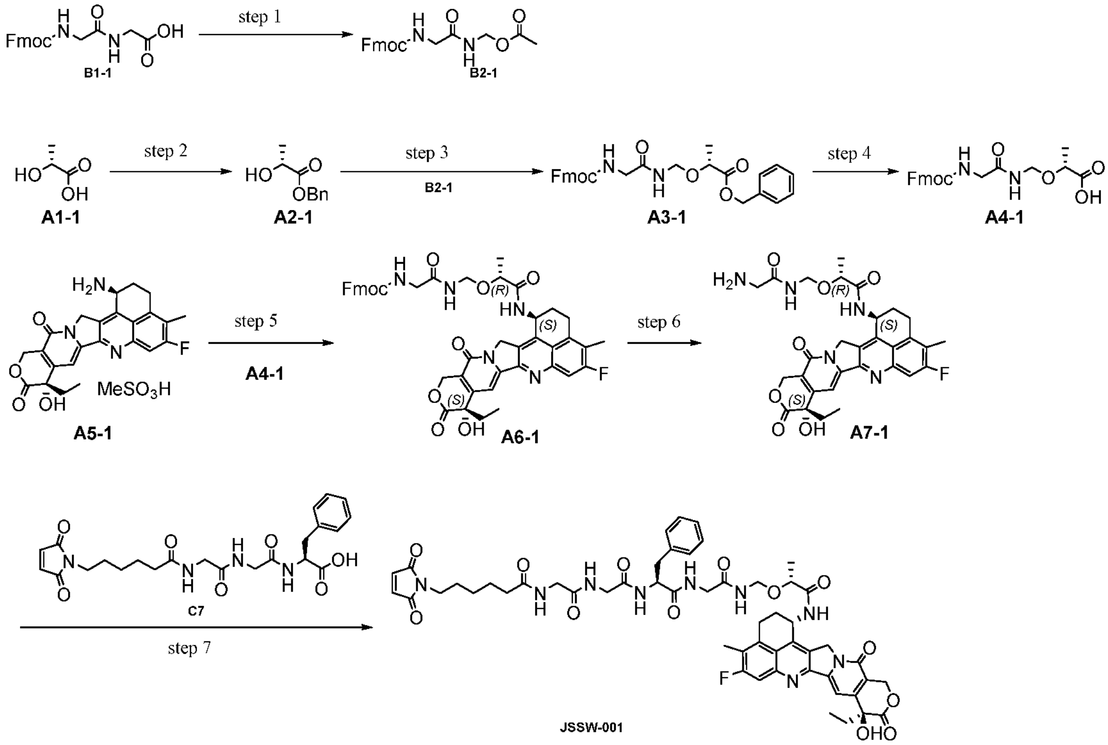


Step 1: Preparation of compound **B2-1**: **BI-1** (1.0g, 2.28mmol), anhydrous tetrahydrofuran (30mL), toluene (10mL), pyridine (0.5mL), and lead tetraacetate (1.83g, 4.12mmol) were added to a 100mL three-neck flask. The solution turned orange and was heated under reflux. After one hour, the solution became colorless, and a white solid was formed. After a three-hour reaction, the solution was filtered using celite, the solid was rinsed with ethyl acetate, and the filtrate was dried using a rotary evaporator. The obtained crude product was purified by column chromatography to produce a solid (640mg). LC-MS$[M+Na]^+$: m/z 391.1.

Step 2: Preparation of compound **A2-1**: **AI-1** (2.7g, 30.0mmol) and N,N-dimethylformamide (30mL) were added to a 100mL three-neck flask, followed by the addition of potassium carbonate (4.17g, 30.0mmol) and benzyl bromide (2.4mL, 20.0mmol). After reacting at room temperature for 14 hours, the reaction mixture was added dropwise into water. Extraction was carried out using ethyl acetate, and the organic layers were combined. The organic layers were combined, washed with saturated brine, dried over anhydrous magnesium sulfate, filtered, and concentrated. The crude product was purified by column chromatography (PE/EA=3:1) to produce 2.61g of the target compound as a colorless liquid.

$^1$H NMR (400 MHz, $CDCl_3$): δ 7.33-7.41 (m, 5H), 5.21 (s, 2H), 4.29-4.35 (m, 1H), 2.77 (d, J = 5.6 Hz, 1H), 1.44 (d, J = 6.8 Hz, 3H).

Step 3: Preparation of compound **A3-1**: **A2-1** (720mg, 4.0mmol), **B2-1** (368mg, 1.0mmol), and dichloromethane (5mL) were added to a 100mL three-neck flask. Pyridinium 4-methylbenzenesulfonate (75mg, 0.30mmol) was then added. The reaction mixture was heated to reflux overnight. After the reaction was complete, ethyl acetate (20mL) was added to the reaction mixture, which was then washed with water three times and dried over anhydrous magnesium sulfate. The magnesium sulfate was removed by filtration, and the solution was concentrated. The obtained crude product was purified by column chromatography (PE/EA=2:1) to produce a white solid **A3-1** (290mg).

LC-MS$[M+Na]^+$: m/z 511.1. $^1$H NMR (400 MHz, $CDCl_3$): δ 7.77 (d, J = 7.2 Hz, 2H), 7.58 (d, J = 7.6 Hz, 2H), 7.40 (t, J = 7.6 Hz, 2H), 7.26-7.37 (m, 6H), 6.70 (brs, 1H), 5.12-5.30(m, 3H), 4.76-4.89 (m, 2H), 4.45 (d, J = 6.8 Hz, 1H), 4.19-4.27 (m, 2H), 3.69-3.85 (m, 2H), 1.41 (d, J = 6.8 Hz, 3H).

Step 4: Preparation of compound **A4-1**: **A3-1** (291mg, 0.60mmol) dissolved in tetrahydrofuran:ethyl acetate (4mL/2mL) was added to a 100mL three-neck flask, along with Pd/C (10% w/w, 60mg). After purging with hydrogen gas three times, a hydrogen balloon was attached, and the mixture was stirred overnight. After the reaction was complete, the mixture was filtered through celite, rinsed with methanol, and the organic layer was concentrated to produce the product **A4-1**. LC-MS$[M+Na]^+$: m/z 399.1.

Step 5: Preparation of compound **A6-1**: **A4-1** (50mg, 0.13mmol, 1.3eq), exatecan mesylate **(AS-1)** (50mg, 0.049mmol, 1.0eq), 2-(7-azabenzotriazol-1-yl)-N,N,N',N'-tetramethyluronium hexafluorophosphate (48mg, 0.13mmol, 1.3eq), and N,N-diisopropylethylamine (36mg, 0.28mmol, 3.0eq) were dissolved in N,N-dimethylformamide (2mL) in a

25mL single-neck flask. The mixture was stirred overnight at room temperature. It was then diluted with ethyl acetate and washed with semi-saturated citric acid, followed by washed with brine, sodium bicarbonate solution, and brine again. The organic layer was dried over anhydrous sodium sulfate, and filtered. The anhydrous sodium sulfate was removed and the filtrate was concentrated. Purification was carried out using prep-TLC with a developer system of (DCM/MeOH=15:1) to produce the product **A6-1** (56mg). LC-MS[M+H]$^+$: m/z 816.3.

Step 6: Preparation of compound **A7-1**: **A6-1** (56mg, 0.069mmol), N,N-dimethylformamide (2mL), and piperidine (12mg, 0.14mmol) were added to a 25mL single-neck flask and stirred for 2 hours. The solution was then concentrated to produce a white solid. LC-MS[M+H]$^+$: m/z 594.3.

Step 7: Preparation of compound **JSSW-001: A7-1** (0.069mmol), **C7** (66mg, 0.14mmol), 2-(7-azabenzotriazol-1-yl)-N,N,N',N'-tetramethyluronium hexafluorophosphate (53mg, 0.14mmol), and N,N-diisopropylethylamine (36mg, 0.28mmol) were dissolved in N,N-dimethylformamide (2mL) in a 25mL single-neck flask. The mixture was stirred for 2 hours at room temperature. It was then diluted with ethyl acetate (30mL) and washed with semi-saturated citric acid, followed by washed with brine, sodium bicarbonate solution, and brine again. The organic layer was dried over anhydrous sodium sulfate, and filtered. The anhydrous sodium sulfate was removed and the filtrate was concentrated. Purification was carried out using prep-TLC with a developer system of (DCM/MeOH=15:1) to produce the product **JSSW-001** (21mg).

LC-MS[M+H]$^+$: m/z 1048. $^1$H NMR (400 MHz, DMSO-d6): $\delta$ 8.63 (t, J = 6.4 Hz, 1H), 8.53 (d, J = 8.8 Hz, 1H), 8.28 (t, J = 6.0 Hz, 1H), 8.03-8.10 (m, 2H), 7.98 (t, J = 6.0 Hz, 1H), 7.77 (d, J = 11.2 Hz, 1H), 7.30 (s, 1H), 7.14-7.25 (m, 5H), 6.99 (s, 2H), 6.51 (s, 1H), 5.56-5.62 (m, 1H), 5.40 (s, 2H), 5.08-5.23 (m, 2H), 4.66-4.71 (m, 1H), 4.42-4.56 (m, 2H), 4.09-4.15 (m, 1H), 3.54-3.76 (m, 7H), 3.09-3.27 (m, 1H), 2.97-3.02 (m, 1H), 2.67-2.77 (m, 1H), 2.38 (s, 3H), 2.16-2.21 (m, 2H), 2.05-2.10 (m, 2H), 1.83-1.89 (m, 2H), 1.37-1.46 (m, 7H), 1.14-1.33 (m, 4H), 0.87 (t, J = 7.2 Hz, 3H).

Scheme II:

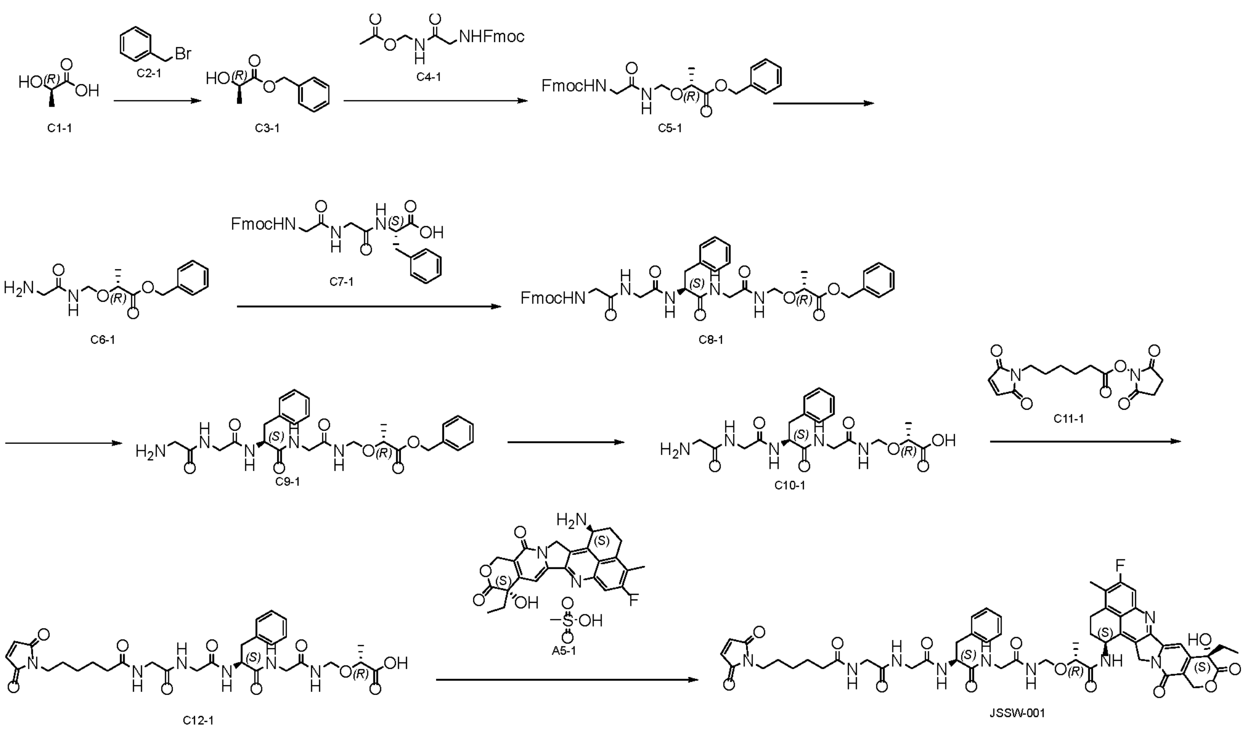


Step 1: Compound **CI-1** (8g, 88.86mmol) and **C2-1** (22.66g, 133.33mmol) were added to a 250mL reaction flask, followed by the addition of DMAc (40mL, 5 v/w). The mixture was stirred until dissolved completely, and the temperature was controlled to 0-10°C. Then, DIEA (34.45g, 266.6mmol) was added dropwise. After the dropwise addition, the mixture was allowed to warm naturally to about 25°C and stirred for 16 hours at this temperature. After TLC monitoring showed the complete reaction of the starting materials, the reaction mixture was poured into 120mL of ice water. MTBE (40mL) was added and the mixture was stirred, allowed to stand for phase separation. The aqueous phase was extracted four times with MTBE (40mL*4). The organic phases were combined and washed once with 0.5M hydrochloric acid solution (40mL), once with water (40mL), and twice with saturated brine (40mL*2), dried over anhydrous sodium sulfate, filtered, concentrated, and purified by wet loading and column chromatography using a gradient system of petroleum ether:ethyl acetate = 30:1→20:1→10:1 to produce a light yellow liquid **C3-1**. LC-MS[M+Na]$^+$: m/z 203.

Step 2: Under nitrogen protection, **C4-1** (6g, 16.29mmol, 1.0eq) and **C3-1** (3.23g, 17.92mmol, 1.1eq) were dissolved in THF (60mL, 10V/w). The temperature was lowered to 10-15°C, and TsOH (281mg, 1.63mmol, 0.1eq) was added. After the completion of the addtion, the reaction was carried out at 14-18°C for 4 hrs under TLC monitoring. After the reaction was complete, the reaction mixture was added to ice water (60mL) and extracted with EA (60mL*3). The organic phases were combined and washed with saturated aqueous NaHCOs solution (60mL), water (60mL*2), and saturated brine (60mL), dried over anhydrous sodium sulfate, filtered, concentrated by evaporating to dryness, and purified by column chromatography using a gradient system of n-hexane:ethyl acetate = 10:1-5:1-3:1-2:1-1:1 to produce a colorless oily substance **CS-1**. LC-MS$[M+Na]^+$: m/z 511.

Step 3: Under nitrogen protection, **CS-1** (2.8g, 5.74mmol, 1.0eq) was dissolved in DMAc (28mL, 10V/w). The temperature was lowered to 14-18°C, and DBU (436.6mg, 2.87mmol, 0.5eq) was added dropwise. The mixture was stirred at this temperature for 1.5h. TLC monitoring showed the complete reaction of the starting materials. The temperature was then lowered to 0-10°C, and PPTS (721.23mg, 2.87mmol, 0.5eq), EDCI (1.1g, 5.74mmol, 1.0eq), HOBT (775mg, 5.74mmol, 1.0eq), and **C7-1** (2.45g, 4.88mmol, 0.85eq) were added. After the completion of the addition, the reaction was carried out at 0-10°C for 3-4 hrs under TLC monitoring until **C7-1** was completely reacted. The reaction mixture was added to ice water (100mL) and extracted with 2-methyl THF (100mL*3). The organic phases were combined and washed with 0.5M hydrochloric acid (150mL*2), saturated aqueous NaHCOs solution (100mL*3), water (100mL), and saturated brine (100mL), dried over anhydrous sodium sulfate, filtered, concentrated by evaporating to dryness, and purified by column chromatography using a gradient system of DCM→DCM:MeOH = 80:1-60:1-50:1-40:1-30:1-20:1 to produce a white foamy solid **C8-1** (3.1g, yield 72%). LC-MS$[M+Na]^+$: m/z 772.

Step 4: Under nitrogen protection, **C8-1** (3.1g, 4.14mmol, 1.0eq) was dissolved in DCM (46.5mL, 15V/w) at room temperature of 25-30°C. DBU (314.6mg, 2.07mmol, 0.5eq) was added dropwise, and the mixture was stirred at this temperature for 16h. TLC monitoring showed the complete reaction of the starting materials. The reaction mixture was directly subjected to wet column chromatography using a gradient system of DCM→DCM:MeOH = 50:1→30:1→20:1→10:1 to produce a white foamy solid **C9-1**. LC-MS$[M+H]^+$: m/z 528.

Step 5: Under nitrogen protection, **C9-1** (1.7g, 3.2mmol, 1.0eq) was dissolved in water (25.5mL, 15V/w) and tert-butanol (8.5mL, 5V/w). The mixture was stirred until dissolved completely. The atmosphere was replaced with nitrogen gas once, and Pd/C (0.34g, 20% w/w) was added. The atmosphere was replaced with hydrogen gas three times. The hydrogenation reaction was carried out at room temperature of 25-30°C for 12hrs. LCMS monitoring showed the complete reaction of starting materials. The reaction mixture was filtered, and the filter cake was washed with water (25mL*2). The filtrate was filtered again, and the tert-butanol was removed by concentration. The aqueous phase was directly lyophilized to produce a white powdery solid **C10-1** (1.5g in total, yield 100%). LC-MS$[M+H]^+$: m/z 438.

Step 6: Under nitrogen protection, **C10-1** (1.4g, 3.2mmol, 1.0eq) was dissolved in acetonitrile (14mL, 10V/w) and water (28mL, 20V/w). **C11-1** (1.86g, 6.097mmol, 1.1eq) was added, and the mixture was stirred until dissolved completely. The temperature was lowered to 0-10°C, and DIEA (330.24mg, 2.56mmol) was added dropwise. After the dropwise addition, the mixture was reacted under stirring at this temperature for 16h. LC-MS monitoring showed the complete reaction of starting materials. A buffer solution was prepared by dissolving $Na_2HPO_4$ (260mg) and $NaH_2PO_4$ (5.6g) in 20mL of water under stirring, and the prepared buffer solution was cooled to 0-5°C. The reaction mixture was poured into the cooled buffer solution and extracted with DCM/isopropanol = 4/1 (50mL*4). The organic phases were combined, dried, filtered, concentrated by evaporating to dryness, and purified by column chromatography using a gradient system of DCM→DCM:MeOH = 50:1-30:1-20:1-15:1 to produce a yellow solid **C12-1.**

Step 7: Under nitrogen protection, **A5-1** (547.4mg, 1.03mmol, 1.0eq) was dissolved in 5% anhydrous sodium sulfate in water (6.5mL) and THF (7.8mL). The mixture was stirred but did not dissolve completely. The temperature was lowered to 0-10°C. Then, NMM (104mg, 1.03mmol, 1.0eq) was added. After the completion of the addtion, the reaction was allowed to proceed at this temperature for 1 hour. **C12-1** (650mg, 1.03mmol, 1.1eq), EDCI (296.2mg, 1.545mmol, 1.5eq), and ethyl cyanoglyoxylate-2-oxime (73.9mg, 0.52mmol, 0.5eq) were added sequentially. After the completion of the addition, the reaction was allowed to proceed at 0-10°C for 4-5 hours. TLC monitoring showed the complete reaction of starting materials. The reaction mixture was then added to 0.1M hydrochloric acid (13mL, 20V/w) in an ice bath, extracted with 2-methyl THF (13mL*3), and the organic phases were combined, washed once with 0.05M hydrochloric acid (13mL, 20V/w) and twice with water (13mL*2), then dried, filtered, concentrated by evaporating to dryness, and purified by column chromatography using a gradient system of DCM-DCM:MeOH=50:1-30:1-20:1-15:1-12:1 to produce a yellow foamy solid **(JSSW-001)**. LC-MS$[M+H]^+$: m/z 1048.

Example 2

N-((S)-13-benzyl-1-(((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)amino)-1,9,12,15,18-pentaoxo-3,6-dioxa-8,11,14,17-tetraazanonadecan-19-yl)-6-(2,5-dioxo-2,5-dihydro-1H-pyrrol-1-yl)hexanamide **(ZW-002)**

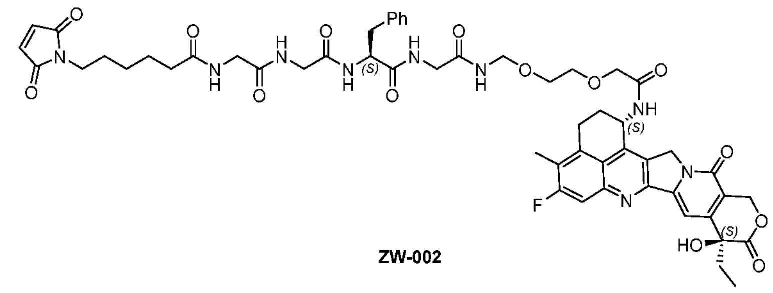

ZW-002

Scheme I:

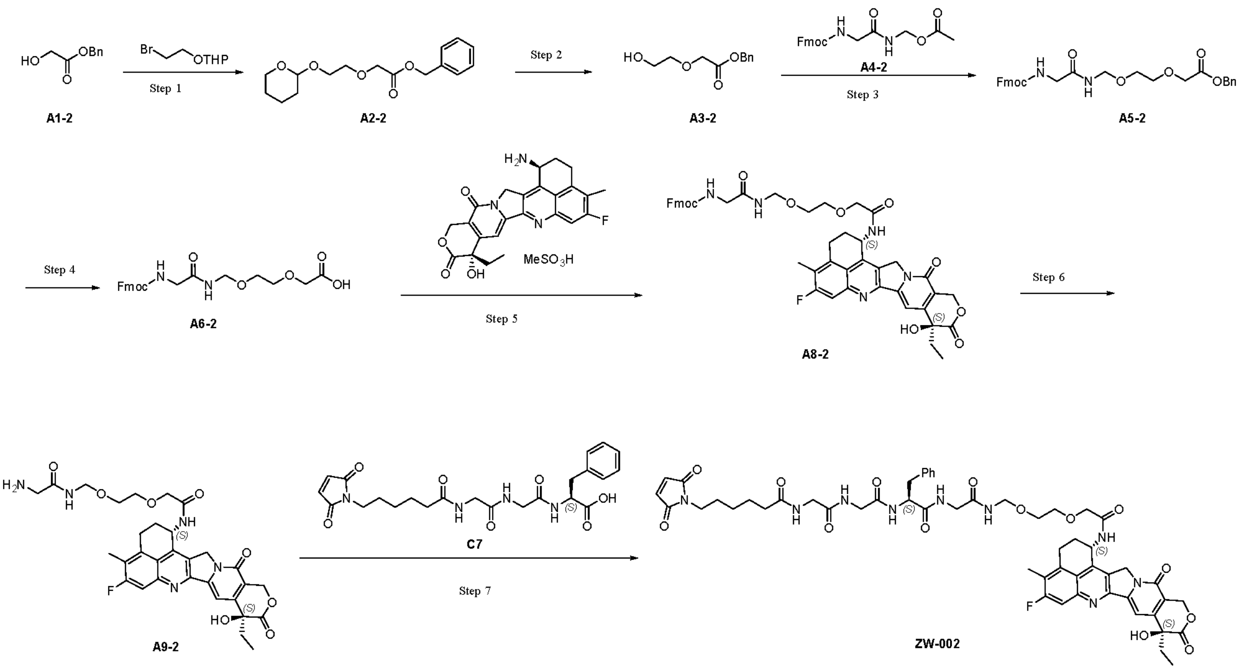


Step 1: Preparation of compound **A2-2**: **A1-2** (4.98g, 30mmol) and N,N-dimethylformamide (40mL) were added to a 100mL three-necked flask. The mixture was stirred at 0°C, and sodium hydride (1.26g, 31.5mmol) was added. The reaction solution was continously stirred for 30 minutes, then 2-(2-bromoethoxy)tetrahydro-2H-pyran (6.59g, 31.5mmol) was added. Then the reaction was allowed to proceed overnight. After the completion of the reaction, water (100mL) was added to quench the reaction. The reaction mixture was extracted with ethyl acetate (150mL*3). The organic layers were combined, washed with saturated brine, dried over anhydrous sodium sulfate, and filtered to remove the solid. The filtrate was concentrated and purified by column chromatography (PE/EA=10/1) to produce a colorless liquid **A2-2** (6.39g). LC-MS $[M+Na]^+$: m/z 317.2.

Step 2: Preparation of compound **A3-2**: **A2-2** (760mg, 2.58mmol) was stirred in dichloromethane (50mL) in a 100mL three-necked flask. Hydrochloric acid methanol solution (0.01mL) was added. The reaction was stirred at room temperature for 1 hour. After the completion of the reaction, the reaction mixture was purified using reverse phase ($H_2O$/ACN=1:1) chromatography to produce the target compound **A3-2** (660mg) as a colorless liquid.

$^1H$ NMR (400 MHz, $CDCl_3$): $\delta$ 7.37-7.36 (m, 5H), 5.20 (s, 2H), 4.18 (s, 2H), 3.75 (t, J = 1.6 Hz, 2H), 3.70 (t, J = 1.6 Hz, 2H).

Step 3: Preparation of compound **AS-2**: **A3-2** (105mg, 0.5mmol) and dichloromethane (10mL) were added to

a 100mL three-necked flask and stirred. **A4-2** (61mg, 0.17mmol) and pyridinium 4-methyltoluenesulfonate (13mg, 0.05mmol) were added. The reaction was refluxed overnight. After the completion of the reaction, ethyl acetate (50mL) was added to the reaction system. The resulting mixture was washed three times with water (20mL*3). The organic layers were dried over anhydrous magnesium sulfate, filtered to remove the solid, and concentrated to dryness. The obtained crude product was purified by column chromatography (PE/EA=1:1) to produce the target compound **A5-2** (80mg) as a light yellow liquid. LC-MS$[M+H]^+$: m/z 519.1.

Step 4: Preparation of compound **A6-2**: **A5-2** (80mg, 0.15mmol), tetrahydrofuran:ethyl acetate (10mL/5mL) and palladium/carbon (10% w/w, 80mg) were added to a 100mL three-necked flask. The reaction was purged with hydrogen three times and allowed to react overnight under hydrogen atmosphere. After the completion of the reaction, the reaction mixture was filtered through celite, rinsed with dichloromethane:methanol=10:1, and concentrated to produce the target compound **A6-2** (73mg). LC-MS $[M+Na]^+$: m/z 451.3.

Step 5: Preparation of compound **A8-2**: **A6-2** (73mg, 0.17mmol), exatecan mesylate (45mg, 0.085mmol), 2-(7-azabenzotriazol-1-yl)-N,N,N',N'-tetramethyluronium hexafluorophosphate (48mg, 0.13mmol), and N,N-diisopropylethylamine (110mg, 0.85mmol) were dissolved in N,N-dimethylformamide (2mL) in a 25mL single-neck flask. The mixture was stirred overnight at room temperature, diluted with ethyl acetate, and washed with ammonium chloride solution followed by brine. The organic layer was dried over anhydrous magnesium sulfate, filtered, concentrated, and purified using prep-TLC with a developer system (DCM/MeOH=15:1) to produce the product **A8-2** (30mg).

LC-MS $[M+H]^+$: m/z 846.4. $^1$H NMR (400 MHz, DMSO-d6): δ 8.62 (t, J = 9.2 Hz, 1H), 8.41 (d, J = 12 Hz, 1H), 7.87 (d, J = 10.8 Hz, 2H), 7.78 (d, J = 13.6 Hz, 1H), 7.68 (d, J = 8.4 Hz, 2H), 7.52 (t, J = 8 Hz, 1H), 7.39 (t, J = 6.8 Hz, 2H), 7.32-7.28 (m, 3H), 6.50 (s, 1H), 5.62-5,54 (m, 1H), 5.41 (s, 2H), 5.19 (s, 2H), 4.50-4.44 (m, 2H), 4.27-4.23 (m, 2H), 4.20-4.14 (m, 1H), 4.01 (s, 2H), 3.64-3.57 (m, 4H), 3.53-3.48 (m, 2H), 3.21-3.12 (m, 2H), 2.38 (s, 3H), 2.22-2.13 (m, 2H), 1.88-1.80 (m, 2H), 0.85 (t, J = 7.6 Hz, 3H).

Step 6: Preparation of compound **A9-2**: **A8-2** (30mg, 0.036mmol), N,N-dimethylformamide (2mL), and piperidine (6mg, 0.07mmol) were added to a 25mL single-neck flask. The mixture was stirred for 2 hours, and the solution was concentrated to produce a yellow solid **A9-2** (30mg). LC-MS $[M+H]^+$: m/z 624.3.

Step 7: Preparation of compound **ZW-002**: **A9-2** (30mg, 0.048mmol), **C7** (23mg, 0.048mmol), 2-(7-azabenzotriazol-1-yl)-N,N,N',N'-tetramethyluronium hexafluorophosphate (28mg, 0.072mmol), and N,N-diisopropylethylamine (31mg, 0.24mmol) were dissolved in N,N-dimethylformamide (5mL) in a 25mL single-neck flask. The mixture was stirred at room temperature for 1 hour, diluted with ethyl acetate (30mL), washed with ammonium chloride solution followed by brine. The organic layer was dried over anhydrous magnesium sulfate, filtered to remove the solid, concentrated, and purified using preparative liquid chromatography to produce the product **ZW-002** (4.2mg). LC-MS $[M+H]^+$: m/z 1078.2.

$^1$H NMR (400 MHz, DMSO-d6): δ 8.46-8.39 (m, 2H), 8.28 (t, J = 5.6 Hz, 1H), 8.11-7.96 (m, 3H), 7.78 (d, J = 10.8 Hz, 1H), 7.29 (s, 1H), 7.20-7.16 (m, 5H), 6.97 (s, 2H), 6.5 (s, 1H), 5.63-5.55 (m, 1H), 5.41 (s, 2H), 5.19 (s, 2H), 4.49-4.42 (m, 3H), 4.01 (s, 2H), 3.74-3.64 (m, 3H), 3.64-3.63 (m, 2H), 3.63-3.40 (m, 2H), 3.34-3.32 (m, 1H), 3.20-3.14 (m, 1H), 3.03-2.98 (dd, J = 5.2 Hz, J = 14.4 Hz, 1H), 2.78-2.73 (m, 1H), 2.38 (s, 3H), 2.24-2.13 (m, 2H), 2.10 (t, J = 7.2 Hz, 2H), 1.91-1.77 (m, 2H), 1.50-1.39 (m, 5H), 1.31-1.12 (m, 6H), 0.85 (t, J = 7.2 Hz, 3H).

Scheme II:

Step 1: **Bl-2** (24g, 169mmol, 1.0eq) was dissolved in DMSO (1200mL, 50v/v), and **B2-2** (43.35g, 253mmol, 5eq) was added dropwise while stirring. After the completion of the addition, the mixture was reacted at room temperature (20-30°C) for 16 hours. After the reaction was completed, the reaction solution was poured into ice water (250mL, 10v/v) and the temperature was controlled at 0-10°C. Extraction was performed three times with EA (300mL, 12-15v/v). The organic phase was washed twice with water (200mL, 8-10v/v) and once with saturated aqueous sodium chloride solution (150mL, 6-10v/v) respectively, dried over anhydrous sodium sulfate (50g), filtered, concentrated, and purified by column chromatography to produce a yellow oily substance **B3-2**.

Step 2: **B3-2** (14g, 66.6mmol, 1.0eq) was dissolved in THF (280mL, 20v/v), and **B4-2** (29.47g, 79.92mmol, 1.2eq) and TsOH (1.15g, 6.66mmol, 0.1eq) were added. After the completion of the addition, the mixture was reacted under stirring at room temperature (20-30°C) for 3 hours. After the completion of the reaction, the reaction solution was poured into a mixture of ice water (500mL, 35-40v/v) and EA (500mL, 35-40v/v). The aqueous phase was extracted three times with EA (500mL, 35-40v/v). The organic layer was washed once each with saturated aqueous $NaHCO_3$ solution (250mL, 15-20v/v) and water (250mL, 15-20v/v). The organic layer was dried over anhydrous sodium sulfate (80g), concentrated into a yellow oil, and purified by column chromatography to produce a white solid **B5-2**.

Step 3: **B5-2** (12g, 23.16mmol, 1.0eq) was dissolved in DMAc (240mL, 20v/v), and DBU (1.76g, 11.8mmol, 0.5eq) was added dropwise. After the completion of the addition, the reaction was performed at 10-15°C for 60 minutes. Then, PPTS (2.91g, 11.8mmol, 0.5eq), HOBt (3.12g, 23.16mmmol, 1.0eq), **B7-2** (11.6g, 23.16mmmol, 1.0eq), and EDCI (4.4g, 23.16mmmol, 1.0eq) were added, and the reaction was performed at 10-15°C for 3 hours. After the completion

of the reaction, the reaction solution was poured into a mixture of ice water (300mL, 25v/v) and methyl tetrahydrofuran (500mL, 40-45v/v) (the temperature was controlled at 0-10°C). The aqueous phase was extracted three times with methyl tetrahydrofuran (300mL, 25v/v). The organic phases were combined, washed once each with saturated aqueous NaHCOs solution (200mL, 15-20v/v) and water (200mL, 15-20v/v). The organic phase was dried over anhydrous sodium sulfate (100g), filtered to remove the drying agent, and the filtrate was pumped at 20-30°C and concentrated under vacuum into a yellow oil. The residue was purified by column chromatography to produce a white solid **B8-2**.

Step 4: **B8-2** (13g, 16.68mmol, 1.0eq) was dissolved in ACN (130mL, lOv/v), and DBU (3.0g, 20.01mmol, 1.2eq) was added dropwise. After the completion of the addition, the reaction was performed at room temperature (20-30°C) for 60 minutes. After the completion of the reaction, the reaction solution was added to ice water (130mL, 10v/v), and the pH was adjusted to 3-4 with 1M HCl. The mixture was extracted four times with EA (150mL, 10-15v/v) (the organic phase was discarded). The pH of the aqueous phase was adjusted to 8-9 with saturated aqueous NaHCOs solution, and the mixture was extracted four times with DCM:isopropanol (4:1, 150mL, 10-15v/v). The organic phases were combined, dried over anhydrous sodium sulfate (80g) and concentrated to produce a white solid **B9-2**.

Step 5: **B9-2** (5.5g, 9.86mmol, 1.0eq) was dissolved in THF (82.5mL, 15v/v) and water (27.5mL, 5v/v), and 10% palladium/carbon (0.55g, weight ratio 0.1) was added. After the completion of the addition, the reaction was carried out under hydrogen protection at 20-30°C for 16 hours.

Post-reaction treatment 1: The reaction solution was filtered to remove the palladium/carbon, and water (100mL, 18-20v/v) was supplemented. The mixture was extracted twice with EA (100mL, 18-20v/v) (the organic phase was discarded), and the aqueous phase was labelled as solution A **(B10-2)**.

Operation 2: To a reaction flask was added the solution A **(B10-2)** (130mL) and **BII-2** (3.6g, 11.83mmol, 1.2eq) dissolved in ACN (49.5mL, 9v/v). DIEA (1.27g, 9.86mmol, 1.0eq) was added dropwise. After the completion of the addition, the reaction was performed at 10-15°C for 5.0 hours.

Post-reaction treatment 2: The reaction solution was poured into ice water (100mL, 18-20v/v), and the aqueous phase was extracted three times with EA (150mL, 10-15v/v). The aqueous phase was controlled at 0-10°C, and the pH was adjusted to 2-3 with 0.5M HCl aqueous solution. The mixture was extracted four times with DCM:isopropanol (4:1, 150mL, 10-15v/v). The organic phases were combined, dried over anhydrous sodium sulfate (50g), and the filtrate was concentrated and dried under vacuum to produce a white solid **B12-2**.

Step 6: Anhydrous sodium sulfate (0.8g, 0.5v/v), water (16mL, lOv/v), and THF (19.2mL, 12v/v) were stirred, and **AS-1** (1.6g, 3.0mmol, 1.0eq) was added. The temperature was lowered to 5-10°C, and N-methylmorpholine (0.304g, 3.0mmol, 1.0eq) was added dropwise. The reaction was carried out at 5-10°C for 40 minutes. Then, **B12-2** (2.4g, 3.6mmol, 1.2eq), EDCI (0.863g, 4.5mmol, 1.5eq), and ethyl cyanoglyoxylate-2-oxime (0.214g, 1.5mmol, 0.5eq) were added sequentially. After the completion of the addition, the reaction was continued at 5-10°C for 3.0 hours. After the completion of the reaction, the reaction solution was poured into 150mL of ice water. The mixture was extracted with EA (150mL*3), washed once with 0.1M HCl (50mL) and once with water (150mL). The organic phase was dried over anhydrous sodium sulfate (30g), concentrated to dryness, and purified by column chromatography to produce a light yellow solid **ZW-002**.

Example 3 Antibody-drug conjugate (DP001-JSSW-001 ADC, DAR=4.0)

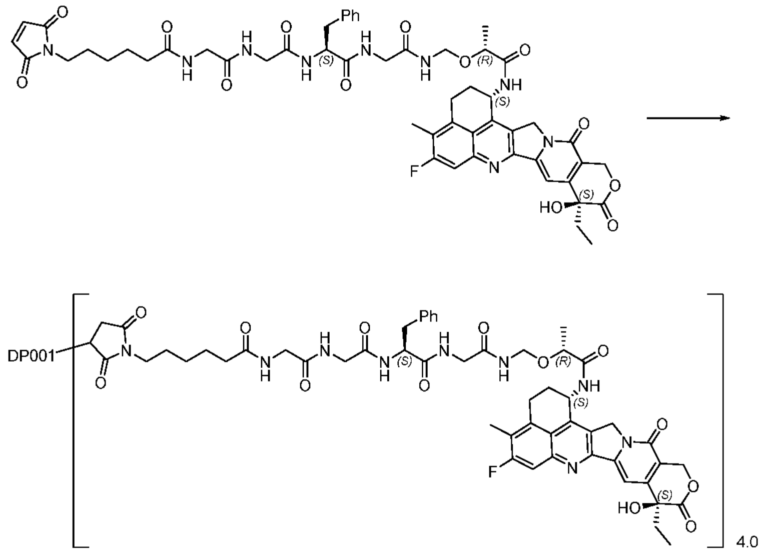

Step 1. Reduction of antibody:

The DP001 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd., 50μL). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (DP001-JSSW-001 ADC, DAR=4.0).

Step 4. Determination of Drug-Antibody Ratio (DAR) of Antibody-Drug Conjugate:

Dithiothreitol with a final concentration of 20 mM was added to 2 mg/kg antibody-drug-conjugate, and the mixture was incubated in a 37°C water bath for 30 minutes. The sample, in which the disulfide bonds between antibody-drug conjugate chains were cleaved, was then used for HPLC analysis. The used HPLC system was the Agilent Technologies 1260 Infinity HPLC, and the used chromatograph column was the PLRP-S column (5 μm particle size; 2.1 mm×50 mm; Agilent Technologies). The column temperature was set at 80°C. The mobile phase A was 0.1% trifluoroacetic acid (TFA) in water, and the mobile phase B was 0.1% TFA in acetonitrile. The injection volume was 10μL, and the gradient program was as follows: 0-3 minutes at 27%-27%, 3-8 minutes at 27%-35%, 8-25 minutes at 35%-43%, 25-26 minutes at 43%-95%, 26-31 minutes at 95%-95%, 31-32 minutes at 95%-27%, and 32-40 minutes at 27%-27%. Compared to the unconjugated light chain (L0) and heavy chain (H0), the hydrophobicity increased with the number of conjugated drugs for the drug-conjugated light chain (L1, conjugated with one drug) and heavy chains (H1, conjugated

with one drug; H2, conjugated with two drugs; H3, conjugated with three drugs).

Therefore, the elution followed the order of L0, L1, H0, H1, H2, and H3. The DAR value was calculated based on the peak area at 280 nm. A result of the DAR value is shown in Figure 1.

$$\mathrm{DAR} = \left(\frac{\mathrm{L1}}{\mathrm{L0}+\mathrm{L1}} \times 1 + \frac{\mathrm{H1}}{\mathrm{H0}+\mathrm{H1}+\mathrm{H2}+\mathrm{H3}} \times 1 + \frac{\mathrm{H2}}{\mathrm{H0}+\mathrm{H1}+\mathrm{H2}+\mathrm{H3}} \times 2 + \frac{\mathrm{H3}}{\mathrm{H0}+\mathrm{H1}+\mathrm{H2}+\mathrm{H3}} \times 3\right) \times 2$$

Example 4 Antibody-drug conjugate (DP001-ZW-002 ADC, DAR=5.78)

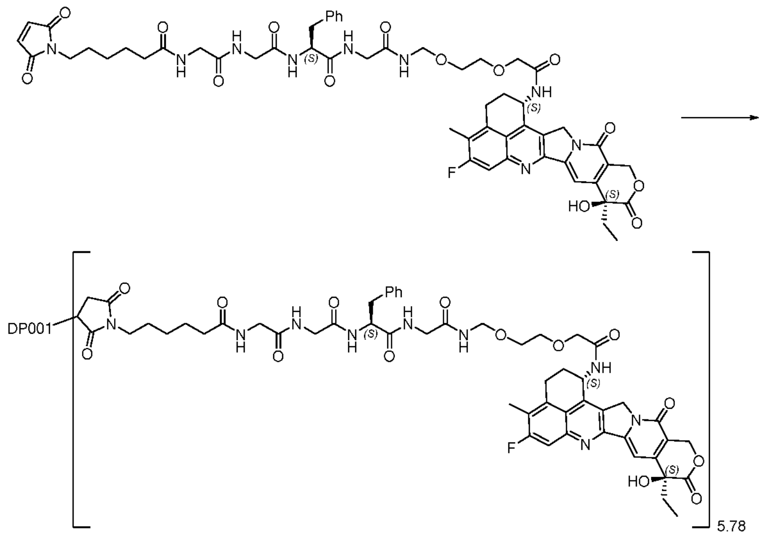

Step 1. Reduction of antibody:

The DP001 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound ZW-002 obtained in Example 2 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (DP001-ZW-002 ADC, DAR=5.78). The DAR value determination method was identical to that in step 4 of Example 3. The DAR value result is shown in Figure 2.

Example 5: Antibody-drug conjugate (DP001-JSSW-001 ADC, DAR=7.32)

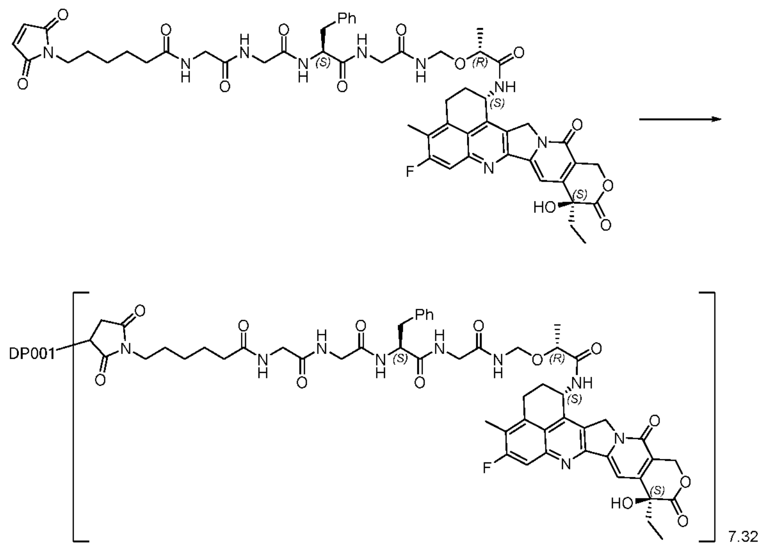

Step 1. Reduction of antibody:

The DP001 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (DP001-JSSW-001 ADC, DAR=7.32). The DAR value determination method was identical to that in step 4 of Example 3. The DAR value result is shown in Figure 3.

Example 6 Antibody-drug conjugate (patritumab-JSSW-001 ADC, DAR=7.36)

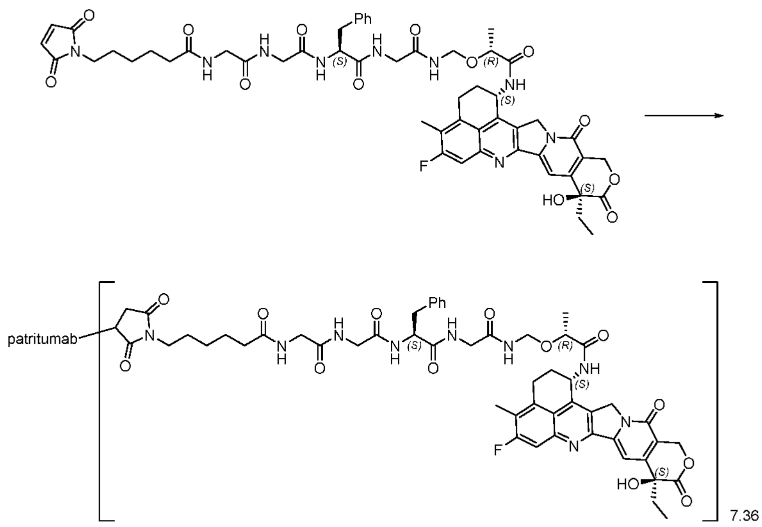

Step 1. Reduction of antibody:

The patritumab antibody medium, which was obtained by purifiction after the expression of plasmids encoding light and heavy chain genes that were transiently transfected into HEK293 cells using known technology, was replaced with PBS6.0/EDTA to prepare a solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2: Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement factor greater than 1000 times. Thus, the non-conjugated linker-drug compound and other low molecular weight reagents were removed to obtain a purified antibody-drug conjugate (patritumab-JSSW-001 ADC, DAR=7.36). The DAR value determination method was identical to that in step 4 of Example 3. The DAR value result is shown in Figure 4.

Example 7 Antibody-drug conjugate (patritumab-ZW-002 ADC, DAR=7.45)

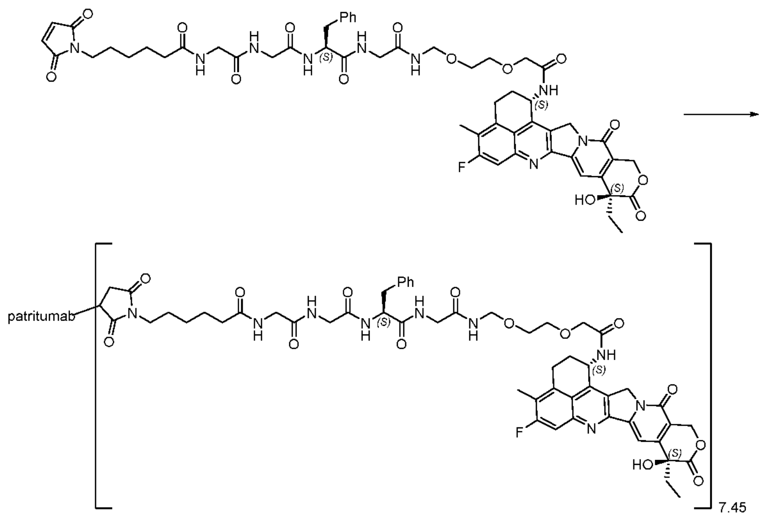

Step 1. Reduction of antibody:

The patritumb antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound ZW-002 obtained in Example 2 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (patritumab-ZW-002 ADC, DAR=7.45). The DAR value determination method was identical to that in step 4 of Example 3. The DAR value result is shown in Figure 5.

Example 8 Antibody-drug conjugate (SWY2110-JSSW-001 ADC, DAR=7.12)

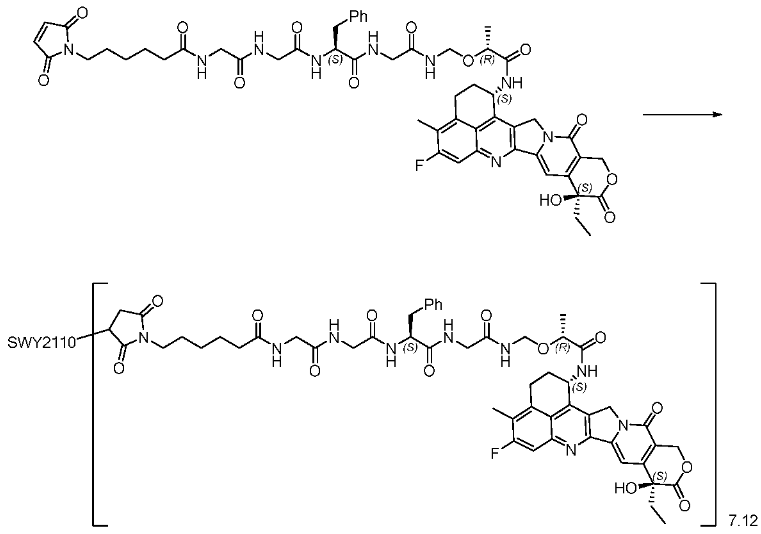

Step 1. Reduction of antibody:

The SWY2110 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90mg/ml, at pH = 5.5. The non-conjugated linker-drug and other low molecular weight reagents were removed to produce a purified antibody-drug conjugate (SWY2110-JSSW-001 ADC, DAR=7.12). The DAR value determination method was identical to that in step 4 of Example 3. The DAR value result is shown in Figure 6.

Example 9 Preparation of pH-dependent antibodies

SWY2110 and EGFR protein were modelled and docked using computer simulation-assisted technology to obtain an antigen-antibody complex. Amino acids within 3Å of the complex underwent D, E, and H scanning, which produced several pH-dependent antibodies that had a reduced affinity at pH 7.4 but basically had an unchanged affinity or had a lower decrease in affinity at pH 6.0. Several obtained antibody sequence genes were cloned into mammalian expression vectors and expressed through transfection of HEK-293 cells. After the completion of the expression, the expression supernatants were collected, and the antibodies were purified using an AKTA system equipped with Protein A pre-packed column to produce the desired antibodies.

Example 10: Antibody affinity determination

MDA-MB-468 cells were digested into single cells using 0.25% trypsin-EDTA and divided equally into multiple

aliquots, with each aliquot containing more than $10^6$ cells. SWY2110 (control) and antibodies obtained from Example 9 (diluted with PBS buffer solutions at pH 7.4 and pH 6.0, respectively) were added to the cells at 300μL with serial 5-fold gradient dilutions. The mixtures were incubated at 4°C for 1 hour and then centrifuged at 1000 rpm for 4 minutes to remove the supernatant. The cells were washed twice with PBS solution, followed by the addition of 200μL of FITC-labelled mouse anti-human Fc secondary antibody with a dilution factor of 1:200. The mixtures were incubated at 4°C for another hour, and then centrifuged at 1000 rpm for 4 minutes to remove the supernatant. Fluorescence intensity was detected using flow cytometry, and data were saved and analyzed using relevant software. The results of the antibody affinity determination after screening are shown in Table 1. The affinity determination results of the optimized antibodies with target antigens after the pH-dependent optimization showed that at pH 7.4, the affinity of the optimized antibodies SWY2111, SWY2112, and SWY2113 decreased significantly (about 3.0 to 9.7 times) compared to SWY2110; and at pH 6.0, the affinity of SWY2111 and SWY2112 remained basically unchanged (1.2 to 1.3 times) compared to SWY2110, while the affinity of the antibody SWY2113 decreased by 6.6 times.

Table 1: Result of Antibody Affinity Determination

| FACS($EC_{50}$) | SWY2110 | SWY2111 | SWY2112 | SWY2113 |
|---|---|---|---|---|
| pH7.4 (nM) | 0.49 | 1.45 | 2.35 | 4.76 |
| pH6.0 (nM) | 0.51 | 0.60 | 0.67 | 3.39 |

Example 11 Antibody-drug conjugate (SWY2111-JSSW-001 ADC, DAR=7.48)

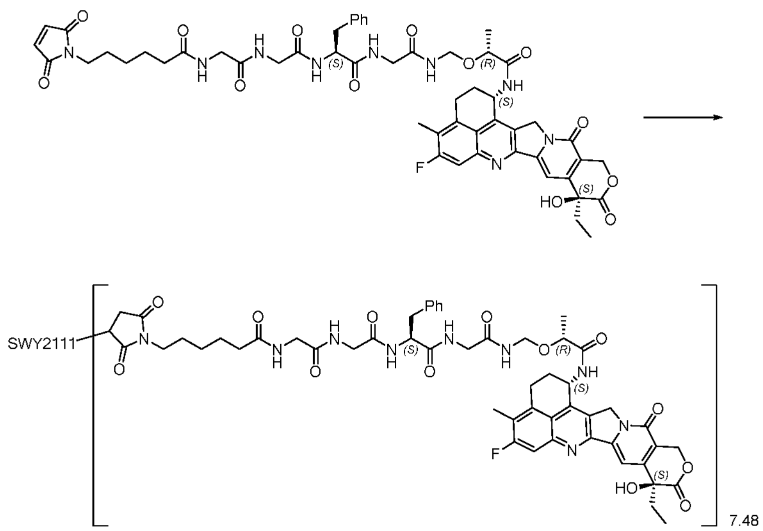

Step 1. Reduction of antibody:

The obtained SWY2111 antibody medium was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90mg/ml, at pH = 5.5. The non-conjugated linker-drug and other low molecular weight reagents were removed to produce a purified antibody-drug conjugate (SWY2111-JSSW-001 ADC, DAR=7.48). The DAR value determination method was identical to that in step 4 of Example 3. The DAR value result is shown in Figure 7.

Example 12 Antibody-drug conjugate (SWY2112-JSSW-001 ADC, DAR=7.51)

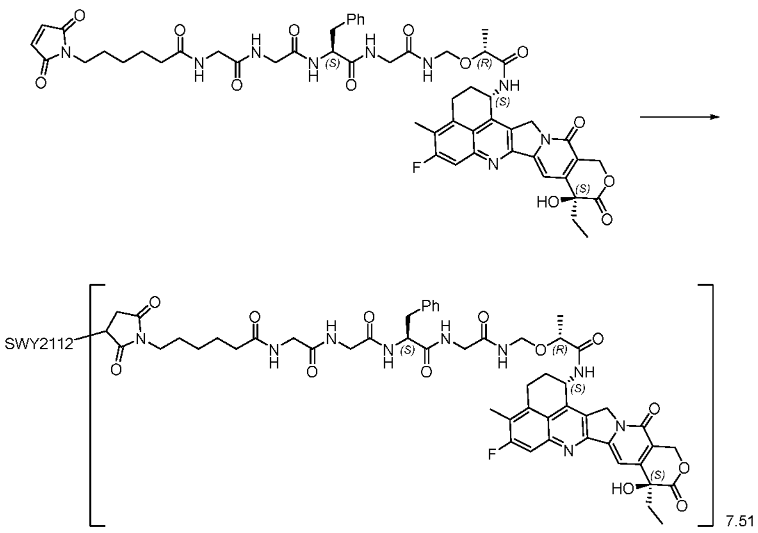

Step 1. Reduction of antibody:

The obtained SWY2112 antibody medium was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90mg/ml, at pH = 5.5. The non-conjugated linker-drug and other low molecular weight reagents were removed to produce a purified antibody-drug conjugate (SWY2112-JSSW-001 ADC, DAR=7.51). The DAR value determination method was identical to that in step 4 of Example 3. The DAR value result is shown in Figure 8.

Example 13 antibody-drug conjugate (SWY2113-JSSW-001 ADC, DAR=7.50)

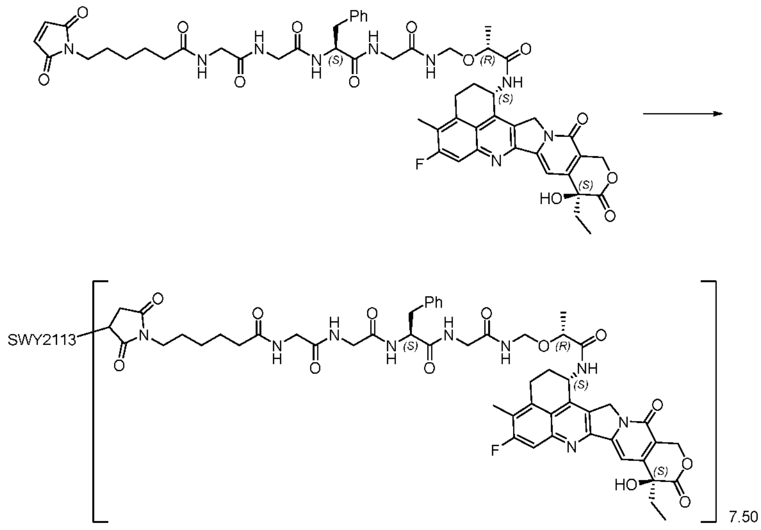

Step 1. Reduction of antibody:

The obtained SWY2113 antibody medium was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of the compound JSSW-001 obtained in Example 1 in dimethylsulfoxide (87μL; 13 equivalents per antibody molecule) at room temperature, and the mixture was mixed evenly and reacted at room temperature for 30 minutes to attach the linker-drug to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). To the reaction solution were added L-histidine 0.89mg/ml and L-histidine hydrochloride 4.04mg/ml as buffer, Tween80 0.03%, and sucrose 90mg/ml, at pH = 5.5. The non-conjugated linker-drug and other low molecular weight reagents were removed to produce a purified antibody-drug conjugate (SWY2113-JSSW-001 ADC, DAR=7.50). The DAR value determination method was identical to that in step 4 of Example 3. The DAR value result is shown in Figure 9.

Reference Example 1 Antibody-drug conjugate (DP001-DXD ADC, DAR=4.3)

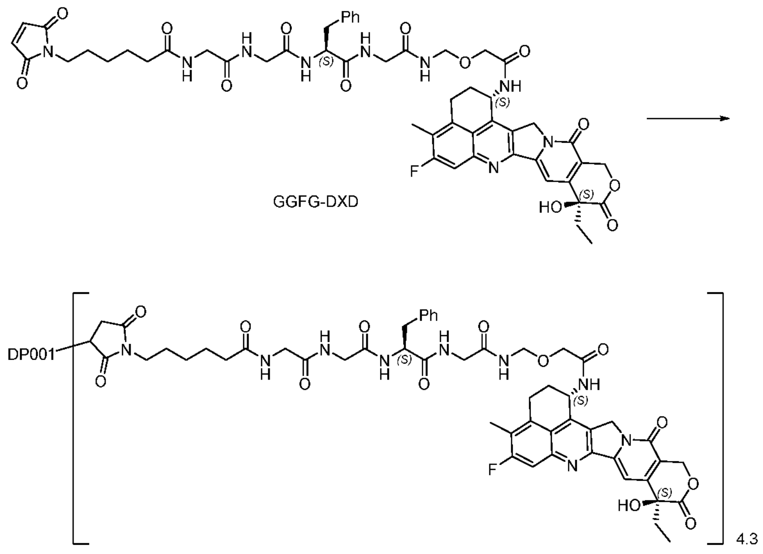

GGFG-DXD

Step 1. Reduction of antibody:

The DP001 antibody medium, which was obtained by purifiction after the expression of plasmids encoding light and heavy chain genes that were transiently transfected into HEK293 cells using known technology, was replaced with PBS6.0/EDTA to prepare a solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd., 50μL). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (DP001-DXD ADC, DAR=4.3). The method for measuring the drug-antibody ratio (DAR) of the antibody-drug conjugate was identical to that in step 4 of Example 3.

Reference Example 2 Antibody-drug conjugate (DP001-DXD ADC, DAR=7.0)

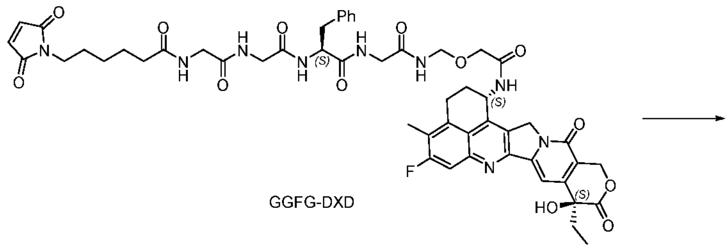

GGFG-DXD

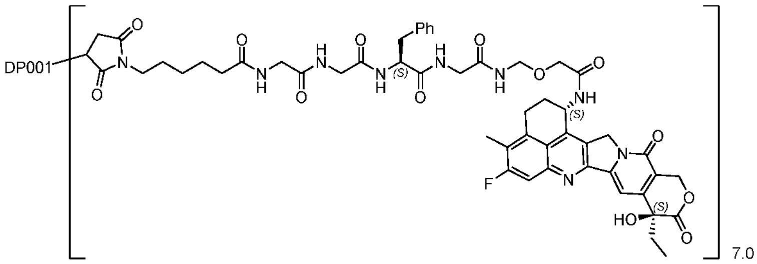

Step 1. Reduction of antibody:

The DP001 antibody medium was replaced with PBS6.0/EDTA to prepare a solution having an antibody concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (DP001-DXD ADC, DAR=7.0). The method for measuring the drug-antibody ratio (DAR) of the antibody-drug conjugate was identical to that in step 4 of Example 3.

Reference Example 3 Antibody-drug conjugate (patritumab-DXD ADC, DAR=7.3)

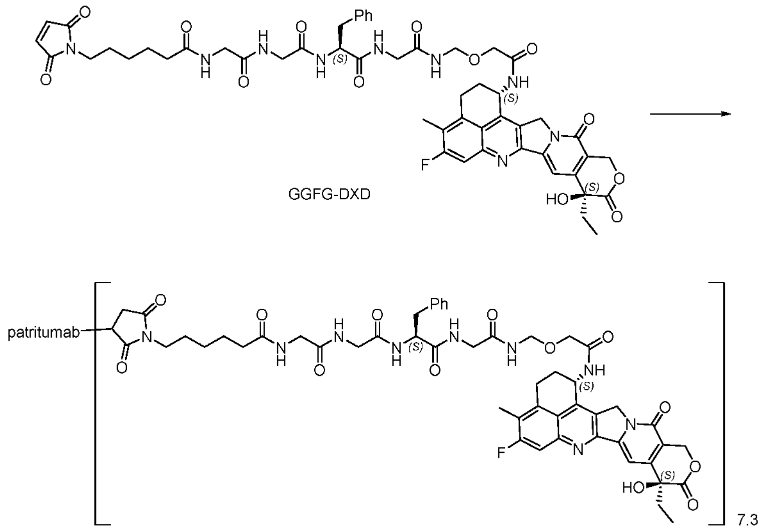


Step 1. Reduction of antibody:

The patritumab antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 100 mM aqueous TCEP (Bailingwei Science and Technology Co., Ltd.) solution (6.66μL; 10 equivalents per antibody molecule) and 1M dipotassium hydrogen phosphate aqueous solution (Tianjin Guangfu Science and Technology Development Co., Ltd., 18μL). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (patritumab-DXD ADC, DAR=7.3). The DAR value determination method was identical to that in step 4 of Example 3. The DAR value result is shown in Figure 10.

Reference Example 4 Antibody-drug conjugate (SWY2110-DXD ADC, DAR=6.93)

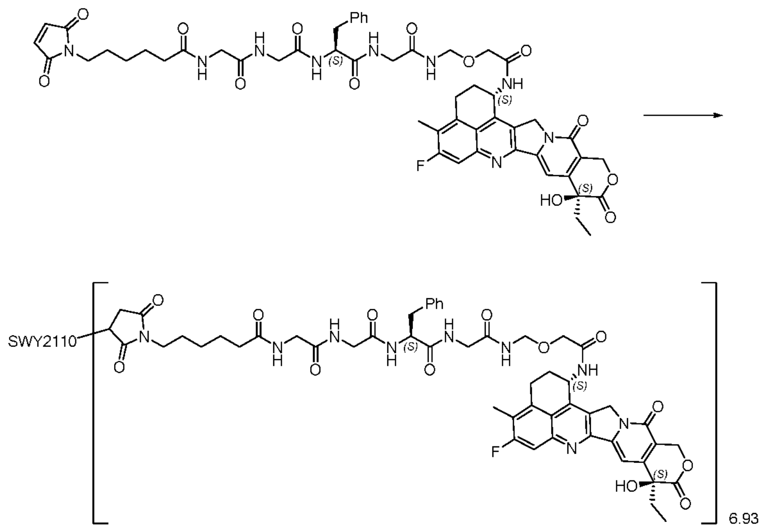

Step 1. Reduction of antibody:

The SWY2110 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (66.6μL; 10 equivalents per antibody molecule) and 1M aqueous dipotassium hydrogen phosphate solution (Tianjin Guangfu Science and Technology Development Co., Ltd.). After confirming that the pH of the solution was within 7.0±0.1, the solution was incubated at 37°C for 3 hours to reduce the disulfide bonds in the antibody.

Step 2. Conjugation of antibody and linker-drug compound:

To the above solution was added a 10 mM solution of commercially available Deruxtecan (CAS No.:1599440-13-7) in dimethylsulfoxide (87μL; 13 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (SWY2110-DXD ADC, DAR=6.93). The DAR value determination method was identical to that in step 4 of Example 3. The DAR value result is shown in Figure 11.

Reference Example 5 Antibody-drug conjugate (SWY2110-VC MMAE ADC, DAR=4.53)

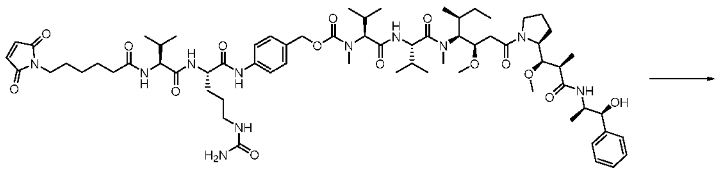

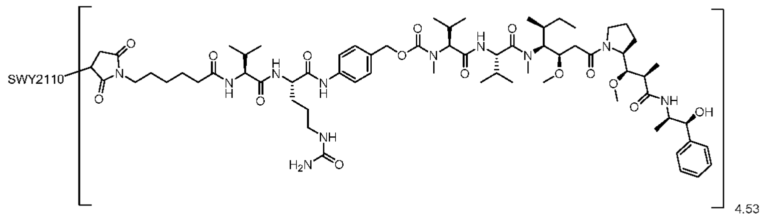

Step 1. Reduction of antibody:

The SWY2110 antibody medium, which was obtained by purifiction after the expression of plasmids encoding light and heavy chain genes that were transiently transfected into HEK293 cells using known technology, was replaced with PBS6.0/EDTA to prepare an antibody solution with a concentration of 10 mg/mL. This solution (1.0mL) was put into a 1.5mL polypropylene tube, to which were added 10 mM aqueous TCEP (Bailingwei Science and Technology Co., Ltd.) solution (17μL; 2.5 equivalents per antibody molecule) and 1M aqueous dipotassium hydrogen phosphate solution (Tianjin Guangfu Science and Technology Development Co., Ltd.). After confirming that the pH of the solution was within 7.4±0.1, the solution was incubated at 37°C for 1 hour to reduce the disulfide bonds in the antibody.

Step 2. Conjugation of antibody and linker-drug compound:

To the above solution was added a 10 mM solution of commercially available mc-vc-PAB-MMAE (CAS No.:646502-53-6, abbrev. Vc MMAE) in dimethylsulfoxide (31.28μL; 4.6 equivalents per antibody molecule) at room temperature, and the mixture was mixed evenly and reacted at room temperature for 30 minutes to attach the linker-drug compound to the antibody. Next, 100mM aqueous NAC (Bailingwei Science and Technology Co., Ltd.) solution (10.2μL) was added, and the mixture was further stirred at room temperature for 20 minutes to terminate the reaction.

Step 3. Purification of antibody-drug conjugate:

The reaction solution was purified by ultrafiltration using ultrafiltration centrifuge tubes (Merck, Ultracel@ Regenerated Cellulose (30kDa MWCO), 15mL sample volume). 25mM 2-(N-morpholine)ethanesulfonic acid (MES) was added as purification buffer to the reaction solution, pH=6.5. The resulting solution was concentrated to 1-2mL, the purification buffer was added again to make the replacement ratio greater than 1000 times. Thus, the non-conjugated linker-drug compound and other low molecular weight reagents were removed to obtain the purified antibody-drug conjugate (SWY2110-VC MMAE ADC, DAR=4.53).

Step 4. Determination of Drug-Antibody Ratio (DAR) of Antibody-Drug Conjugate:

10 μg SWY2110-Vc MMAE ADC sample was loaded onto the chromatographic column (2.5 μm particle size; 4.6 mm × 10 cm; TSKgel Butyl-NPR). The elution mobile phase A was 20 mM PB, pH 7.0, 1.5 M $(NH_4)_2SO_4$ aqueous solution, and the mobile phase B was 20 mM PB, pH 7.0, 25% isopropanol solution. The flow rate was controlled at 0.8mL/min, the column temperature was 30°C, the detection wavelength was 280 nm, and the gradient program was set as follows: 0-20 minutes, mobile phase B from 0 to 100%; 20-25 minutes, 100% mobile phase B; and 25-30 minutes, 100% mobile phase A. Being non-conjugated was DAR0, being conjugated with 2 small molecules was DAR2, being conjugated with 4 small molecules was DAR4, being conjugated with 6 small molecules was DAR6, and being conjugated with 8 small molecules was DAR8. Therefore, elution was performed in the order of DAR0, DAR2, DAR4, DAR6, and DAR8. The DAR value was calculated based on the peak area at 280nm, where A represents the percentage of each peak area. A result of the DAR value is shown in Figure 12.

$$DAR=2\times A_{DAR2}+4\times A_{DAR4}+6\times A_{DAR6}+8\times A_{DAR8}$$

Biological Examples

Assay 1: Activity of antibody-drug in cells in vitro

1. Assay object:

The object of this assay was to detect the inhibitory activity of the antibody-drug conjugate of the present invention on the in-vitro proliferation of SK-BR-3 (ATCC/HTB-30) tumour cell, human colorectal cancer cell DiFi, human lung cancer gefitinib resistant cell PC9-GR, or human lung cancer mutant cell PC-9 (Del19-T790M-C797S). The cells were treated in vitro with compounds of different concentrations. After culture, resazurin was added to read the fluorescence values of ex550nm/em610nm, and the data were fitted with four parameters to obtain the $IC_{50}$ values, thereby calculating the biological activities of the compound.

2. Assay materials and equipments:

Materials

| Product Name | Manufacturer/Item Number |
|---|---|
| SK-BR-3 cell line | ATCC/HTB-30 |
| DiFi cell line | Shanghai Jinmante Biotechnology Co., Ltd |
| PC9-GR cell line | Nanjing Cobioer Biosciences Co., Ltd |
| MTT Assay Kit | Abcam |
| DMEM, high glucose | Hyclone/SH30022.01 |
| 0.25% Trypsin-EDTA | Gibco/25200-072 |
| fetal bovine serum (FBS) | Gibco/16000-044 |
| 100×bispecific antibody | Gibco/15240-062 |
| black walled, transparent bottom tissue culture plate | Corning/3603 |
| resazurin sodium | Sigma Aldrich/199303-25G |

Equipments

| Name | Model | Manufacturer |
|---|---|---|
| Biosafety cabinet | 1300 series A2 model | Thermo Fisher Scientific |
| $CO_2$ incubator | 3111 model | Thermo Fisher Scientific |
| Inverted microscope | CKX31 | Olympus |
| Microplate reader | Infinite M200 | Tecan Company |
| Micro-oscillator | MM-I model | Shanghai Yarong Biochemistry Instrument Factory |

3. Assay operation procedure 1:

3.1 culture medium: DMEM, 10% FBS, 1×bispecific antibody

3.2 Cell culturing: Frozen SK-BR-3/MDA-MB-468 cells were taken out of liquid nitrogen, and revived in a $75cm^2$ culture vessel for culturing. The cells were grown until the confluency of cells reached >75%, and had undergone

at least 3 passages.

3.2.1 If cell passage was not performed, the culture medium should be replaced every 3-4 days.

3.2.2 If the cell expansion was needed, the cells were passaged and inoculated into a larger culture vessel, ensuring that the confluency of cells reached >75% before use in assays.

3.3 Cell Collection: SK-BR-3/MDA-MB-468 cells were collected when the culture vessel was nearly full.

3.3.1 The culture medium was discarded and the cells were washed with PBS to remove dead cells and residual culture medium.

3.3.2 2-3mL of 0.25% Trypsin-EDTA was added, the culture vessel was gently shaken and then incubated at 37°C for 2-3 minutes to digest the cells.

3.3.3 5mL of the culture medium was immediately added to the culture vessel and gently pipetted up and down to spread the cells.

3.3.4 The cell suspension was transferred to a sterile centrifuge tube and centrifugingd 200×g for 3 minutes.

3.3.5 The culture medium in the tube was discarded, and 5-10mL of the fresh culture medium was added to resuspend the cells.

3.4 Cell density determination: the cells were counted with a cell counting plate under a microscope.

3.5 Assay plate inoculation

3.5.1 Cells were diluted to $1\times10^5$ cells/mL with the culture medium and seeded at 100μL/well in the assay plate (except rows A and H).

3.5.2 120μL of the culture medium was added to each well in rows A and H as blank.

3.5.3 The incubation was performed at 37°C with 5% $CO_2$ for 4-6 hrs to allow the cells to adhere to the wall.

3.6 Preparation of diluted compound samples:

The compound was diluted to an initial concentration of 18 μg/ml, and then further diluted by a factor of 3 to obtain a total of 11 gradient solutions. The 12th column was left as blank control.

3.7 Drug treatment

3.7.1 20μL of drug diluent from each of the 1st to 11th columns of the dilution plate was taken and added to the corresponding column of the assay plate.

3.7.2 20μL of fresh culture medium was added to each well in the 12th column and the A and H rows.

3.7.4 The plate was gently shaked on a plate shaker for 10-15 seconds, and then the culture plate was incubated at 37°C for 3 days.

3.8 Analyses

3.8.1 After incubation, 20μL of 0.03% resazurin (1×PBS diluted) was added to each well and gently shaked for 10-15 seconds.

3.8.2 After incubating at 37°C for 3-4 hours, the plate was read using a microplate reader with the following parameters:

Exciting light: 550nm
Emitting light: 610nm
Integration time: 50
Shaking: 15 seconds, vortex
Reading: top reading
Number of reading/well: 1
Gain: optimization setting (between 35 and 42)
If reading multiple plates, it was ensured that the gain setting was consistent for all plates.

3.8.3 EXCEL was used to plot the data and fit the $IC_{50}$ values for the reference standards and samples.

3.8.3.1 Model 201 was used to plot the data points.

3.8.3.2 For Fit parameters, the following commands were used:

a. A: Pre-fitted

b. B: Pre-fitted

c. C: Pre-fitted

d. D: Pre-fitted

e. No constraints for all

3.8.3.3 The output parameter C represented the $IC_{50}$ in unit of ng/mL.

The results are shown in Table 1.

Assay operation procedure 2:

This assay verified the inhibition effects of SWY2110 monoclonal antibody (SWY2110 mAb), SWY2110-DXD ADC (DAR=6.93), and SWY2110-JSSW-001 ADC (DAR=7.12) on the proliferation of two cell lines, DiFi (human colorectal cancer cells) and PC9-GR (gefitinib-resistant human lung cancer cells). The specific concentration settings were as follows:
DiFi: SWY2110 mAb, SWY2110-DXD ADC (DAR=6.93) and SWY2110-JSSW-001 ADC (DAR=7.12) started at a concentration of 3000 ng/ml, with a 3x serial dilution of the drug, resulting in 10 concentrations: 3000, 1000, 333.33, 111.11, 37.04, 12.35, 4.12, 1.37, 0.46, 0.15 ng/ml respectively. The drug exposure lasted for 144 hours.
PC9-GR: SWY2110 mAb, SWY2110-DXD ADC (DAR=6.93), SWY2110-JSSW-001 ADC (DAR=7.12) started at a concentration of 1000 ng/ml, with a 5x serial dilution of the drug, resulting in 8 concentrations: 1000, 200, 40, 8. 1.6, 0.32, 0.64, 0.013 ng/ml respectively. The drug exposure lasted for 144 hours.
Cells in logarithmic growth phase were seeded in a 96-well plate (100μL/well) at a certain number. After the adherent cells had adhered for 24 hours, 100×L of culture medium containing different concentrations of SWY2110 mAb, SWY2110-DXD ADC (DAR=6.93), or SWY2110-JSSW-001 ADC (DAR=7.12) was added to each well. Three replicate wells were set for each drug concentration, along with corresponding blank wells (only containing culture medium) and normal wells (drug concentration of 0). After 144 hours of drug exposure, a MTT working solution (5 mg/mL) was added to each well (20μL per well). The plates were incubated at 37°C for 4 hours, and then the supernatant was discarded. DMSO (analytical grade) was added (150μL per well), and the plates were shaken on a microplate shaker to mix thoroughly. The plates were then wiped clean, and the optical density (OD) was measured at 550 nm using the microplate reader.
The inhibition rate of the cell growth was calculated with the following equation:

$$\text{Inhibition Rate}(\%)=(\text{OD Value}_{\text{normal well}}-\text{OD Value}_{\text{drug well}})/(\text{OD Value}_{\text{normal well}}-\text{OD Value}_{\text{blank well}})\times 100\ \%$$

Based on the inhibition rates at different concentrations, the half-maximal inhibitory concentration ($IC_{50}$) of the drug was calculated using SPSS 19.0. The results are shown in Tables 2 and 3, and Figures 17 and 18.
Assay data:

Table 2: $IC_{50}$ value of DP001-ADC against SK-BR-3 cells

| Compound No. | $IC_{50}$ value (ng/mL) |
|---|---|
| DP001-DXD ADC (DAR=7.0) | 7.2481 |
| DP001-JSSW-001 ADC (DAR=7.32) | 5.1824 |

It can be seen from the data recorded in Table 2 that the antibody-drug DP001-JSSW-001 ADC (DAR=7.12) of the present invention had a lower $IC_{50}$ value against SK-BR-3 cells than DP001-DXD ADC (DAR=6.93), showing better anti-cell proliferation effect.

Table 3: $IC_{50}$ value of SWY2110-ADC against two cell lines (ng/mL)

| Cell | SWY2110 mAb | SWY2110-DXD ADC (DAR=6.93) | SWY2110-JSSW-001 ADC (DAR=7.12) |
|---|---|---|---|
| DiFi | 43.94 | 14.85 | 15.84 |
| PC9-GR | -- | 1.00 | 1.12 |

wherein "--" represents not showing anti-cell proliferation effect.
It can be seen from the data in Table 3 that the antibody-drug conjugates SWY2110-JSSW-001 ADC (DAR=7.12) and SWY2110-DXD ADC (DAR=6.93) of the present invention had similar $IC_{50}$ values for EGFR overexpressing cell line DiFi and gefitinib-resistant cell line PC9-GR, and showed better anti-cell proliferation effect compared to SWY2110 mAb.
Assay 2: Efficiency assay of antibody-drug conjugates on HER2-expressing human breast cancer JIMT-1

xenograft tumour in NU/NU mice

1. Assay animals

NU/NU mice, 5-6 weeks old, 24 mice

2. Assay object

To investigate the antitumour effect of antibody-drug conjugates in vivo using a HER2-expressing human breast cancer JIMT-1 cell xenograft tumour model in NU/NU mice.

3. Drug dosage and grouping

Table 4. Table of animal grouping and dosage

| Group | Animal Number | Dosage (mg/kg) | Administration Route/Frequency |
|---|---|---|---|
| Vehicle | 6 | 0 | i.v./once |
| DP001-DXD ADC (DAR=4.30) | 6 | 3 | i.v./once |
| DP001-JSSW-001 ADC (DAR=4.0) | 6 | 3 | i.v./once |
| DP001-ZW-002ADC (DAR=5.78) | 6 | 3 | i.v./once |
| Note: i.v.: intravenous injection. | | | |

4. Assay method

In this assay, human breast cancer JIMT-1 cells were used to construct a xenograft tumour model of the tumour in nude mice. When the tumour volume reached about 127 $mm^3$, the animals were evenly divided into 4 groups (d0) according to Table 4 based on tumour volume with 6 animals in each group. Intravenous administration was given once, with an administration volume of 10mL/kg. The vehicle control group (Vehicle) received 0.9% sodium chloride injection. Observations were made for 13 days after administration to compare the inhibition effects of three antibody-drug conjugates in the table on HER2-expressing human breast cancer JIMT-1 xenograft tumours in nude mice.

5. Observation indicator

5.1 Evaluation indicator

(1) Tumour volume: $V = 1/2 \times A \times B^2$

(2) Relative tumour volume: $$\mathrm{RTV} = \frac{TV_{nd}}{TV_{0d}}$$

(3) Relative tumour volume proliferation rate: $$\frac{\mathrm{T}}{\mathrm{C}}\% = \frac{\text{Drug Group } \mathrm{RTV_{xnd}}}{\text{Vehicle Group } \mathrm{RTV_{xnd}}} \times 100\%$$

(4) Tumour (growth) inhibition rate: $$\mathrm{TGI}\% = [1 - \frac{\mathrm{TV}_{Xn} - \mathrm{TV_{X0}}}{\mathrm{TV_{Mn}} - \mathrm{TV_{M0}}}] \times 100\%$$

Note: V: Tumour volume

A: tumour length
B: tumour width
RTV: relative tumour volume
TVnd: tumour volume on day n
TV0d: tumour volume on day 0

RTVxnd: average relative tumour volume on day n
TVXn: average tumour volume of drug group on day n
TVX0: average tumour volume of drug group on day 0
TVMn: average tumour volume of vehicle group on day n
TVM0: average tumour volume of vehicle group on day 0

6 Result

6.1 Tumour volume

At the end of the assay, compared with the vehicle group, DP001-DXDADC (DAR=4.3) (P<0.05), DP001-JSSW-001 ADC (DAR=4.0) (P<0.01) and DP001-ZW-002 ADC (DAR=5.78) (P<0.05) all could significantly inhibit the volume of transplantation tumour (see Figure 13 and Table 5 for details).

Table 5 Tumour parameters of each group 13 days after administration of antibody-drug conjugates

| Group | Animal Number | Dosage (mg/kg) | Tumour Volume ($mm^3$) | TGI(%) Day 13 |
|---|---|---|---|---|
| Vehicle | 6 | 0 | 322.7±82.2 | --- |
| DP001-DXD ADC (DAR=4.30) | 6 | 3 | 192.7±70.2* | 67.3 |
| DP001-JSSW-001 ADC (DAR=4.0) | 6 | 3 | 202.9±38.1** | 61.4 |
| DP001-ZW-002ADC (DAR=5.78) | 6 | 3 | 194.8±68.2* | 65.3 |
| *: compared with vehicle group P<0.05, **: compared with vehicle group P<0.01 | | | | |

Assay 3: Efficacy assay of antibody-drug conjugate on human lung adenocarcinoma PC9-GR (gefitinib-resistant cell) xenograft tumour in NU/NU mice

1. Assay animals

NU/NU mice, 5-6 weeks old, 16 mice

2. Assay object

To investigate the anti-tumour effect of two patritumab-ADC drugs in vivo using a human lung adenocarcinoma PC-9/GR cell (gefitinib-resistant cell) xenograft tumour model in NU/NU mice.

3. Drug dosage and grouping

Table 6. Table of animal grouping and dosage

| Group | Animal Number | Dosage (mg/kg) | Administration Route/Frequency |
|---|---|---|---|
| Vehicle | 6 | 0 | i.v./qw*4w |
| patritumab-DXD ADC (DAR=7.3) | 5 | 3 | i.v./qw*4w |
| patritumab-JSSW-001 ADC (DAR=7.36) | 5 | 3 | i.v./qw*4w |
| Note: i.v.: intravenous injection, qw*4w: administration once weekly for 4 weeks. | | | |

4. Assay method

In this assay, human lung adenocarcinoma PC9-GR cells (gefitinib-resistant cells) were used to construct a xenograft tumour model of the tumour in nude mice. When the tumour volume reached approximately 110 $mm^3$, the animals were divided into 3 groups (d0) based on tumour volume according to Table 6, with 6 animals in vehicle group, each 5 animals in patritumab-DXD ADC (DAR=7.3) group and patritumab-JSSW-001 ADC (DAR=7.36) group. Intravenous administration was given once weekly for 4 weeks, with an administration volume of 10mL/kg. The vehicle control group (Vehicle) received 0.9% sodium chloride injection. The assay was terminated on day 28 after the administration. The inhibition effects of patritumab-DXD ADC (DAR=7.3) and patritumab-JSSW-001 ADC (DAR=7.36) on human lung adenocarcinoma PC9-GR (Gefitinib-resistant cell) xenograft tumour in nude mice were compared.

5. Observation indicator

5.1 Evaluation indicator

See the section of Assay 2: 5.1 Evaluation indicator

6 Result

6.1 Tumour volume

At the end of the assay, the patritumab-JSSW-001 ADC (DAR=7.36) group ($P<0.001$) exhibited the superior effect of inhibiting the volume of xenograft tumour compared to the patritumab-DXD ADC (DAR=7.3) ($P<0.01$) group. For details, see Figure 14 and Table 7.

Table 7 Tumour parameters of each group 28 days after administration of patritumab-ADC

| Group | Animal Number | Dosage (mg/kg) | Administration Route/ Frequency | Tumour Volume ($mm^3$) | TGI(%) Day28 |
|---|---|---|---|---|---|
| Vehicle | 6 | 0 | i.v./qw*4w | 761.4±272.6 | --- |
| patritumab-DXD ADC (DAR=7.3) | 5 | 3 | i.v./qw*4w | 373.4±102.8** | 59.5 |
| patritumab-JSSW-001 ADC (DAR=7.36) | 5 | 3 | i.v./qw*4w | 312.7±86.7*** | 68.9 |
| **: compared with vehicle group P<0.01, ***: compared with vehicle group P<0.001 | | | | | |

Assay 4: Efficacy Assay of SWY2110-ADC on human colorectal cancer DiFi xenograft tumour in NU/NU mice 1. Assay animals
NU/NU mice, 5-6 weeks old, 14 mice

2. Assay object

To investigate the anti-tumour effect of two SWY2110-ADC drugs in vivo using a human colorectal cancer DiFi cell xenograft tumour model in NU/NU mice.

3. Drug dosage and grouping

Table 8. Table of animal grouping and dosage

| Group | Animal Number | Dosage (mg/kg) | Administration Route/Frequency |
|---|---|---|---|
| Vehicle | 5 | 0 | i.v./once |
| SWY2110-Vc MMAE ADC (DAR=4.53) | 4 | 0.5 | i.v./once |
| SWY2110-JSSW-001 ADC (DAR=7.12) | 5 | 0.5 | i.v./once |
| Note: i.v.: intravenous injection. | | | |

4. Assay method

In this assay, human colorectal cancer DiFi cells were used to construct a xenograft tumour model of the tumour in nude mice. When the tumour volume reached approximately 110 $mm^3$, the animals were evenly divided into 3 groups (d0) based on tumour volume according to Table 8, with each 5 animals in vehicle group and SWY2110-JSSW-001 ADC (DAR=7.12) group and 4 animals in SWY2110-Vc MMAE ADC (DAR=4.53) group. Intravenous administration was given once with an administration volume of 10mL/kg. The vehicle control group (Vehicle) received 0.9% sodium chloride injection. Observation was made for 27 days after the administration. The inhibition effects of two SWY2110-ADC drugs in the table on human colorectal cancer DiFi xenograft tumour in nude mice were compared.

5. Observation indicator

5.1 Evaluation indicator

See the section of Assay 2: 5.1 Evaluation indicator

6 Result

6.1 Tumour volume

At the end of the assay, compared with the vehicle group, both SWY2110-Vc MMAE ADC (DAR=4.53) ($P<0.05$) and SWY2110-JSSW-001 ADC (DAR=7.12) ($P<0.05$) could significantly inhibit the volume of the xenograft tumour. The tumour inhibition rate on day 27 of the SWY2110-JSSW-001 ADC (DAR=7.12) group reached as high as 74.6%, followed by the SWY2110-Vc MMAE ADC (DAR=4.53) group with a tumour inhibition rate of 69.7%. Since the toxicity of the JSSW-001 small molecule is lower than that of Vc MMAE, it suggested that SWY2110-JSSW-001 ADC (DAR=7.12) can achieve a more efficient and less toxic therapeutic effect. For details, see Figure 15 and Table 9.

Table 9 Tumour parameters of each group 27 days after administration of SWY2110-ADC

| Group | Animal Number | Dosage (mg/kg) | Tumour Volume ($mm^3$) | TGI(%) Day27 |
|---|---|---|---|---|
| Vehicle | 5 | 0 | $763.1\pm293.8$ | -- |
| SWY2110-Vc MMAE ADC (DAR=4.53) | 4 | 0.5 | $306.5\pm332.4$* | 69.7 |
| SWY2110-JSSW-001 ADC (DAR=7.12) | 5 | 0.5 | $275.5\pm297.5$* | 74.6 |
| *: compared with vehicle group P<0.05 | | | | |

Assay 5 Efficacy assay of SWY2110-ADC on human lung adenocarcinoma PC9-GR (gefitinib-resistant cell) xenograft tumour in NU/NU mice

1. Assay animals

NU/NU mice, 5-6 weeks old, 30 mice

2. Assay object

To investigate the anti-tumour effect of SWY2110 monoclonal antibody and three SWY2110-ADC drugs in vivo using a human lung adenocarcinoma PC-9-GR cell (gefitinib-resistant cell) xenograft tumour model in NU/NU mice.

3. Drug dosage and grouping

Table 10. Table of animal grouping and dosage

| Group | Animal Number | Dosage (mg/kg) | Administration Route/Frequency |
|---|---|---|---|
| Vehicle | 6 | 0 | i.v./qw*4w |
| SWY2110 mAb | 6 | 1 | i.v./qw*4w |
| SWY2110-Vc MMAE ADC (DAR=4.53) | 6 | 1 | i.v./qw*4w |
| SWY2110-DXD ADC (DAR=6.93) | 6 | 1 | i.v./qw*4w |
| SWY2110-JSSW-001 ADC (DAR=7.12) | 6 | 1 | i.v./qw*4w |
| Note: i.v.: intravenous injection, qw*4w: administration once weekly for 4 weeks. | | | |

4. Assay method

In this assay, human lung adenocarcinoma PC9-GR cells (gefitinib-resistant cells) were used to construct a xenograft tumour model of the tumour in nude mice. When the tumour volume reached approximately 110 $mm^3$, the animals were evenly divided into 5 groups (d0) based on tumour volume according to Table 10, with 6 animals in each group. Intravenous administration was given once weekly for 4 weeks, with an administration volume of 10mL/kg. The vehicle control group (Vehicle) received 0.9% sodium chloride injection. The assay was terminated on day 28 after the administration. The inhibition effects of SWY2110 monoclonal antibody and three SWY2110-ADC drugs on human lung adenocarcinoma PC9-GR (Gefitinib-resistant cell) xenograft tumour in nude mice were compared. 5. Observation indicator

5.1 Evaluation indicator

See the section of Assay 2: 5.1 Evaluation indicator

6 Result

6.1 Tumour volume

At the end of the assay, compared with the vehicle group, SWY2110-Vc MMAE ADC (DAR=4.53) (P<0.05), SWY2110-DXD ADC (DAR=6.93) (P<0.05), and SWY2110-JSSW-001 ADC (DAR=7.12) (P<0.001) all exhibited inhibition effects on the volume of the xenograft tumour. The antibody SWY2110 showed poor tumour inhibition effect, with a tumour inhibition rate of only 20.3%. The SWY2110-JSSW-001 ADC (DAR=7.12) group had the most significant inhibition effect on the xenograft tumour volume of PC9-GR, with a tumour inhibition rate as high as 77.7%. The tumour inhibition rates of SWY2110-Vc MMAE ADC (DAR=4.53) and SWY2110-DXD ADC (DAR=6.93) were 40.4% and 50.2%, respectively. SWY2110-JSSW-001 ADC (DAR=7.12) showed 154% of the tumour inhibition effect of SWY2110-DXD ADC (DAR=6.93) on the PCR-GR, and SWY2110-JSSW-001 ADC (DAR=7.12) showed 192% of the tumour inhibition effect of SWY2110-Vc MMAE ADC (DAR=4.53) on PCR-GR, suggesting that SWY2110-JSSW-001 ADC (DAR=7.12) still had a significant inhibition effect on gefitinib-resistant tumour cells. For details, see Figure 16 and Table 11.

Table 11 Tumour parameters of each group 28 days after administration of SWY2110-ADC

| Group | Animal Number | Dosage (mg/kg) | Administration Route/ Frequency | Tumour Volume (mm3) | TGI(%) Day28 |
|---|---|---|---|---|---|
| Vehicle | 6 | 0 | i.v./qw*4w | 761.4±272.6 | - |
| SWY2110 mAb | 6 | 1 | i.v./qw*4w | 628.9±290.0 | 20.3 |
| SWY2110-Vc MMAE ADC (DAR=4.53) | 6 | 1 | i.v./qw*4w | 498.0±179.1* | 40.4 |
| SWY2110-DXD ADC (DAR=6.93) | 6 | 1 | i.v./qw*4w | 434.6±136.3* | 50.2 |

(continued)

| Group | Animal Number | Dosage (mg/kg) | Administration Route/ Frequency | Tumour Volume (mm3) | TGI(%) Day28 |
|---|---|---|---|---|---|
| SWY2110-JSSW-001 ADC (DAR=7.12) | 6 | 1 | i.v./qw*4w | 255.1±117.7*** | 77.7 |
| *: compared with vehicle group P<0.05, ***: compared with vehicle group P<0.001 | | | | | |

Assay 6: In Vitro Plasma Stability of Antibody-Drug Conjugates

DP001-DXD ADC (DAR=7.0), DP001-JSSW-001 ADC (DAR=7.32) and DP001-ZW-002 ADC (DAR=5.78) were incubated with 0.5% BSA-PBS and ICR mouse plasma, respectively, at concentrations of DP001-DXD ADC (DAR=7.0) (91.7μg/mL), DP001-JSSW-001 ADC (DAR=7.32) (80μg/mL) and DP001-ZW-002ADC (DAR=5.78) (80μg/mL). Samples were collected after incubation for 0h, 24h, 48h, 72h, 96h, 120h, 144h, 168 h, 336h and 504h, respectively, under sterile conditions at 37°C. The concentration of dropped molecules in the plasma samples was detected using LC-MS/MS. The results are as follows:

Table 12 In vitro plasma stability

| | DP001-DXD ADC (DAR=7.0) | | DP001-JSSW-001 ADC (DAR=7.32) | | DP001-ZW-002ADC (DAR=5.78) |
|---|---|---|---|---|---|
| Time | 0.5% BSA-PBS | ICR | 0.5% BSA-PBS | ICR | 0.5% BSA-PBS |
| (h) | dropping rate (%) | dropping rate (%) | dropping rate (%) | dropping rate (%) | dropping rate (%) |
| 0 | 0.02 | 0.02 | 0.03 | 0.01 | 0.39 |
| 24 | - | - | 0.13 | 0.15 | 0.00 |
| 48 | 0.13 | 0.61 | 0.16 | 1.02 | 2.57 |
| 72 | 0.13 | 0.65 | 0.18 | 1.13 | 3.24 |
| 96 | 0.13 | 0.97 | 0.20 | 0.77 | 3.51 |
| 120 | 0.16 | 1.18 | - | 0.82 | 2.99 |
| 144 | 0.16 | 1.29 | 0.19 | 1.18 | 1.53 |
| 168 | 0.14 | 1.43 | 0.20 | 1.36 | 0.86 |
| 336 | 0.41 | 2.50 | 0.27 | 1.54 | 1.18 |
| 504 | 3.90 | 4.07 | 1.09 | 2.75 | 2.91 |
| Note: 1. Calculation of the dropping rate: according to the DAR value, the mass ratio of DXD/ADC was calculated to determine DP001-DXD ADC (DAR=7.0) (91.7μg/mL), DP001-JSSW-001 ADC (DAR=7.32) (80μg/mL) and DP001-ZW-002 ADC (DAR=5.78) (80μg/mL). Dropping rate (%)= Ct/ Ctotal*100%, wherein Ctotal represents the total small molecule concentration, and Ct represents the free small molecule concentration at each time point. "-" indicates no sampling. | | | | | |

The results showed that ADCs generated free small molecules in both 0.5% BSA-PBS and ICR mouse plasma after incubation at 37°C for 0h, 24h, 48h, 72h, 96h, 120h, 144h, 168 h, 336h and 504h. After 504 hours of incubation, the dropping rates in 0.5% BSA-PBS were DP001-DXD ADC (DAR=7.0) (3.90%), DP001-JSSW-001 ADC (DAR=7.32) (1.09%) and DP001-ZW-002 ADC (DAR=5.78) (2.91%) respectively, and in ICR mouse plasma, the dropping rates were DP001-DXD ADC (DAR=7.0) (4.07%), DP001-JSSW-001 ADC (DAR=7.32) (2.75%) respectively. In 0.5% BSA-PBS, the dropping rates of small molecules from the antibodies were all less than 4.0%: DP001-DXD ADC (DAR=7.0)> DP001-ZW-002 ADC (DAR=5.78)> DP001-JSSW-001 ADC (DAR=7.32), indicating that DP001-JSSW-001 ADC (DAR=7.32) and DP001-ZW-002 ADC (DAR=5.78) are more stable in 0.5% BSA-PBS. In ICR mouse plasma, the dropping rates of small molecules from the antibodies were all less than 5.0%: DP001-DXD ADC (DAR=7.0)> DP001-JSSW-001 ADC (DAR=7.32), indicating that DP001-JSSW-001 ADC (DAR=7.32) was more stable in ICR mouse plasma than DP001-DXD ADC (DAR=7.0).

Assay 7: Study on in vitro bystander effect of antibody-drug

For the study on the bystander effect, positive cell line SK-BR-3 and negative control cell MDA-MB-468 were co-cultured with DP001-DXD ADC (DAR=7.0) and DP001-JSSW-001 ADC (DAR=7.32), respectively. The released small molecule toxins could cause apoptosis of co-cultured negative cells in the periphery, known as the bystander effect. In this study, reporter gene cells HEK293-Luc were co-cultured with positive cell line SK-BR-3 and negative control cell MDA-MB-468 for four days. The effect of released small molecule toxins on the growth of HEK293-Luc was determined by adding a chemiluminescent substrate. The study results showed that DP001-DXD ADC (DAR=7.0) and DP001-JSSW-001 ADC (DAR=7.32) could bind to HER2 receptors on the surface of tumour cells, enter the cells through endocytosis and release small molecule toxins. Meanwhile, DP001-JSSW-001 ADC (DAR=7.32) showed a stronger inhibition effect on the proliferation of bystander cells.

2. Assay preparation

2.1 Assay equipments:

| Apparatus and equipment | Manufacturer | Model |
|---|---|---|
| $CO_2$ incubator | Thermo Fisher | 3111 model |
| Biosafety cabinet | ThermoFisher | 1300 Series A2 6 inches |
| Desktop high speed freezing centrifuge | Thermo Fisher | SORVALL Stratos |
| Inverted microscope | Olympus | CKX31 inverted microscope |
| Pipettor | Eppendorf | Research Plus |
| Microplate reader | Tecan | M200 |

2.2 Assay materials:

| Name | Manufacturer | Item Number |
|---|---|---|
| 96-well transparent flat-bottom culture plate | Corning | 3599 |
| cell culture plate | Corning | 3917 |
| 50mL centrifuge tube | Corning | 430828 |
| 15mL centrifuge tube | Corning | 430790 |
| T75 cell culture flask | Corning | 430641 |
| T25 cell culture flask | Corning | 430639 |

2.3 Assay reagents:

| Name | Manufacturer | Item Number |
|---|---|---|
| DPBS | BBI | E607009-0500 |
| Fetal Bovine Serum | Gibco | 10099-141C |
| DMEM culture medium | HyClone | SH30243.01 |
| 0.25% Trypsin-EDTA | Gibco | 25200-072 |
| Bright-GloTM Luciferase assay system | Promega | E2620 |

2.4 Cell information

| Name | Manufacturer | culture condition |
|---|---|---|
| SK-BR-3 | ATCC | DMEM culture medium +10% |
| MDA-MB-468 | Nanjing Cobioer | DMEM culture medium +10% |
| HEK293-Luc | self-made in the laboratory | DMEM culture medium +10% FBS+400 μg/ml G418+100 μg/ml HygromycinB |

3. Assay method

3.1 Reagent preparation

3.1.1 Preparation of cell recovery medium

DMEM complete medium: Adding to a DMEM basic culture medium so that the final system contained 10% of Fetal Bovine Serium and 1% of Penicilin/Streptomycin, fully mixing uniformly, and storing at 2-8°C.

3.1.2 preparation of cell culture medium (containing a screening agent)

Adding to a DMEM basic culture medium so that the final system contained 10% of Fetal Bovine Serium and 1% of Penicilin/Streptomycin and adding 200μg/mL of G418, fully mixing uniformly, and storing at 2-8°C.

3.2 Cell preparation

3.2.1 Cell recovery

A cell cryopreservation tube was placed in a water bath at 37°C and shaked quickly until it was completely thawed, then transferred to an ultra-clean workbench or a biosafety cabinet. The cell suspension was diluted with a cell recovery medium. The mixture was centrifuged, and the centrifugation supernatant was discarded. Cells were resuspended by using the cell recovery medium, and the cell density and viability were measured. The cell density was adjusted with the cell culture medium. The cell suspension was uniformly mixed,transferred to a cell culture flask. After the microscopic examining, it was transferred to an incubator for culturing at 37°C with 5% $CO_2$.

3.2.2 cell passages

The cell culture flask was removed from the $CO_2$ incubator and transferred to the ultra-clean workbench or biosafety cabinet. After the cells were washed with PBS, digested with trypsin, and the digestion was terminated with cell growth medium, the cell suspension was collected, and cell density and viability were measured. An appropriate amount of the cell suspension was added to a suitable amount of the fresh medium (with or without a screening agent), and the cells were passaged and expanded according to their growth characteristics and experimental needs. After passaging, the cells were placed in an incubator for continued culture.

3.3 Bystander effect test procedure

3.3.1 When the cell confluence reached 90%, SK-BR-3, MDA-MB-468, and HEK293-Luc cells were digested with trypsin and collected separately, and the cells were resuspended in their respective media.

3.3.2 The resuspended target cells were gently pipetted several times to form a single-cell suspension. Cell viability and cell count were identified using the trypan blue staining method, and the cell density was adjusted to $2.0\times10^5$ cells/mL.

3.3.3 The density-adjusted SK-BR-3 and MDA-MB-468 cells were evenly mixed with HEK293-Luc cell suspension at a 1:1 ratio. Fifty microliters per well were added to a 96-well transparent flat-bottom culture plate. The positive cell group was a mixture of SK-BR-3 and HEK293-Luc cells, while the negative cell group was a mixture of MDA-MB-468 and HEK293-Luc cells.

3.3.4 DP001-DXD ADC (DAR=7.0) and DP001-JSSW-001 ADC (DAR=7.32) groups (drug administration groups) were provided at three concentrations with final concentrations of 100 ng/ml, 500 ng/ml, and 1000 ng/ml. Fifty microliters per well were added to the cell-inoculated 96-well transparent flat-bottom culture plate and placed in a 37°C $CO_2$ incubator for 4 days.

3.3.5 After incubation, the plate was removed from the incubator, and the temperature was allowed to equilibrate to room temperature. Bright-Glo™ Luciferase Assay System was added to the plate at 100μL per well, and chemiluminescence was read after 5-15 minutes in the dark.

4. Test result: DP001-DXD ADC (DAR=7.0) and DP001-JSSW-001 ADC (DAR=7.32) could bind to HER2 receptors on the surface of tumour cells, enter the cells through endocytosis and release small molecule toxins. Meanwhile, DP001-JSSW-001 ADC (DAR=7.32) showed a stronger inhibition effect on the proliferation of bystander cells than DP001-DXD ADC (DAR=7.0). See Table 13 and Figure 19 for the test result.

Table 13: Inhibition rate of the antibody-drug conjugates of the present invention on fluorescent cells

| Concentration/ng/ml | SKBR3+HEK293-GLP1/DP001-DXD ADC (DAR=7.0) | MDA-MB-468+HEK293-GLP1/DP001-DXD ADC (DAR=7.0) | SKBR3+HEK293-GLP1/DP001-JSSW-001 ADC (DAR=7.32) | MDA-MB-468+HEK293-GLP1/DP001-JSSW-001 ADC (DAR=7.32) |
|---|---|---|---|---|
| | Inhibition rate | | | |
| 1000 | 17.1% | 2.7% | 18.7% | 3.1% |
| 500 | 16.4% | 2.6% | 18.1% | -0.4% |
| 100 | 10.0% | 3.4% | 16.8% | 2.7% |

Assay 8: Study on the stability of linker-drug compounds

Different linker-drug compounds such as Deruxtecan, JSSW-001, and ZW-002 were studied under high temperature (40°C, 60°C), high humidity (25°C/75% RH and 25°C/92.5% RH), illumination (illuminance 4500Lux, near-UV energy 90μw/$cm^2$), or 0.5% BSA-PBS conditions. Samples were collected at different times after exposure to these conditions, and the concentration changes of the linker-drug compounds in the samples were detected using the LC-MS/MS method. The test results showed that the compounds JSSW-001 and ZW-002 had better stability.

Assay 9: Efficacy assay of SWY2110-ADC on human lung adenocarcinoma PC9-AR (PC9-Del19/T790M/C797S, osimertinib-resistant cell) xenograft tumour

1. Assay animals

Balb/c nude mice, 5-6 weeks old, 24 mice

2. Assay object

To investigate the anti-tumour effect of three SWY2110-ADC drugs in vivo using a human lung adenocarcinoma PC9-Del19/T790M/C797S cells (osimertinib-resistant cell, hereinafter referred to as PC9-AR) xenograft tumour model in NU/NU mice.

3. Drug dosage and grouping

Table 14. Table of animal grouping and dosage

| Group | Animal Number | Dosage (mg/kg) | Administration Route/Frequency |
|---|---|---|---|
| Vehicle | 6 | 0 | i.v./once |
| SWY2110-Vc MMAE ADC | 6 | 3 | i.v./once |
| (DAR=4.53) | | | |
| SWY2110-DXD ADC (DAR=6.93) | 6 | 3 | i.v./once |

(continued)

| Group | Animal Number | Dosage (mg/kg) | Administration Route/Frequency |
|---|---|---|---|
| SWY2110-JSSW-001 ADC (DAR=7.12) | 6 | 3 | i.v./once |
| Note: i.v.: intravenous injection, i.v./once: administration once. | | | |

4. Assay method

In this assay, human lung adenocarcinoma PC9-Del19/T790M/C797S cells (osimertinib-resistant cell) were used to construct a xenograft tumour model of the tumour in nude mice. When the tumour volume reached approximately 110 $mm^3$, the animals were evenly divided into 4 groups (d0) based on tumour volume according to Table 14, with 6 animals in each group. Intravenous administration was given once, with an administration volume of 10mL/kg. The vehicle control group (Vehicle) received 0.9% sodium chloride injection. The assay was terminated on day 24 after the administration. The inhibition effects of three SWY2110-ADC drugs on human lung adenocarcinoma PC9-Del19/T790M/C797S (osimertinib-resistant cell) xenograft tumour in nude mice were compared.

5. Observation indicator

5.1 Evaluation indicator

See the section of Assay 2: 5.1 Evaluation indicator

6 Result

6.1 Tumour volume

At the end of the assay, compared with the vehicle group, SWY2110-Vc MMAE ADC (DAR=4.53) (P<0.001), SWY2110-DXD ADC (DAR=6.93) (P<0.001) and SWY2110-JSSW-001 ADC (DAR=7.12) (P<0.001) all exhibited inhibition effects on the volume of the xenograft tumour. The SWY2110-JSSW-001 ADC (DAR=7.12) group demonstrated the most significant inhibitory effect on the PC9-AR xenograft tumour volume, achieving a tumour inhibition rate of as high as 97.5%. The tumour inhibition rates of SWY2110-Vc MMAE ADC (DAR=4.53) and SWY2110-DXD ADC (DAR=6.93) were 89.7% and 76.2% respectively. SWY2110-JSSW-001 ADC (DAR=7.12) showed 128% of the tumour inhibition effect of SWY2110-DXD ADC (DAR=6.93), and SWY2110-JSSW-001 ADC (DAR=7.12) showed 109% of the tumour inhibition effect of SWY2110-Vc MMAE ADC (DAR=4.53), suggesting that SWY2110-JSSW-001 ADC (DAR=7.12) still had a significant inhibition effect on osimertinib-resistant tumour cells. For details, see Figure 20 and Table 15.

Table 15 Tumour parameters of each group 24 days after administration of SWY2110-ADC

| Group | Animal Number | Dosage (mg/kg) | Administration Route/ Frequency | Tumour Volume ($mm^3$) | TGI(%) Day 24 |
|---|---|---|---|---|---|
| Vehicle | 6 | 0 | i.v./once | 1138.3 | --- |
| SWY2110-Vc MMAE ADC (DAR=4.53) | 6 | 3 | i.v./once | 212.3*** | 89.7 |
| SWY2110-DXD ADC (DAR=6.93) | 6 | 3 | i.v./once | 352.0*** | 76.2 |
| SWY2110-JSSW-001 ADC (DAR=7.12) | 6 | 3 | i.v./once | 131.3*** | 97.5 |
| ***: statistical significance relative to the vehicle group p<0.001 | | | | | |

Assay 10 Efficacy Assay of SWY2110-ADC, SWY2111-ADC, SWY2112-ADC, SWY2113-ADC on human lung adenocarcinoma NCI-H1975 (human lung adenocarcinoma cell) xenograft tumour

1. Assay animals

NU/NU mice, 5-6 weeks old, 30 mice

2. Assay object

To investigate the anti-tumour effect of SWY2110-JSSW-001, SWY2111-JSSW-001, SWY2112-JSSW-001, SWY2113-JSSW-001 drugs in vivo using a human lung adenocarcinoma NCI-H1975 cell (human lung adenocarcinoma cell) xenograft tumour model in NU/NU mice.

3. Drug dosage and grouping

Table 16. Table of animal grouping and dosage

| Group | Animal Number | Dosage (mg/kg) | Administration Route/Frequency |
|---|---|---|---|
| Vehicle | 6 | - | - |
| SWY2110-JSSW-001 ADC (DAR=7.12) | 6 | 1 | i.v./once |
| SWY2111-JSSW-001 ADC (DAR=7.48) | 6 | 1 | i.v./once |
| SWY2112-JSSW-001 ADC (DAR=7.51) | 6 | 1 | i.v./once |
| SWY2113-JSSW-001 ADC (DAR=7.50) | 6 | 1 | i.v./once |
| Note: i.v.: intravenous injection, i.v./once: administration once; | | | |

4. Assay method

In this assay, human lung adenocarcinoma NCI-H1975 cells (human lung adenocarcinoma cells) were used to construct a xenograft tumour model of the tumour in nude mice. When the tumour volume reached approximately 110 $mm^3$, the animals were evenly divided into 5 groups (d0) based on tumour volume according to Table 16, with 6 animals in each group. Each dosage group received a single administration at 1mg/kg. Intravenous administration was given once, with an administration volume of 10mL/kg. The vehicle control group (Vehicle) received 0.9% sodium chloride injection. The assay was terminated on day 21 after the administration. The inhibition effects of SWY2110-JSSW-001, SWY2111-JSSW-001, SWY2112-JSSW-001, SWY2113-JSSW-001 drugs on human lung adenocarcinoma NCI-H1975 xenograft tumour in nude mice were compared.

5. Observation indicator

5.1 Evaluation indicator

See Assay 2: the part of 5.1 Evaluation indicator

6 Result

6.1 Tumour volume

At the end of the assay, compared to the vehicle group, SWY2110-JSSW-001 ADC (DAR=7.12), SWY2112-JSSW-001 ADC (DAR=7.51), SWY2111-JSSW-001 ADC (DAR=7.48), SWY2113-JSSW-001 ADC (DAR=7.50) (P<0.001) all demonstrated significant tumour inhibition effects (P<0.001), achieving tumour inhibition rates of 84.1%, 83.0%, 80.9% and 74.2% (P<0.001) respectively. These results indicated that all four ADCs had significant inhibition effects on human lung adenocarcinoma NCI-H1975 tumour cells. For details, see Figure 21 and Table 17.

Table 17 Tumour parameters of each group 21 days after administration of SWY2110 to SWY2113-ADCs

| Group | Animal Number | Dosage (mg/kg) | Administration Route/ Frequency | Tumour volume ($mm^3$) | TGI (%) Day 21 |
|---|---|---|---|---|---|
| Vehicle | 6 | 0 | i.v./once | 852.4±97.8 | --- |
| SWY2110-JSSW-001 ADC (DAR=7.12) | 6 | 1 | i.v./once | 223.3±71.7*** | 84.2 |
| SWY2111-JSSW-001 ADC (DAR=7.48) | 6 | 1 | i.v./once | 248.4±80.3*** | 80.9 |
| SWY2112-JSSW-001 ADC (DAR=7.51) | 6 | 1 | i.v./once | 231.8±106.5*** | 83.0 |
| SWY2113-JSSW-001 ADC (DAR=7.50) | 6 | 1 | i.v./once | 298.4±108.4*** | 74.2 |
| ***: compared with vehicle group P<0.001 | | | | | |

Assay 11: Efficacy Assay of SWY2110-ADC, SWY2111-ADC, SWY2112-ADC, SWY2113-ADC on human breast cancer MDA-MB-468 (human breast cancer cell) xenograft tumour

1. Assay animals

NOD-SCID mice, 5-6 weeks old, 28 mice

2. Assay object

To investigate the anti-tumour effect of SWY2110-JSSW-001, SWY2111-JSSW-001, SWY2112-JSSW-001, SWY2113-JSSW-001 drugs in vivo using a human breast cancer MDA-MB-468 cell (human breast cancer cell) xenograft tumour model in NOD-SCID mice.

3. Drug dosage and grouping

Table 18. Table of animal grouping and dosage

| Group | Animal Number | Dosage (mg/kg) | Administration Route/Frequency |
|---|---|---|---|
| Vehicle | 8 | 0 | i.v./once |
| SWY2110-JSSW-001 ADC (DAR=7.12) | 5 | 2 | i.v./once |
| SWY2111-JSSW-001 ADC (DAR=7.48) | 5 | 2 | i.v./once |
| SWY2112-JSSW-001 ADC (DAR=7.51) | 5 | 2 | i.v./once |
| SWY2113-JSSW-001 ADC (DAR=7.50) | 5 | 2 | i.v./once |
| Note: i.v.: intravenous injection, i.v./once: administration once; | | | |

4. Assay method

In this assay, human breast cancer MDA-MB-468 cells (human breast cancer cells) were used to construct a

xenograft tumour model of the tumour in mice. When the tumour volume reached approximately 150 $mm^3$, the animals were divided into 5 groups (d0) based on tumour volume according to Table 18, with 8 animals in the control group and 5 animals in the experimental group. Intravenous administration was given once, with an administration volume of 10mL/kg. The vehicle control group (Vehicle) received 0.9% sodium chloride injection.

The assay was terminated on day 28 after the administration. The anti-tumour effects of four ADC drugs in vivo were investigated.

5. Observation indicator

5.1 Evaluation indicator

See the section of Assay 2: 5.1 Evaluation indicator

6 Result

6.1 Tumour volume

At the end of the assay, compared to the vehicle group, the tumour inhibition rates of SWY2110-JSSW-001 (DAR=7.12), SWY2111-JSSW-001 ADC (DAR=7.48), SWY2112-JSSW-001 ADC (DAR=7.51) and SWY2113-JSSW-001 ADC (DAR=7.50) experimental groups reached 65.8%, 65.1%, 58.3%, 47.1% (P<0.001) respectively, all demonstrating good tumour inhibition activity. It suggested that four ADC drugs had significant inhibition effect on human breast cancer MDA-MB-468 tumour cells. For details, see Figure 22 and Table 19.

Table 19 Tumour parameters of each group 28 days after administration of SWY2110 to SWY2113-ADCs

| Group | Animal Number | Dosage (mg/kg) | Administration Route/ Frequency | Tumour Volume ($mm^3$) | TGI (%) Day 28 |
|---|---|---|---|---|---|
| Vehicle | 8 | - | - | 525.0±28.4 | --- |
| SWY2110-JSSW-001 ADC (DAR=7.12) | 5 | 2 | i.v./once | 275.9±50.6*** | 65.8 |
| SWY2111-JSSW-001 ADC (DAR=7.48) | 5 | 2 | i.v./once | 278.8±22.9*** | 65.1 |
| SWY2112-JSSW-001 ADC (DAR=7.51) | 5 | 2 | i.v./once | 304.3±40.6*** | 58.3 |
| SWY2113-JSSW-001 ADC (DAR=7.50) | 5 | 2 | i.v./once | 346.9±46.2*** | 47.1 |
| ***: compared with vehicle group P<0.001 | | | | | |

Assay 12 Efficacy Assay of SWY2110-ADC, SWY2111-ADC, SWY2112-ADC, SWY2113-ADC on human colorectal cancer DiFi (human colorectal cancer cell) xenograft tumour

1. Assay animals

NU/NU mice, 5-6 weeks old, 35 mice

2. Assay object

To investigate the anti-tumour effect of SWY2110-JSSW-001, SWY2111-JSSW-001, SWY2112-JSSW-001, SWY2113-JSSW-001 drugs in vivo using a human colorectal cancer DiFi (human colorectal cancer cell) xenograft tumour model in NU/NU mice.

3. Drug dosage and grouping

Table 20. Table of animal grouping and dosage

| Group | Animal Number | Dosage (mg/kg) | Administration Route/Frequency |
|---|---|---|---|
| Vehicle | 7 | - | - |
| SWY2110-JSSW-001 ADC (DAR=7.12) | 7 | 1 | i.v./qw*twice |
| SWY2111-JSSW-001 ADC | 7 | 1 | i.v./qw*twice |
| (DAR=7.48) | | | |
| SWY2112-JSSW-001 ADC (DAR=7.51) | 7 | 1 | i.v./qw*twice |
| SWY2113-JSSW-001 ADC (DAR=7.50) | 7 | 1 | i.v./qw*twice |
| Note: i.v.: intravenous injection, i.v./qw*twice: administration twice weekly; | | | |

4. Assay method

In this assay, human colorectal cancer DiFi cells (human colorectal cancer cells) were used to construct a xenograft tumour model of the tumour in mice. When the tumour volume reached approximately 100 $mm^3$, the animals were divided into 5 groups (d0) based on tumour volume according to Table 20, with 7 animals in each group. Intravenous administration was given twice, with an administration volume of 10mL/kg. The vehicle control group (Vehicle) received 0.9% sodium chloride injection. The assay was terminated on day 21 after the administration. The anti-tumour effects of four ADC drugs in vivo were investigated. 5. Observation indicator

5.1 Evaluation indicator

See the section of Assay 2: 5.1 Evaluation indicator

6 Result

6.1 Tumour volume

At the end of the assay, compared to the vehicle group, the tumour inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 86.5%, 82.3%, 83.5%, 76.8% (P<0.001) respectively, all demonstrating good tumour inhibition activity. It suggested that four ADC drugs had significant inhibition effect on human colorectal cancer DiFi tumour cells. For details, see Figure 23 and Table 21.

Table 21 Tumour parameters of each group 21 days after administration of SWY2110 to SWY2113-ADCs

| Group | Animal Number | Dosage (mg/kg) | Administration Route/ Frequency | Tumour Volume (mm3) | TGI (%) Day 28 |
|---|---|---|---|---|---|
| Vehicle | 7 | 0 | - | 562.2±272.8 | - |
| SWY2110-JSSW-001 ADC (DAR=7.12) | 7 | 1 | i.v./qw*twice | 161.9±41.2*** | 86.5 |
| SWY2111-JSSW-001 ADC (DAR=7.48) | 7 | 1 | i.v./qw*twice | 180.3±98.8*** | 82.3 |

(continued)

| Group | Animal Number | Dosage (mg/kg) | Administration Route/ Frequency | Tumour Volume (mm3) | TGI (%) Day 28 |
|---|---|---|---|---|---|
| SWY2112-JSSW-001 ADC (DAR=7.51) | 7 | 1 | i.v./qw*twice | 175.3±36.7*** | 83.5 |
| SWY2113-JSSW-001 ADC (DAR=7.50) | 7 | 1 | i.v./qw*twice | 205.3±155.6*** | 76.8 |
| ***: compared with vehicle group P<0.001 | | | | | |

Assay 13: Efficacy Assay of SWY2110-ADC, SWY2111-ADC, SWY2112-ADC, SWY2113-ADC on human lung adenocarcinoma PC9-GR (human lung adenocarcinoma gefitinib-resistance acquired cell line) xenograft tumour

1. Assay animals

NU/NU mice, 5-6 weeks old, 30 mice

2. Assay object

To investigate the anti-tumour effect of SWY2110-JSSW-001, SWY2111-JSSW-001, SWY2112-JSSW-001, SWY2113-JSSW-001 drugs in vivo using a human lung adenocarcinoma cell PC9-GR xenograft tumour model in NU/NU mice.

3. Drug dosage and grouping

Table 22. Table of animal grouping and dosage

| Group | Animal Number | Dosage (mg/kg) | Administration Route/Frequency |
|---|---|---|---|
| Vehicle | 6 | - | - |
| SWY2110-JSSW-001 ADC (DAR=7.12) | 6 | 3 | i.v./once |
| SWY2111-JSSW-001 ADC (DAR=7.48) | 6 | 3 | i.v./once |
| SWY2112-JSSW-001 ADC (DAR=7.51) | 6 | 3 | i.v./once |
| SWY2113-JSSW-001 ADC (DAR=7.50) | 6 | 3 | i.v./once |
| Note: i.v.: intravenous injection, i.v./once: administration once; | | | |

4. Assay method

In this assay, human lung adenocarcinoma cell PC9-GR cells were used to construct a xenograft tumour model of the tumour in mice. When the tumour volume reached approximately 110 $mm^3$, the animals were divided into 5 groups (d0) based on tumour volume according to Table 22, with 6 animals in each group. Intravenous administration was given once, with an administration volume of 10mL/kg. The vehicle control group (Vehicle) received 0.9% sodium chloride injection. The assay was terminated on day 28 after the administration. The anti-tumour effects of four ADC drugs in vivo were investigated. 5. Observation indicator

5.1 Evaluation indicator

See the section of Assay 2: 5.1 Evaluation indicator

6 Result

6.1 Tumour volume

At the end of the assay, compared to the vehicle group, the tumour inhibition rates of SWY2110-JSSW-001 (DAR=7.12), SWY2111-JSSW-001 ADC (DAR=7.48), SWY2112-JSSW-001 ADC (DAR=7.51), SWY2113-JSSW-001 ADC (DAR=7.50) experimental groups reached 99.0%, 100.1%, 92.4%, 89.3% (P<0.001) respectively, all demonstrating good tumour inhibition effect. It suggested that four ADC drugs had significant inhibition effect on human lung adenocarcinoma cell PC9-GR tumour cells. For details, see Figure 24 and Table 23.

Table 23 Tumour parameters of each group 28 days after administration of SWY2110 to SWY2113-ADCs

| Group | Animal Number | Dosage (mg/kg) | Administration Route/ Frequency | Tumour Volume ($mm^3$) | TGI(%) Day 28 |
|---|---|---|---|---|---|
| Vehicle | 6 | - | - | 1159.3 | --- |
| SWY2110-JSSW-001 ADC (DAR=7.12) | 6 | 3 | i.v./once | 124.3*** | 99.0 |
| SWY2111-JSSW-001 | 6 | 3 | i.v./once | 112.9*** | 100.1 |
| ADC (DAR=7.48) | | | | | |
| SWY2112-JSSW-001 ADC (DAR=7.51) | 6 | 3 | i.v./once | 193.0*** | 92.4 |
| SWY2113-JSSW-001 ADC (DAR=7.50) | 6 | 3 | i.v./once | 225.4*** | 89.3 |
| ***: compared with vehicle group P<0.001 | | | | | |

Assay 14 Efficacy Assay of antibody-drug conjugate on human lung adenocarcinoma PC9-DTC (Del19/T790M/C797S, osimertinib-resistant cell) xenograft tumour in NOD-SCID mice

1. Assay animals

NOD-SCID mice, 5-6 weeks old, 18 mice

2. Assay object

To investigate the anti-tumour effect of two patritumab-ADC drugs in vivo using a human lung adenocarcinoma PC9-DTC cell (Del19/T790M/C797S, osimertinib-resistant cell) xenograft tumour model in NU/NU mice.

3. Drug dosage and grouping

Table 24. Table of animal grouping and dosage

| Group | Animal Number | Dosage (mg/kg) | Administration Route/Frequency |
|---|---|---|---|
| Vehicle | 6 | 0 | i.v./qw*3w |
| patritumab-DXD ADC (DAR=7.3) | 6 | 10 | i.v./qw*3w |

(continued)

| Group | Animal Number | Dosage (mg/kg) | Administration Route/Frequency |
|---|---|---|---|
| patritumab-JSSW-001 ADC (DAR=7.36) | 6 | 10 | i.v./qw*3w |
| Note: i.v.: intravenous injection, qw*3w: administration once weekly for three weeks. | | | |

4. Assay method

In this assay, human lung adenocarcinoma PC9-DTC cells (Del19/T790M/C797S, osimertinib-resistant cells) were used to construct a xenograft tumour model of the tumour in nude mice. When the tumour volume reached approximately 115 $mm^3$, the animals were divided into 3 groups (d0) based on tumour volume according to Table 24, with each 6 animals in the vehicle group, the patritumab-DXD ADC (DAR=7.3) group and patritumab-JSSW-001 ADC (DAR=7.36) group. Intravenous administration was given once weekly for 3 weeks, with an administration volume of 10mL/kg. The vehicle control group (Vehicle) received 0.9% sodium chloride injection. The assay was terminated on day 26 after the administration. The inhibition effects of patritumab-DXD ADC (DAR=7.3) and patritumab-JSSW-001 ADC (DAR=7.36) on human lung adenocarcinoma PC9-DTC (Del19/T790M/C797S, osimertinib-resistant cell) xenograft tumour in nude mice were compared.

5. Observation indicator

5.1 Evaluation indicator

See the section of Assay 2: 5.1 Evaluation indicator

6 Result

6.1 Tumour volume

At the end of the assay, the patritumab-JSSW-001 ADC (DAR=7.36) group (P<0.001) exhibited the superior effect of inhibiting the volume of xenograft tumour compared to the patritumab-DXD ADC (DAR=7.3) (P<0.001) group. For details, see Figure 25 and Table 25

Table 25 Tumour parameters of each group 26 days after administration of patritumab-ADC

| Group | Animal Number | Dosage (mg/kg) | Administration Route/ Frequency | Tumour Volume ($mm^3$) | TGI(%) Day 26 |
|---|---|---|---|---|---|
| Vehicle | 6 | 0 | i.v./qw*3w | 465.1±78.9 | --- |
| patritumab-DXD ADC (DAR=7.3) | 6 | 10 | i.v./qw*3w | 132.9±18.8*** | 71.3 |
| patritumab-JSSW-001 ADC (DAR=7.36) | 6 | 10 | i.v./qw*3w | 82.9±14.4*** | 82.3 |
| ***: compared with vehicle group P<0.001 | | | | | |

Assay 15 Efficacy Assay of antibody-drug conjugates on human colon cancer SW620 xenograft tumour in NU/NU mice

1. Assay animals

NU/NU mice, 5-6 weeks old, 18 mice

2. Assay object

To investigate the anti-tumour effect of two patritumab-ADC drugs in vivo using a human colon cancer SW620 cell xenograft tumour model in NU/NU mice.

3. Drug dosage and grouping

Table 26. Table of animal grouping and dosage

| Group | Animal Number | Dosage (mg/kg) | Administration Route/Frequency |
|---|---|---|---|
| Vehicle | 6 | 0 | i.v./single administration |
| patritumab-DXD ADC (DAR=7.3) | 6 | 7 | i.v./single administration |
| patritumab-JSSW-001 ADC (DAR=7.36) | 6 | 7 | i.v./single administration |

4. Assay method

In this assay, human colon cancer SW620 cells were used to construct a xenograft tumour model of the tumour in nude mice. When the tumour volume reached approximately 110 $mm^3$, the animals were divided into 3 groups (d0) based on tumour volume according to Table 26, with each 6 animals in the vehicle group, the patritumab-DXD ADC (DAR=7.3) group and the patritumab-JSSW-001 ADC (DAR=7.36) group. Intravenous administration was given once, with an administration volume of 10mL/kg. The vehicle control group (Vehicle) received 0.9% sodium chloride injection. The assay was terminated on day 26 after the administration. The inhibition effects of patritumab-DXD ADC (DAR=7.3) and patritumab-JSSW-001 ADC (DAR=7.36) on human colon cancer SW620 xenograft tumour in nude mice were compared.

5. Observation indicator

5.1 Evaluation indicator

See the section of Assay 2: 5.1 Evaluation indicator

6 Result

6.1 Tumour volume

At the end of the assay, the patritumab-JSSW-001 ADC (DAR=7.36) group (P<0.001) exhibited the superior effect of inhibiting the volume of xenograft tumour compared to the patritumab-DXD ADC (DAR=7.3) (P<0.001) group. For details, see Figure 26 and Table 27.

Table 27 Tumour parameters of each group 21 days after administration of patritumab-ADC

| Group | Animal Number | Dosage (mg/kg) | Administration Route/ Frequency | Tumour Volume ($mm^3$) | TGI (%) Day 21 |
|---|---|---|---|---|---|
| Vehicle | 6 | 0 | i.v./single administration | 1422.8±416.0 | --- |
| patritumab-DXD ADC (DAR=7.3) | 6 | 7 | i.v./single administration | 461.3±278.1*** | 73.2 |
| patritumab-JSSW-001 ADC (DAR=7.36) | 6 | 7 | i.v./single administration | 187.5±91.6 *** | 94.0 |
| ***: compared to the vehicle control group P<0.001 | | | | | |

Assay 16 Efficacy Assay of antibody-drug conjugates on human colon cancer DiFi xenograft tumour in NU/NU mice

1. Assay animals

NU/NU mice, 5-6 weeks old, 18 mice

2. Assay object

To investigate the anti-tumour effect of two patritumab-ADC drugs in vivo using a human colorectal cancer DiFi cell xenograft tumour model in NU/NU mice.

3. Drug dosage and grouping

Table 28. Table of animal grouping and dosage

| Group | Animal Number | Dosage (mg/kg) | Administration Route/Frequency |
|---|---|---|---|
| Vehicle | 6 | 0 | i.v./single administration |
| patritumab-DXD ADC (DAR=7.3) | 6 | 3 | i.v./single administration |
| patritumab-JSSW-001 ADC (DAR=7.36) | 6 | 3 | i.v./single administration |
| Note: i.v.: intravenous injection. | | | |

4. Assay method

In this assay, human colorectal cancer DiFi cells were used to construct a xenograft tumour model of the tumour in nude mice. When the tumour volume reached approximately 110 $mm^3$, the animals were divided into 3 groups (d0) based on tumour volume according to Table 28, with 6 animals in vehicle group, each 5 animals in patritumab-DXD ADC (DAR=7.3) group and patritumab-JSSW-001 ADC (DAR=7.36) group. Intravenous administration was given once, with an administration volume of 10mL/kg. The vehicle control group (Vehicle) received 0.9% sodium chloride injection. The assay was terminated on day 28 after the administration. The inhibition effects of patritumab-DXD ADC (DAR=7.3) and patritumab-JSSW-001 ADC (DAR=7.36) on human colorectal cancer DiFi xenograft tumour in nude mice were compared.

5. Observation indicator

5.1 Evaluation indicator

See the section of Assay 2: 5.1 Evaluation indicator

6 Result

6.1 Tumour volume

At the end of the assay, the patritumab-JSSW-001 ADC (DAR=7.36) group (P<0.01) exhibited the superior effect of inhibiting the volume of xenograft tumour compared to the patritumab-DXD ADC (DAR=7.3) (P<0.05) group.

For details, see Figure 27 and Table 29.

Table 29 Tumour parameters of each group 28 days after administration of patritumab-ADC

| Group | Animal Number | Dosage (mg/kg) | Administration Route/ Frequency | Tumour Volume ($mm^3$) | TGI(%) Day28 |
|---|---|---|---|---|---|
| Vehicle | 6 | 0 | i.v./qw*3w | 989.8±317.6 | --- |
| patritumab-DXD ADC (DAR=7.3) | 6 | 10 | i.v./qw*3w | 512.5±470.8* | 54.0 |

(continued)

| Group | Animal Number | Dosage (mg/kg) | Administration Route/ Frequency | Tumour Volume ($mm^3$) | TGI(%) Day28 |
|---|---|---|---|---|---|
| patritumab-JSSW-001 ADC (DAR=7.36) | 6 | 10 | i.v./qw*3w | 257.9±189.1** | 82.9 |
| *: compared with vehicle group P<0.05, **: compared with vehicle group P<0.01 | | | | | |

Assay 17: Safety Evaluation Study

In this assay, six appropriately aged crab-eating macaques were selected, with three males and three females, and were intravenously injected with SWY2110-JSSW-001, SWY2111-JSSW-001, and SWY2113-JSSW-001. The dosing regimen was designed as shown in the table below, with intravenous injections once every three weeks for three successive injections. After the administration, a continuous observation was made for 7 days, a general observation was made twice daily, and a detailed observation was made once daily. Changes in body weight were observed before and after administration, and hematological tests were performed. Assay results showed that compared to the ADCs prepared with pH-dependent antibodies (SWY2111-JSSW-001 and SWY2113-JSSW-001), SWY2110-JSSW-001 exhibited reduced toxicity in skin and gastrointestinal tracts, and no obvious target-related toxicity (such as skin ulceration, scabbing, loose stools, etc.), indicating that the pH-modified ADCs had reduced toxicity to normal tissues, however, there was no significant difference in the haematological toxicity caused by three ADC molecules.

Table 30: Pre-toxicology experiment design scheme

| Administration frequency | Q3W×3 | | | |
|---|---|---|---|---|
| | Group | D1 | D22 | D43 |
| Administration dosage | Group 1: SWY2110-JSSW-001 | 6mg/kg | 30mg/kg | 30mg/kg |
| | Group 2: SWY2111-JSSW-001 | 6mg/kg | 30mg/kg | 30mg/kg |
| | Group 3: SWY2113-JSSW-001 | 6mg/kg | 30mg/kg | 30mg/kg |
| Animal number | 1 animal/sex/group | | | |

Table 31: Pre-toxicity assay results

| | |
|---|---|
| General observation | Group 1 showed loose stools, skin flakes on limbs, ulceration, and scabbing;<br>Group 2 showed soft stools, mild skin flakes, and no ulceration;<br>Group 3 showed no abnormalities. |
| Body Weight | Group 1 showed a decrease in body weight within 7 days after each administration, with partial recovery before the next administration;<br>Group 2 showed a slight decrease in body weight within 7 days after each administration, with full recovery before the next administration;<br>Group 3 showed no significant decrease in body weight within 7 days after each administration. |
| Hematology | Group 1 HGB, WBC, LYMP↓;<br>Group 2 HGB, WBC, LYMP↓; |
| | Group 3 HGB, WBC, LYMP↓; |
| HGB: hemoglobin; WBC: white blood count; LYMP: lymphocyte absolute number. | |

## Claims

1. An antibody-drug conjugate represented by formula I or formula II, and pharmaceutically acceptable salts, hydrates, solvates, stereoisomers or isotopic labels thereof:

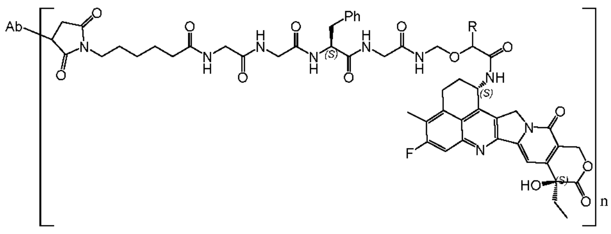

formula I

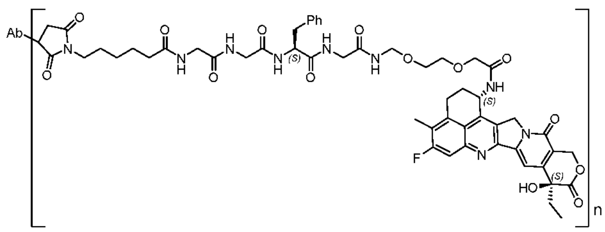

formula II

wherein Ab is an antibody targeting HER2, HER3 or EGFR or an antigen-binding fragment thereof, R is $C_{1-6}$alkyl, and n is an integer of 1-8 or a decimal of 1-8.

**2.** The antibody-drug conjugate according to claim 1, and pharmaceutically acceptable salts, hydrates, solvates, stereoisomers or isotopic labels thereof, wherein formula I is formula Ia or formula Ib:

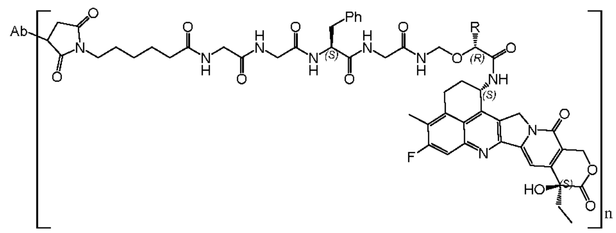

formula Ia

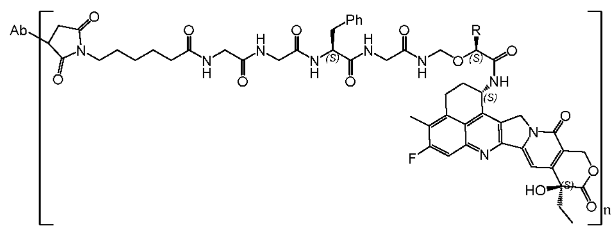

formula Ib

wherein in formula Ia and formula Ib, Ab, R and n are defined as in formula I.

**3.** The antibody-drug conjugate according to claim 1, and pharmaceutically acceptable salts, hydrates, solvates, stereoisomers or isotopic labels thereof, wherein formula I is formula I-1, formula Ia-1 or formula Ib-1:

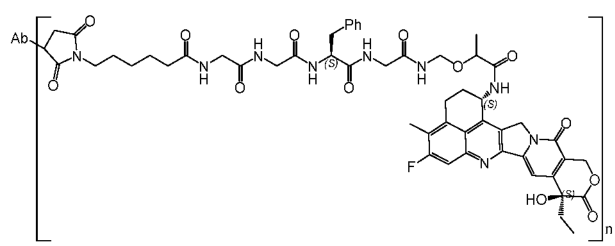

formula I-1

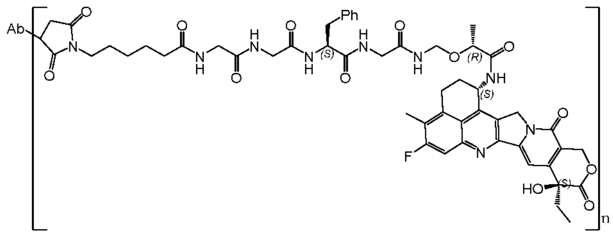

formula Ia-1

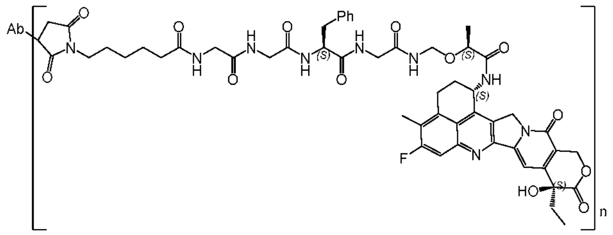

formula Ib-1

wherein in formula I-1, formula Ia-1 and formula Ib-1, Ab and n are defined as in formula I.

**4.** The antibody-drug conjugate according to any of claims 1-3, and pharmaceutically acceptable salts, hydrates, solvates, stereoisomers or isotopic labels thereof, wherein

in case that the Ab is an antibody targeting HER2 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:1, the amino acid sequence of HCDR2 is represented by SEQ ID NO:2, the amino acid sequence of HCDR3 is represented by SEQ ID NO:3, and/or the amino acid sequence of LCDR1 is represented by SEQ ID NO:4, the amino acid sequence of LCDR2 is represented by SEQ ID NO:5, the amino acid sequence of LCDR3 is represented by SEQ ID NO:6; further preferably, in case that the Ab is an antibody targeting HER2 or an antigen-binding fragment thereof, it comprises a variable region of a heavy chain (HV) and a variable region of a light chain (LV), wherein the amino acid sequence of HV is represented by SEQ ID NO:7, the amino acid sequence of LV is represented by SEQ ID NO:8; more preferably, in case that the Ab is an antibody targeting HER2 or an antigen-binding fragment thereof, it comprises a heavy chain (HC) and a light chain (LC), wherein the amino acid sequence of HC is represented by SEQ ID NO:9, the amino acid sequence of LC is represented by SEQ ID NO:10;

in case that the Ab is an antibody targeting HER3 or an antigen-binding fragment thereof, it comprises a variable region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:11, the amino acid sequence of HCDR2 is represented by SEQ ID NO: 12, the amino acid sequence of HCDR3 is represented by SEQ ID NO:13, and/or the amino acid sequence of LCDR1 is represented by SEQ ID NO:14, the amino acid sequence of LCDR2 is represented by SEQ ID NO: 15, the amino acid sequence of LCDR3 is represented by SEQ ID NO:16; further preferably, in case that the Ab is an antibody targeting HER3 or an antigen-binding fragment thereof, it comprises a variable region of a heavy chain (HV) and a variable region of a light chain (LV), wherein the amino acid sequence of HV is represented by SEQ ID NO: 17, the amino acid sequence of LV is represented by SEQ ID NO:18; more preferably, in case that the Ab is an antibody targeting HER3 or an antigen-binding fragment thereof, it comprises a heavy chain (HC) and a light chain (LC), wherein the amino acid sequence of HC is represented by SEQ ID NO:19, the amino acid sequence of LC is represented by SEQ ID NO:20;

in case that the Ab is an antibody targeting EGFR or an antigen-binding fragment thereof, it comprises a variable

region of a heavy chain (HV) and a variable region of a light chain (LV), wherein, a sequence of the variable region of the heavy chain comprises a heavy chain complementarity determining region 1 (HCDR1), a heavy chain complementarity determining region 2 (HCDR2) and a heavy chain complementarity determining region 3 (HCDR3), a sequence of the variable region of the light chain comprises a light chain complementarity determining region 1 (LCDR1), a light chain complementarity determining region 2 (LCDR2) and a light chain complementarity determining region 3 (LCDR3), wherein the amino acid sequence of HCDR1 is represented by SEQ ID NO:21 or SEQ ID NO:31 or SEQ ID NO:35 or SEQ ID NO:38, the amino acid sequence of HCDR2 is represented by SEQ ID NO:22, the amino acid sequence of HCDR3 is represented by SEQ ID NO:23 or SEQ ID NO:32, and/or the amino acid sequence of LCDR1 is represented by SEQ ID NO:24, the amino acid sequence of LCDR2 is represented by SEQ ID NO:25, the amino acid sequence of LCDR3 is represented by SEQ ID NO:26; further preferably, in case that the Ab is an antibody targeting EGFR or an antigen-binding fragment thereof, it comprises a variable region of a heavy chain (HV) and a variable region of a light chain (LV), wherein the amino acid sequence of HV is represented by SEQ ID NO:27 or SEQ ID NO:33 or SEQ ID NO:36 or SEQ ID NO:39, the amino acid sequence of LV is represented by SEQ ID NO:28; more preferably, in case that the Ab is an antibody targeting EGFR or an antigen-binding fragment thereof, it comprises a heavy chain (HC) and a light chain (LC), wherein the amino acid sequence of HC is represented by SEQ ID NO:29 or SEQ ID NO:34 or SEQ ID NO:37 or SEQ ID NO:40, the amino acid sequence of LC is represented by SEQ ID NO:30.

**5.** The antibody-drug conjugate according to any one of claims 1-4 and pharmaceutically acceptable salts, hydrates, solvates, stereoisomers or isotopic labels thereof, wherein said antibody is preferably a monoclonal antibody, the monoclonal antibody is preferably selected from human-source antibody, humanized antibody, and chimeric antibody.

**6.** The antibody-drug conjugate according to any one of claims 1-4, and pharmaceutically acceptable salts, hydrates, solvates, stereoisomers or isotopic labels thereof, wherein the antigen-binding fragment is selected from Fab', (Fab')2, Fab, Fv, scFv, dAb.

**7.** A linker-drug compound represented by formula III, and pharmaceutically acceptable salts, hydrates, solvates, stereoisomers or isotopic labels thereof:

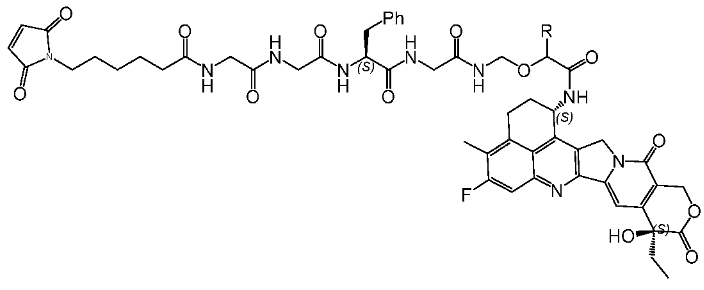

formula III

wherein R is $C_{1-6}$alkyl, preferably $C_{1-3}$alkyl, further preferably methyl, ethyl, propyl, isopropyl.

**8.** The linker-drug compound according to claim 7, and pharmaceutically acceptable salts, hydrates, solvates, stereoisomers or isotopic labels thereof, wherein formula III is formula IIIa or formula IIIb:

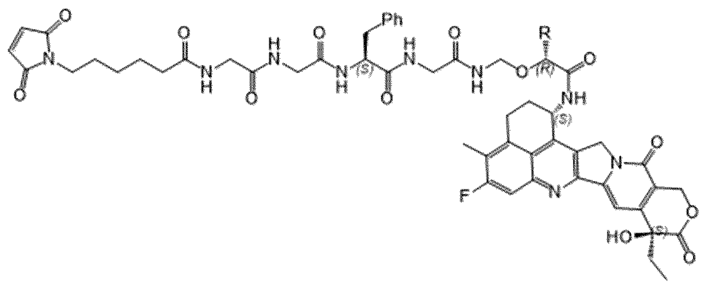

formula IIIa

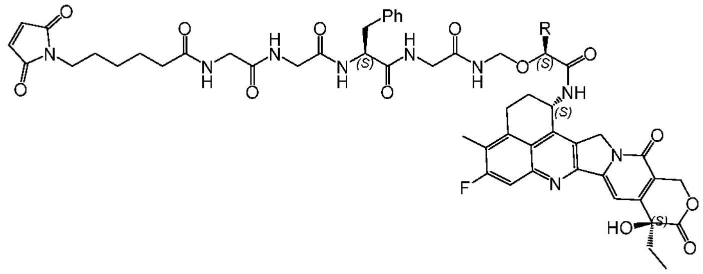

formula IIIb.

9. A linker-drug compound represented by the following formulae, and pharmaceutically acceptable salts, hydrates, solvates, stereoisomers or isotopic labels thereof:

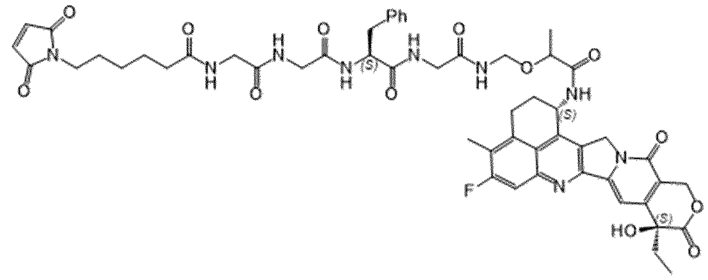

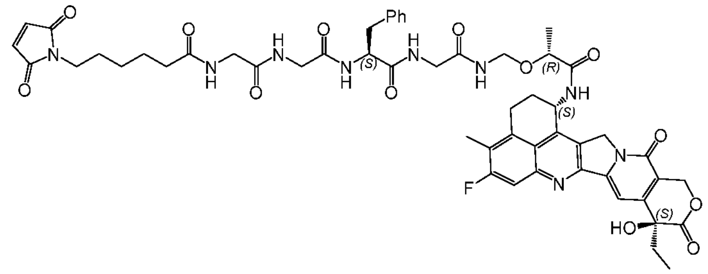

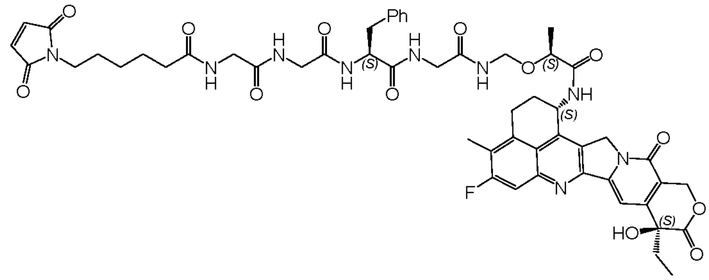

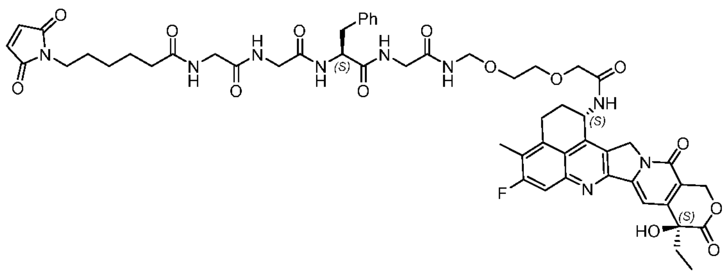

.

10. A pharmaceutical composition, which contains an antibody-drug conjugate according to any one of claims 1-6, and pharmaceutically acceptable salts, hydrates, solvates, stereoisomers or isotopic labels thereof.

11. Use of the antibody-drug conjugate according to any one of claims 1-6 and pharmaceutically acceptable salts, hydrates, solvates, stereoisomers or isotopic labels thereof in manufacture of a medicament for treating proliferative

diseases.

**12.** Use according to claim 11, **characterized in that** the proliferative disease is a disease related to abnormal expression of HER2, HER3 or EGFR, including cancer, wherein the cancer is preferably selected from breast cancer, ovarian cancer, cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma).

**13.** Use of the antibody-drug conjugate according to any one of claims 1-6 and pharmaceutically acceptable salts, hydrates, solvates, stereoisomers or isotopic labels thereof in manufacture of a medicament for treating drug-resistant proliferative diseases.

**14.** Use according to claim 13, wherein the drug-resistant proliferative diseases is a drug-resistant disease related to abnormal expression of HER2, HER3 or EGFR, including cancer, the drug-resistant cancer is preferably a drug-resistant cancer caused by mutation in HER2, HER3 or EGFR genes, the cancer is preferably breast cancer, ovarian cancer, cervical carcinoma, uterine cancer, prostate cancer, kidney cancer, urethra cancer, bladder cancer, liver cancer, gastric cancer, endometrial cancer, salivary gland cancer, esophageal cancer, melanoma, neuroglioma, neuroblastoma, sarcoma, lung cancer (e.g., small cell lung cancer and non-small cell lung cancer), colon cancer, rectal cancer, colorectal cancer, leukemia (e.g., acute lymphoblastic leukemia, acute myeloid leukemia, acute promyelocytic leukemia, chronic myelocytic leukemia, chronic lymphocytic leukemia), bone cancer, skin cancer, thyroid cancer, pancreas cancer or lymphoma (e.g., Hodgkin lymphoma, non-Hodgkin lymphoma or relapsed anaplastic large cell lymphoma), further preferably colon cancer, rectal cancer, lung cancer or pancreas cancer.

**15.** Use according to claim 13, wherein the drug resistance refers to being resistant to receptor tyrosine kinase inhibitors, further preferably resistant to first-generation, second-generation or third-generation EGFR inhibitors, still further preferably resistant to gefitinib, erlotinib, icotinib, afatinib, dacomitinib, osimertinib or almonertinib, especially resistant to gefitinib, afatinib, osimertinib or almonertinib, more preferably resistant to gefitinib or osimertinib.

**16.** A process for preparing the antibody-drug conjugate according to any one of claims 1-6, and pharmaceutically acceptable salts, hydrates, solvates, stereoisomers or isotopic labels thereof, which comprises the following steps:

S1. Reduction of antibody;
S2. Conjugation of antibody and linker-drug;
S3. Purification of antibody-drug conjugates.

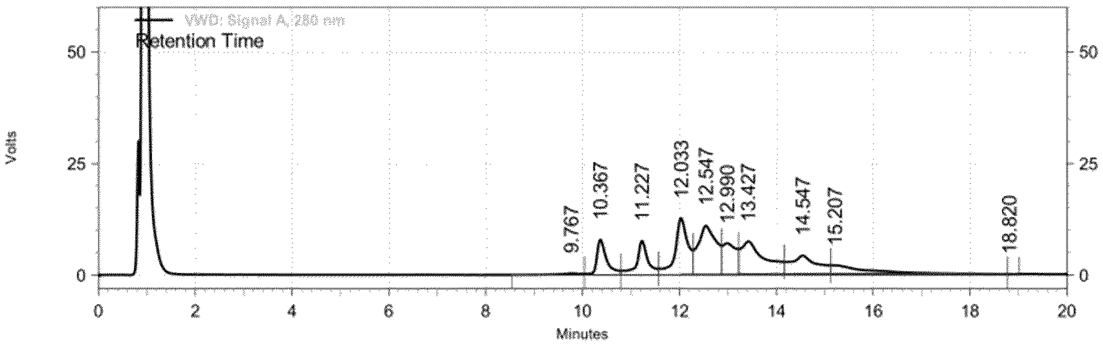


Fig.1

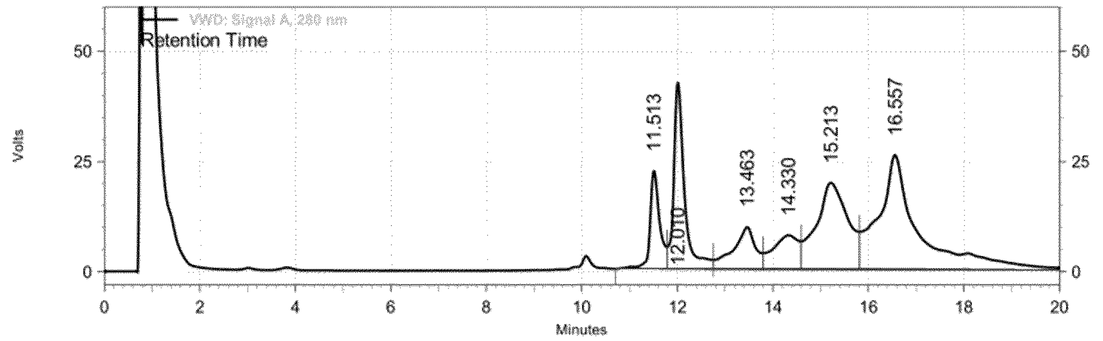


Fig.2

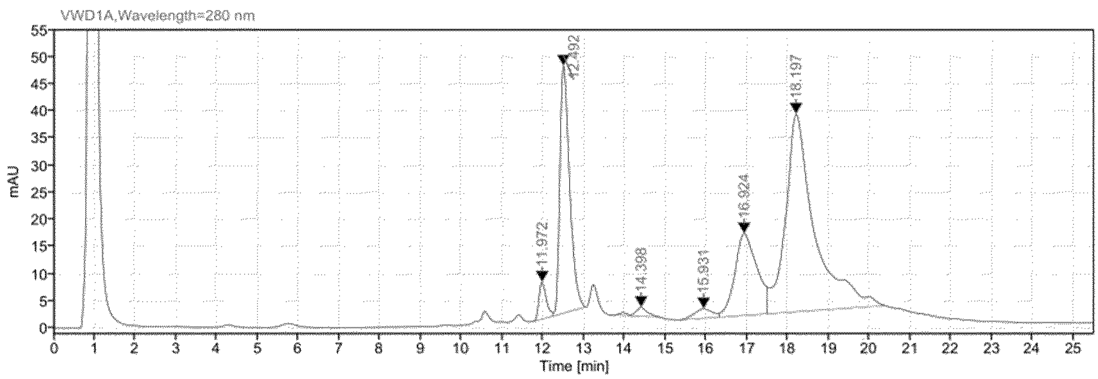


Fig.3

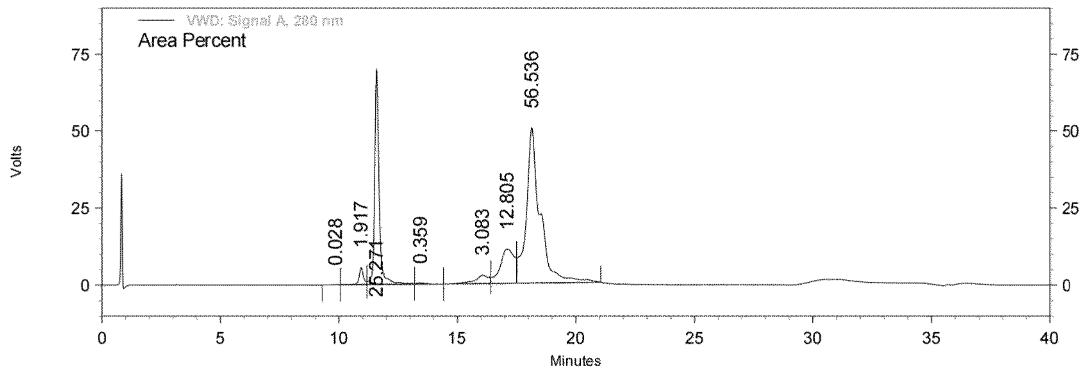


Fig.4

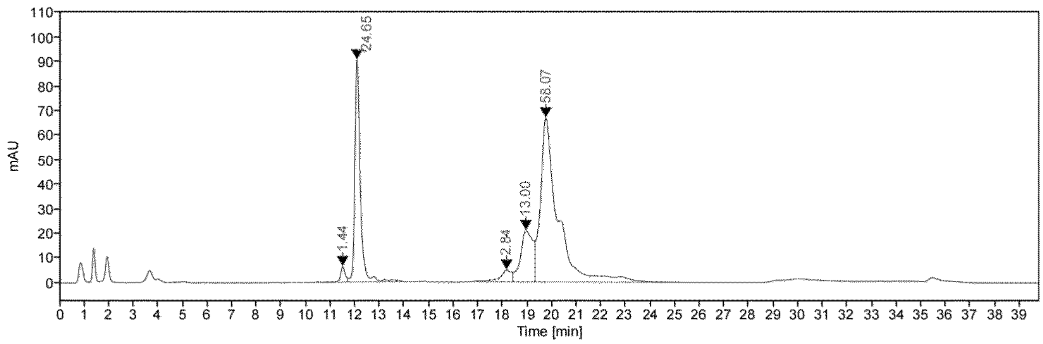


Fig.5

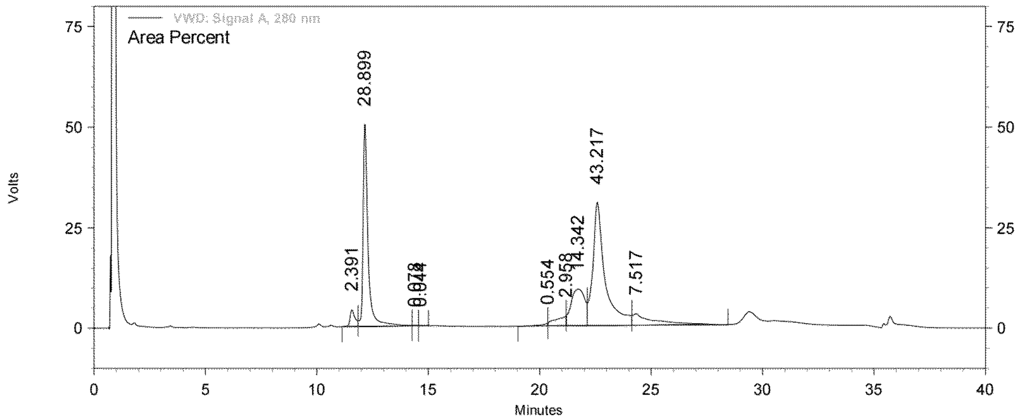


Fig.6

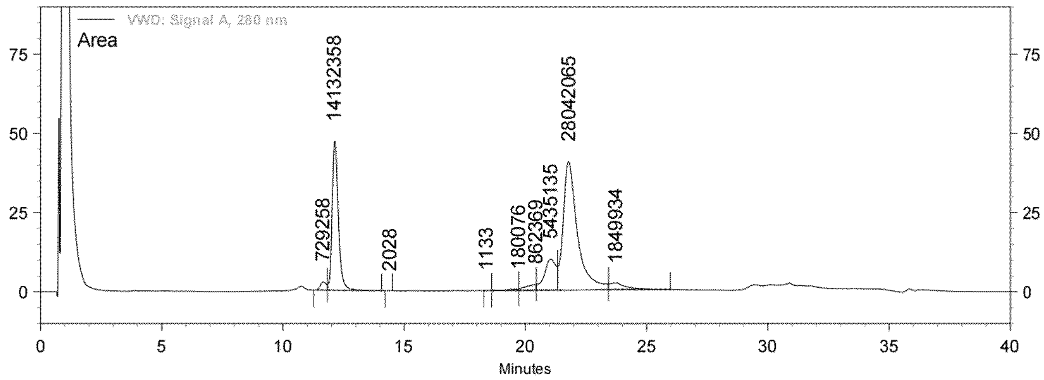


Fig.7

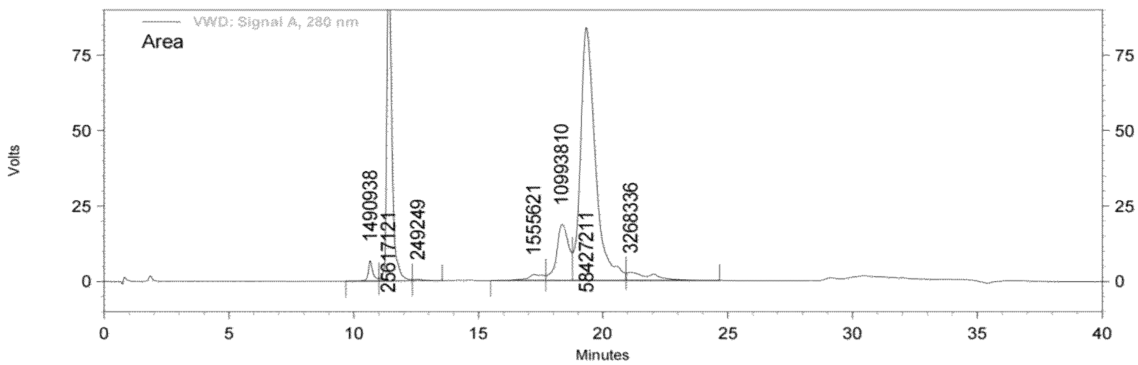


Fig.8

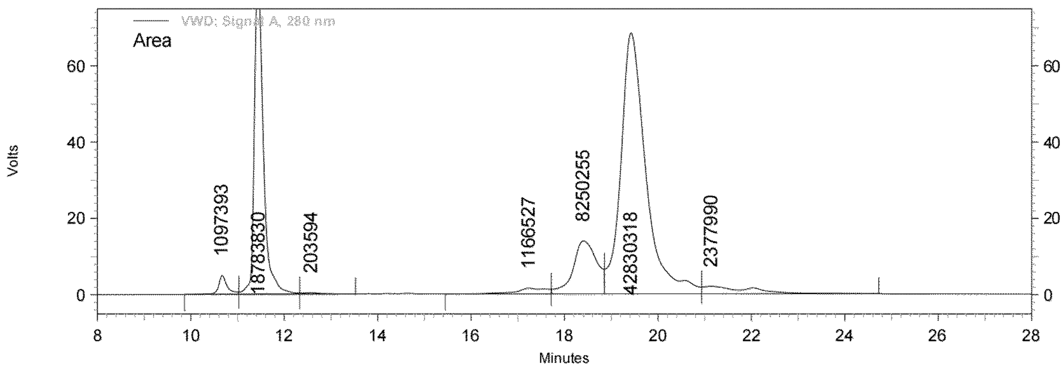


Fig.9

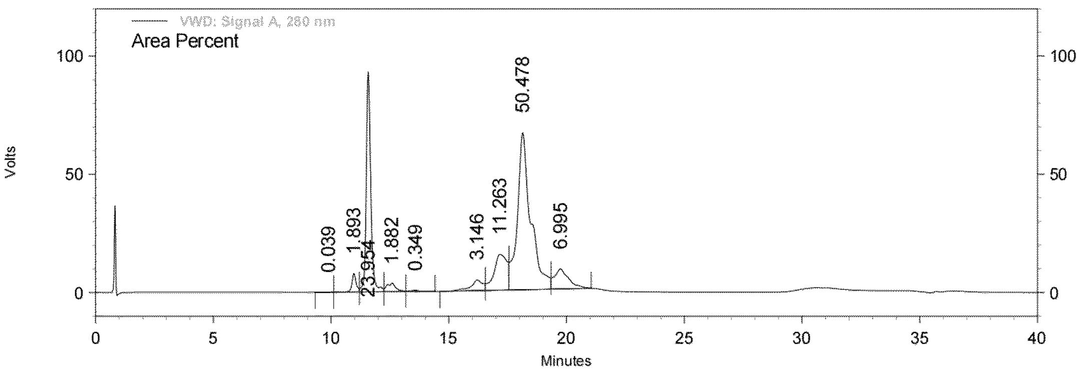


Fig.10

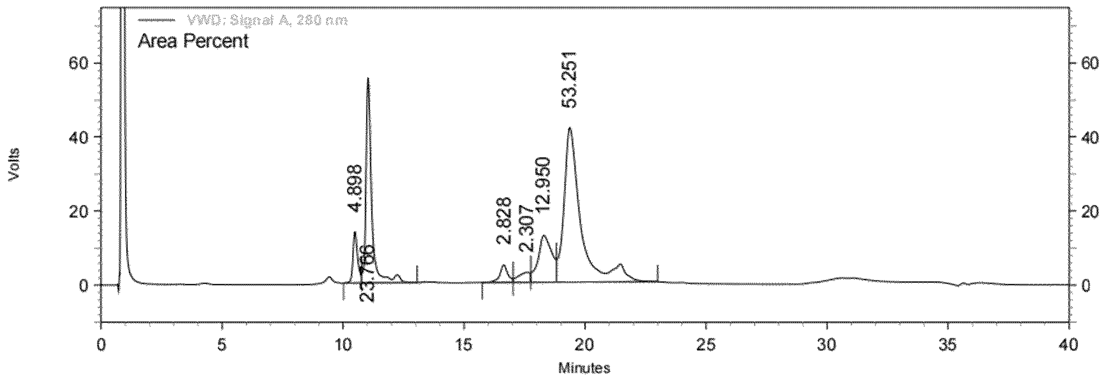


Fig.11

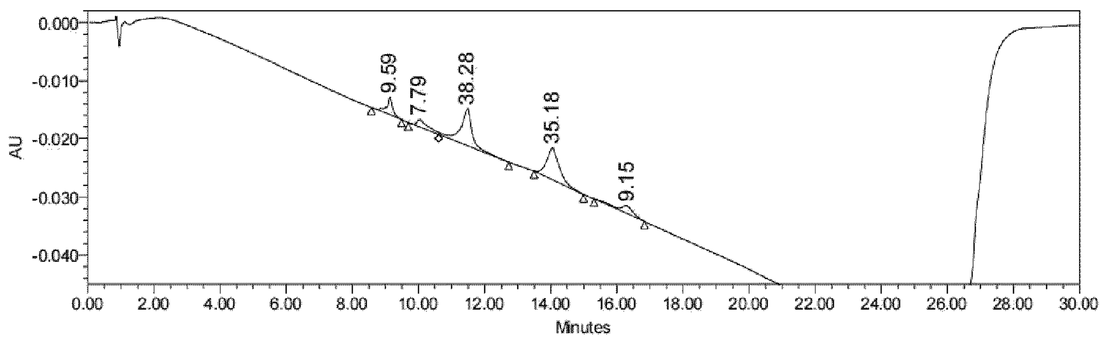


Fig.12

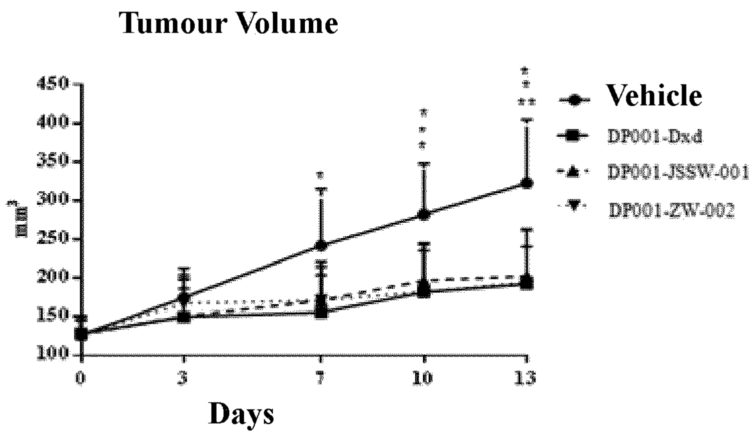


Fig.13

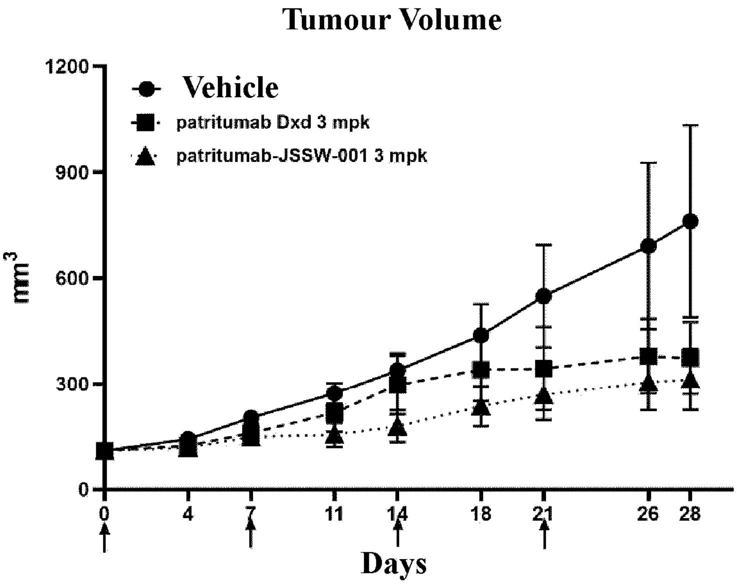


Fig.14

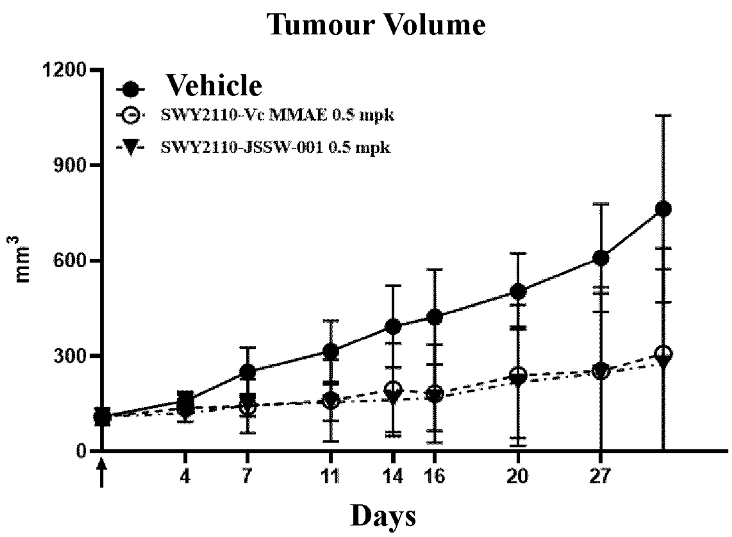


Fig.15

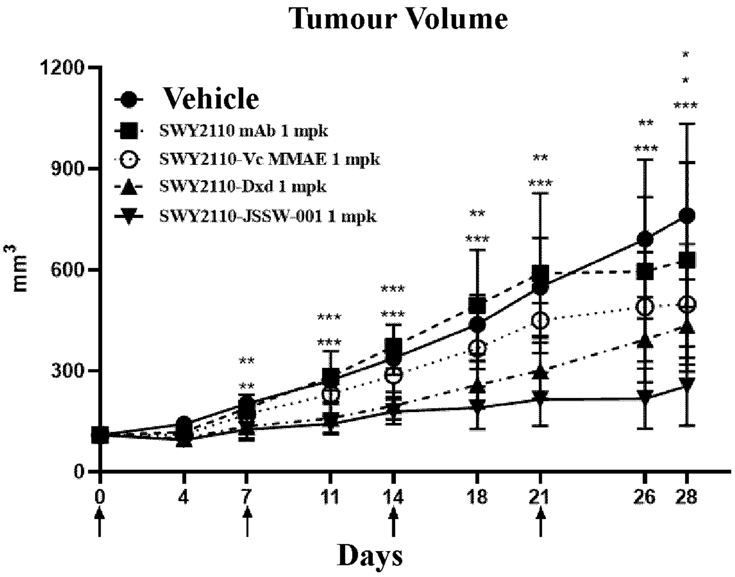


Fig.16

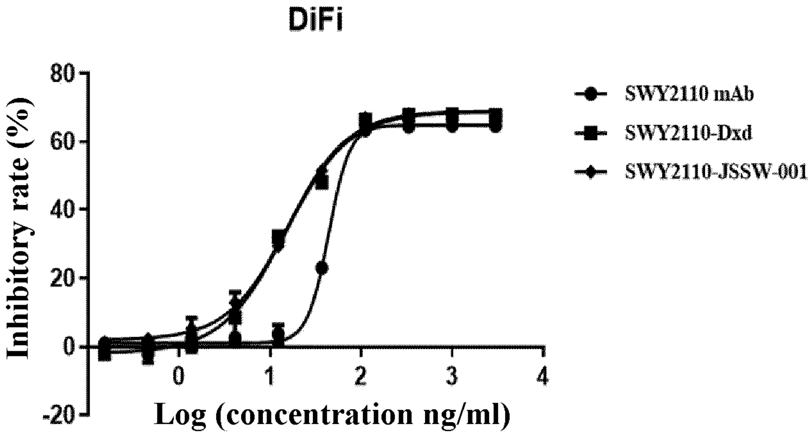


Fig.17

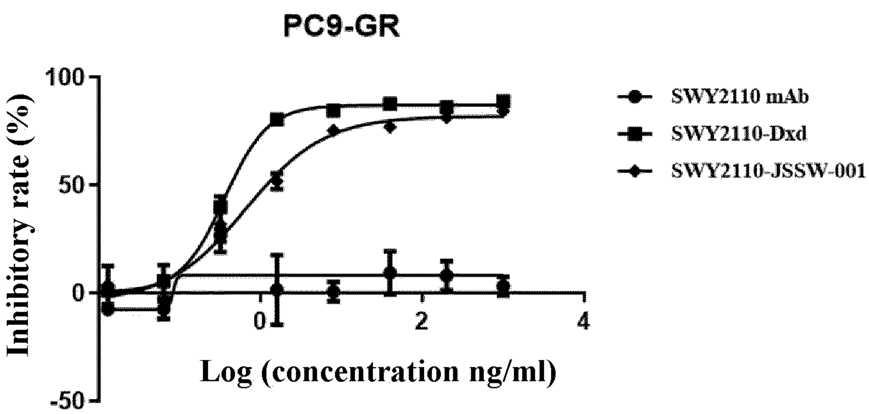


Fig.18

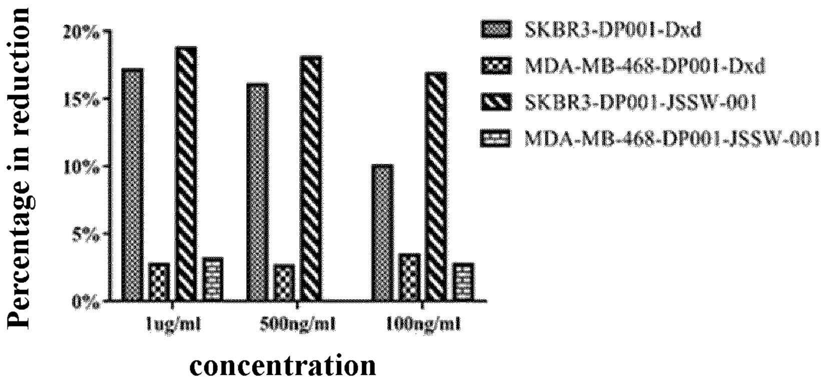


Fig.19

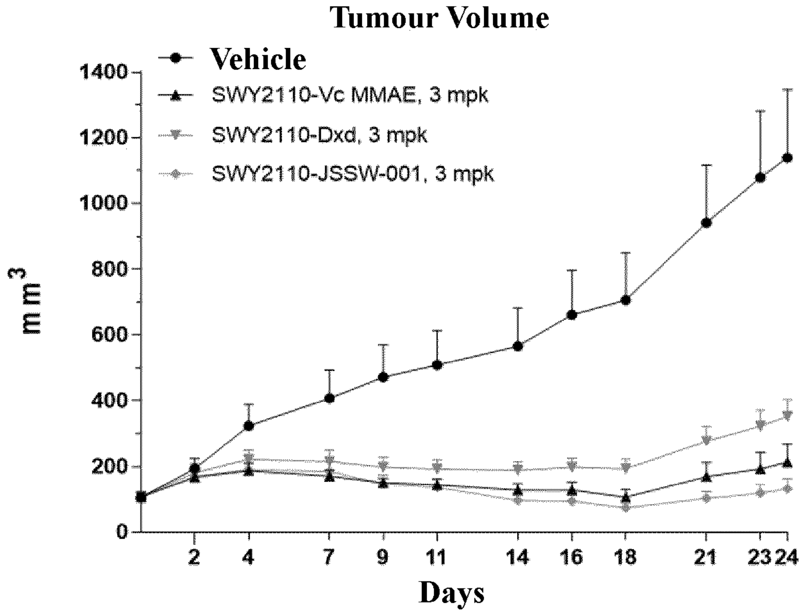


Fig.20

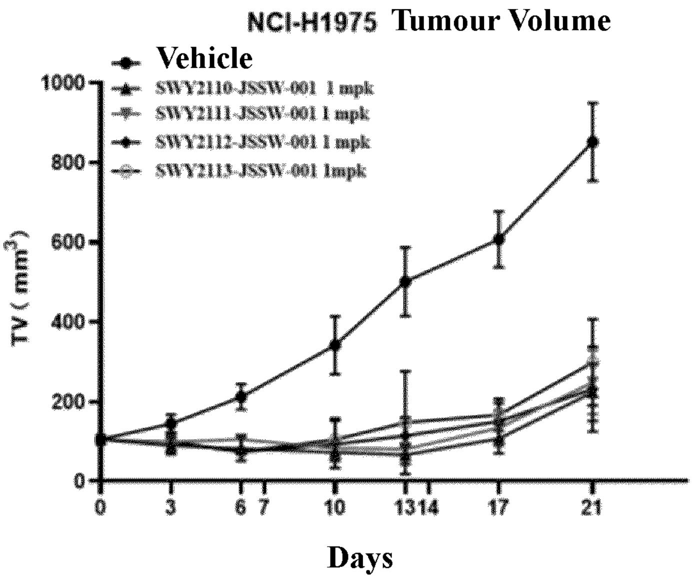


Fig.21

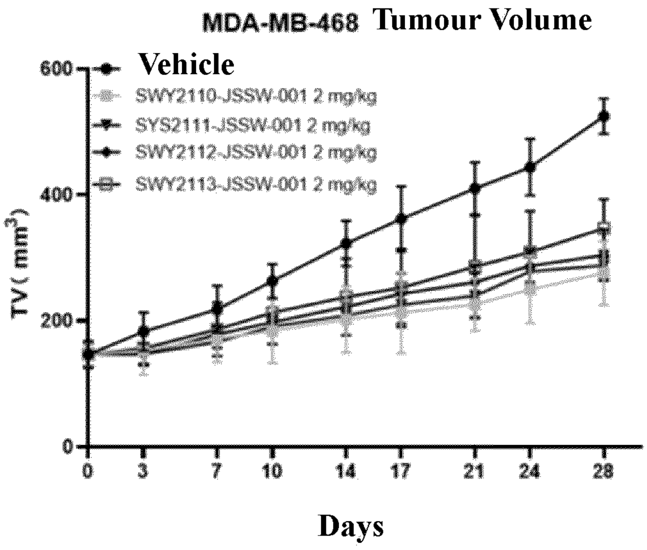


Fig.22

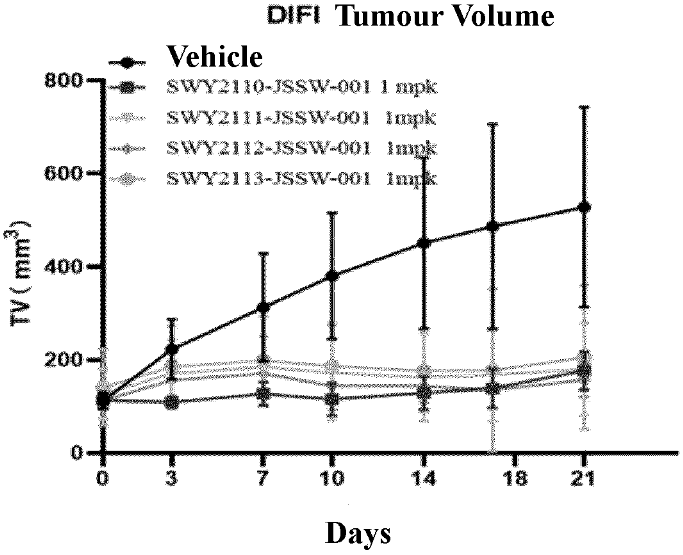


Fig.23

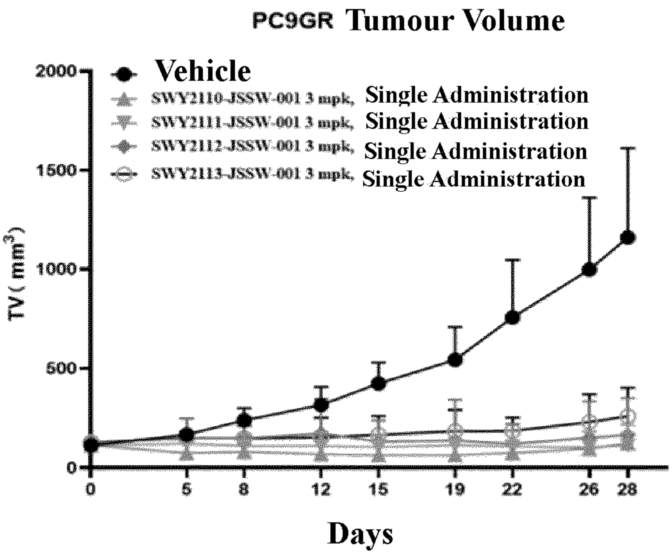


Fig.24

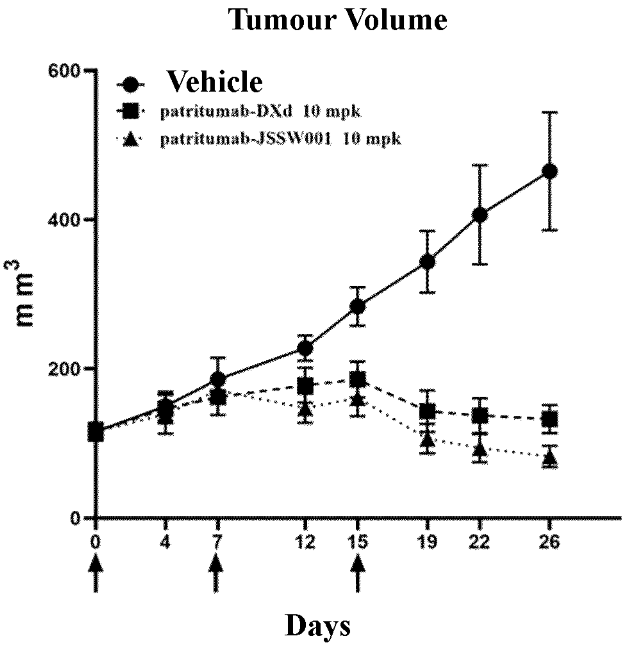
Tumour Volume
Vehicle
patritumab-DXd 10 mpk
patritumab-JSSW001 10 mpk
600
400
200
0
m m3
0
4
7
12
15
19
22
26
Days

Fig.25

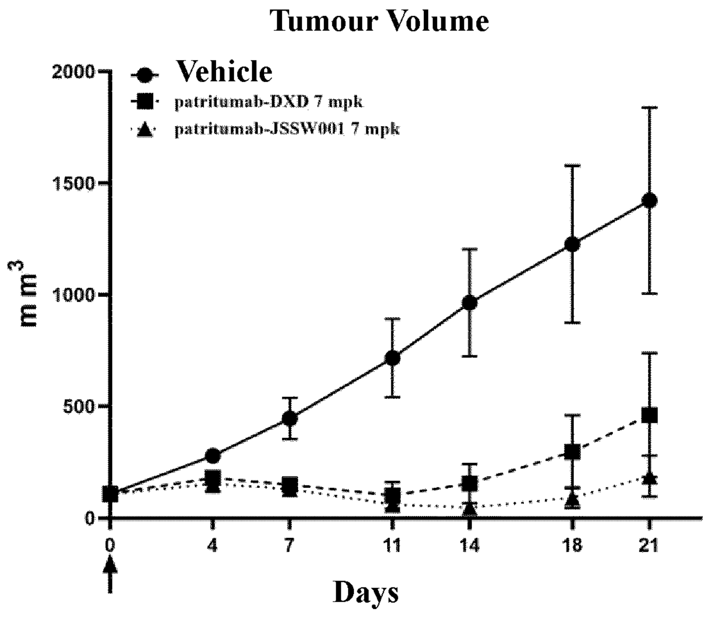
Tumour Volume
Vehicle
patritumab-DXD 7 mpk
patritumab-JSSW001 7 mpk
2000
1500
1000
500
0
m m3
0
4
7
11
14
18
21
Days

Fig.26

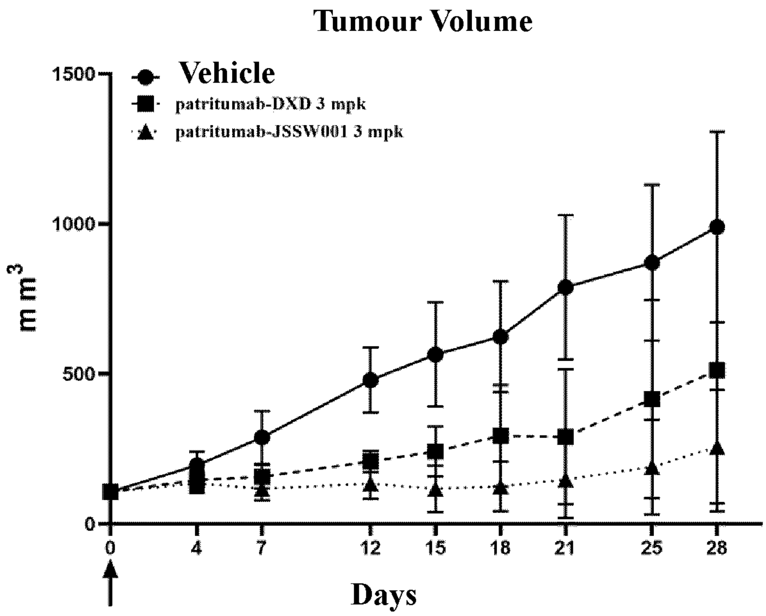
Tumour Volume
Vehicle
patritumab-DXD 3 mpk
patritumab-JSSW001 3 mpk
1500
1000
500
0
m m3
0
4
7
12
15
18
21
25
28
Days

Fig.27

## INTERNATIONAL SEARCH REPORT

International application No.
**PCT/CN2022/132627**

**A. CLASSIFICATION OF SUBJECT MATTER**

A61K47/68(2017.01)i;A61K39/395(2006.01)i;A61P35/00(2006.01)i

According to International Patent Classification (IPC) or to both national classification and IPC

**B. FIELDS SEARCHED**

Minimum documentation searched (classification system followed by classification symbols)

A61K,A61P

Documentation searched other than minimum documentation to the extent that such documents are included in the fields searched

Electronic data base consulted during the international search (name of data base and, where practicable, search terms used)

CNABS, DWPI, CNTXT, EPTXT, WOTXT, USTXT, CNKI, ISI web of science , baidu, Pudmed, STN on web: 石药集团巨石生物制药有限公司, 姚兵, 偶联, conjugate, 喜树碱, 依喜替康, exatecan, camptothecin, ph依赖, HER2, HER3, EGFR, SEQ ID NOs: 1-40, 171335-80-1, 2886725-88-6, 2761690-75-7, 2761690-74-6

**C. DOCUMENTS CONSIDERED TO BE RELEVANT**

| Category* | Citation of document, with indication, where appropriate, of the relevant passages | Relevant to claim No. |
|---|---|---|
| X | WO 2020063676 A1 (JIANGSU HENGRUI MEDICINE CO., LTD. et al.) 02 April 2020 (2020-04-02)<br>claims 22-28, 32-34, and 38-46 | 1-3,7-16 |
| Y | WO 2020063676 A1 (JIANGSU HENGRUI MEDICINE CO., LTD. et al.) 02 April 2020 (2020-04-02)<br>claims 22-28, 32-34, and 38-46 | 4-6,10-16 |
| Y | CN 112566942 A (DAIICHI SANKYO CO., LTD.) 26 March 2021 (2021-03-26)<br>description, paragraphs 597-613 | 4-6,10-16 |
| Y | WO 2016131409 A1 (SHANGHAI MIRACOGEN INC.) 25 August 2016 (2016-08-25)<br>description, page 19 | 4-6,10-16 |
| Y | CN 106163559 A (DAICHI SANKYO CO., LTD. et al.) 23 November 2016 (2016-11-23)<br>claims 1-38 | 4-6,10-16 |
| Y | CN 109922864 A (THE NEW DRUG RESEARCH AND DEVELOPMENT CO., LTD., BEIJING HISTORY) 21 June 2019 (2019-06-21)<br>description, page 20, paragraph 171, and SEQ ID NOs: 7-8 | 4-6,10-16 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 February 2023** | **11 February 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/132627**

**C. DOCUMENTS CONSIDERED TO BE RELEVANT**

| Category* | Citation of document, with indication, where appropriate, of the relevant passages | Relevant to claim No. |
|---|---|---|
| A | CN 112125915 A (SICHUAN BAILI PHARMACEUTICAL CO., LTD.) 25 December 2020 (2020-12-25)<br>entire document | 1-16 |
| A | CN 111689980 A (SICHUAN BAILI PHARMACEUTICAL CO., LTD.) 22 September 2020 (2020-09-22)<br>entire document | 1-16 |
| A | WO 2020063673 A1 (JIANGSU HENGRUI MEDICINE CO., LTD. et al.) 02 April 2020 (2020-04-02)<br>entire document | 1-16 |
| A | WO 2021147993 A1 (JIANGSU HENGRUI MEDICINE CO., LTD.) 29 July 2021 (2021-07-29)<br>entire document | 1-16 |

Form PCT/ISA/210 (second sheet) (July 2022)

**INTERNATIONAL SEARCH REPORT**

International application No.
**PCT/CN2022/132627**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2022/132627**

| Patent document cited in search report | Publication date (day/month/year) | Patent family member(s) | Publication date (day/month/year) |
|---|---|---|---|
| WO 2020063676 A1 | 02 April 2020 | CA 3114137 A1 | 02 April 2020 |
| | | JP 2022511351 A | 31 January 2022 |
| | | BR 112021004656 A2 | 01 June 2021 |
| | | EP 3858386 A1 | 04 August 2021 |
| | | EP 3858386 A4 | 12 October 2022 |
| | | KR 20210066833 A | 07 June 2021 |
| | | US 2021353764 A1 | 18 November 2021 |
| | | AU 2019349207 A1 | 08 April 2021 |
| | | TW 202027795 A | 01 August 2020 |
| CN 112566942 A | 26 March 2021 | AU 2019311557 A1 | 04 February 2021 |
| | | CA 3107417 A1 | 30 January 2020 |
| | | KR 20210038904 A | 08 April 2021 |
| | | IL 280295 A | 25 March 2021 |
| | | TW 202019972 A | 01 June 2020 |
| | | SG 11202100653 YA | 25 February 2021 |
| | | US 2021283269 A1 | 16 September 2021 |
| | | JPWO 2020022363 A1 | 02 August 2021 |
| | | EP 3828206 A1 | 02 June 2021 |
| | | EP 3828206 A4 | 20 April 2022 |
| | | BR 112021001194 A2 | 27 April 2021 |
| | | WO 2020022363 A1 | 30 January 2020 |
| WO 2016131409 A1 | 25 August 2016 | US 2018036423 A1 | 08 February 2018 |
| | | US 10792370 B2 | 06 October 2020 |
| CN 106163559 A | 23 November 2016 | MX 2016010533 A | 12 December 2016 |
| | | KR 20220068270 A | 25 May 2022 |
| | | KR 102445502 B1 | 21 September 2022 |
| | | KR 20200077620 A | 30 June 2020 |
| | | KR 102186027 B1 | 03 December 2020 |
| | | CA 2939802 A1 | 15 October 2015 |
| | | CA 2939802 C | 01 November 2022 |
| | | JP 6148422 B1 | 14 June 2017 |
| | | JP 2017197515 A | 02 November 2017 |
| | | KR 20190004837 A | 14 January 2019 |
| | | KR 102127623 B1 | 29 June 2020 |
| | | JP 2022180416 A | 06 December 2022 |
| | | TW 201542230 A | 16 November 2015 |
| | | TWI 704928 B | 21 September 2020 |
| | | RS 61711 B1 | 31 May 2021 |
| | | LT 3129063 T | 25 June 2021 |
| | | US 2019151328 A1 | 23 May 2019 |
| | | US 11298359 B2 | 12 April 2022 |
| | | HRP 20210593 T1 | 14 May 2021 |
| | | RU 2019143766 A | 20 February 2020 |
| | | KR 20200136052 A | 04 December 2020 |
| | | KR 102239413 B1 | 12 April 2021 |
| | | JP 2017503784 A | 02 February 2017 |
| | | JP 6105171 B2 | 29 March 2017 |
| | | KR 20210115056 A | 24 September 2021 |
| | | KR 102351755 B1 | 14 January 2022 |
| | | JP 2019135248 A | 15 August 2019 |

Form PCT/ISA/210 (patent family annex) (July 2022)

**INTERNATIONAL SEARCH REPORT**
**Information on patent family members**

International application No.
**PCT/CN2022/132627**

| Patent document cited in search report | Publication date (day/month/year) | Patent family member(s) | Publication date (day/month/year) |
|---|---|---|---|
| | | JP 6707696 B2 | 10 June 2020 |
| | | IL 248239 A0 | 30 November 2016 |
| | | IL 248239 B | 01 July 2022 |
| | | SI 3129063 T1 | 31 August 2021 |
| | | TW 202108176 A | 01 March 2021 |
| | | TWI 745021 B | 01 November 2021 |
| | | ES 2859648 T3 | 04 October 2021 |
| | | US 2017021031 A1 | 26 January 2017 |
| | | US 10383878 B2 | 20 August 2019 |
| | | AU 2020200227 A1 | 06 February 2020 |
| | | AU 2020200227 B2 | 07 October 2021 |
| | | JP 2021169515 A | 28 October 2021 |
| | | JP 7138750 B2 | 16 September 2022 |
| | | BR 112016017893 A2 | 17 October 2017 |
| | | BR 112016017893 B1 | 23 August 2022 |
| | | KR 20210041126 A | 14 April 2021 |
| | | SG 10201907807 XA | 27 September 2019 |
| | | RU 2016143351 A | 15 May 2018 |
| | | RU 2016143351 A3 | 05 March 2019 |
| | | RU 2711640 C2 | 17 January 2020 |
| | | IL 293177 A | 01 July 2022 |
| | | EP 3129063 A1 | 15 February 2017 |
| | | EP 3129063 B1 | 27 January 2021 |
| | | PL 3129063 T3 | 12 July 2021 |
| | | DK 3129063 T3 | 06 April 2021 |
| | | SG 11201608309 PA | 29 November 2016 |
| | | KR 20160144396 A | 16 December 2016 |
| | | KR 101937549 B1 | 10 January 2019 |
| | | EP 3789042 A1 | 10 March 2021 |
| | | JP 2020143114 A | 10 September 2020 |
| | | JP 6918182 B2 | 11 August 2021 |
| | | KR 20220012402 A | 03 February 2022 |
| | | KR 102399277 B1 | 18 May 2022 |
| | | TW 202218685 A | 16 May 2022 |
| | | HUE 054411 T2 | 28 September 2021 |
| | | AU 2015245122 A1 | 18 August 2016 |
| | | AU 2015245122 B2 | 24 October 2019 |
| | | AU 2021286320 A1 | 20 January 2022 |
| | | PH 12016501711 A1 | 19 December 2016 |
| | | WO 2015155998 A1 | 15 October 2015 |
| | | KR 20220132025 A | 29 September 2022 |
| | | JP 2017222638 A | 21 December 2017 |
| | | JP 6513128 B2 | 15 May 2019 |
| | | PT 3129063 T | 01 April 2021 |
| CN 109922864 A | 21 June 2019 | US 2020140568 A1 | 07 May 2020 |
| | | US 11325982 B2 | 10 May 2022 |
| | | WO 2018191188 A1 | 18 October 2018 |
| | | EP 3609580 A1 | 19 February 2020 |
| | | EP 3609580 A4 | 24 March 2021 |
| CN 112125915 A | 25 December 2020 | EP 4032892 A1 | 27 July 2022 |

Form PCT/ISA/210 (patent family annex) (July 2022)

**INTERNATIONAL SEARCH REPORT**
**Information on patent family members**

International application No.
**PCT/CN2022/132627**

| Patent document cited in search report | Publication date (day/month/year) | Patent family member(s) | Publication date (day/month/year) |
|---|---|---|---|
| | | WO 2021052402 A1 | 25 March 2021 |
| | | JP 2022548908 A | 22 November 2022 |
| | | BR 112022004913 A2 | 07 June 2022 |
| | | IL 291337 A | 01 May 2022 |
| | | AU 2020347715 A1 | 07 April 2022 |
| CN 111689980 A | 22 September 2020 | None | |
| WO 2020063673 A1 | 02 April 2020 | JP 2022512568 A | 07 February 2022 |
| | | EP 3854816 A1 | 28 July 2021 |
| | | EP 3854816 A4 | 07 September 2022 |
| | | TW 202028242 A | 01 August 2020 |
| | | CA 3114474 A1 | 02 April 2020 |
| | | AU 2019351427 A1 | 15 April 2021 |
| | | KR 20210068457 A | 09 June 2021 |
| | | BR 112021004829 A2 | 08 June 2021 |
| | | US 2021347894 A1 | 11 November 2021 |
| WO 2021147993 A1 | 29 July 2021 | KR 20220130717 A | 27 September 2022 |
| | | AU 2021210074 A1 | 28 July 2022 |
| | | CA 3168260 A1 | 29 July 2021 |
| | | BR 112022014189 A2 | 29 November 2022 |
| | | EP 4095148 A1 | 30 November 2022 |
| | | TW 202140077 A | 01 November 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020063673 A1
- WO 2020063676 A1
- CN 111689980 A
- US 4816567 A


### Non-patent literature cited in the description


- Antibody-drug conjugates: present and future. **BECK, ALAIN ; JANICE M. REICHERT.** MAbs. Taylor & Francis, 2014, vol. 6
- **MCCOMBS, JESSICA R ; SHAWN C. OWEN.** Antibody drug conjugates: design and selection of linker, payload and conjugation chemistry. *The AAPS journal,* 2015, vol. 17, 339-351
- *CHEMICAL ABSTRACTS,* 1599440-13-7
- *CHEMICAL ABSTRACTS,* 646502-53-6